(19) Europäisches Patentamt
European Patent Office
Office européen des brevets

(11) EP 0 873 990 A1

(12) **EUROPEAN PATENT APPLICATION**
published in accordance with Art. 158(3) EPC

(43) Date of publication:
**28.10.1998 Bulletin 1998/44**

(21) Application number: **96931265.1**

(22) Date of filing: **20.09.1996**

(51) Int. Cl.[6]: **C07D 207/14**, C07D 209/14, C07D 211/34, C07D 211/40, C07D 233/48, C07D 239/42, C07D 261/20, C07D 307/79, C07D 401/06, C07D 401/12, C07D 405/12

(86) International application number:
**PCT/JP96/02711**

(87) International publication number:
**WO 97/11054 (27.03.1997 Gazette 1997/14)**

(84) Designated Contracting States:
**AT BE CH DE DK ES FI FR GB GR IE IT LI LU MC NL PT SE**

(30) Priority: **22.09.1995 JP 244040/95**
**29.03.1996 JP 77232/96**

(71) Applicant:
**YOSHITOMI PHARMACEUTICAL INDUSTRIES, LTD.**
**Osaka-shi Osaka 541 (JP)**

(72) Inventors:
- **ITO, Katsuhiko,**
  **Yoshitomi Pharma. Industries Ltd.**
  **Chikujo-gun, Fukuoka 871 (JP)**
- **SONDA, Shuji,**
  **Yoshitomi Pharma. Industries Ltd.**
  **Chikujo-gun, Fukuoka 871 (JP)**
- **KAWAHARA, Toshio,**
  **Yoshitomi Pharma. Industries Ltd**
  **Chikujo-gun, Fukuoka 871 (JP)**
- **ASANO, Kiyoshi,**
  **Yoshitomi Pharma. Industries Ltd.**
  **Chikujo-gun, Fukuoka 871 (JP)**
- **KAWAKıTA, Takeshi,**
  **Yoshitomi Pharma. Ind. Ltd.**
  **Chikujo-gun, Fukuoka 871 (JP)**

(74) Representative:
**von Kreisler, Alek, Dipl.-Chem. et al**
**Patentanwälte,**
**von Kreisler-Selting-Werner,**
**Bahnhofsvorplatz 1 (Deichmannhaus)**
**50667 Köln (DE)**

# (54) BENZOIC ACID COMPOUNDS AND MEDICINAL USE THEREOF

(57) A benzoic acid compound of the formula

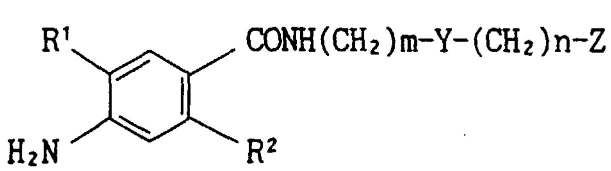

(I)

wherein each symbol is as defined in the specification, an optical isomer thereof and a pharmaceutically acceptable salt thereof. A pharmaceutical composition comprising this compound and a pharmaceutically acceptable additive, a serotonin 4 receptor agonist comprising this compound as an active ingredient, a gastrointestinal prokinetic agent and a therapeutic agent for gastrointestinal diseases.

The compound of the present invention shows selective and high affinity for serotonin 4 receptors, activates same, is useful as a pharmaceutical agent for the prophylaxis and treatment of gastrointestinal diseases (e.g., reflux esophag-

EP 0 873 990 A1

itis; gastroesophageal reflux such as that accompanying cystic fibrosis; Barrett syndrome; intestinal pseudoileus; acute or chronic gastritis; gastric or duodenal ulcer; Crohn's disease; non-ulcer dyspepsia; ulcerative colitis; postgastrectomy syndrome; postoperative digestive function failure; delayed gastric emptying caused by gastric neurosis, gastroptosis, diabetes, and the like; gastrointestinal disorders such as indigestion, meteorism, aodominal indefinite complaint, and the like; constipation such as atonic constipation, chronic constipation, and that caused by spinal cord injury, pelvic diaphragm failure and the like; and irritable bowel syndrome), central nervous disorders (e.g., schizophrenia, depression, anxiety, disturbance of memory and dementia), cardiac function disorders (e.g., cardiac failure and myocardial ischemia), urinary diseases (e.g., dysuria caused by urinary obstruction, ureterolith, prostatomegaly, spinal cord injury, pelvic diaphragm failure, etc.), and shows superior absorption.

## Description

### Technical Field

The present invention relates to novel benzoic acid compounds. More particularly, the present invention relates to novel benzoic acid compounds having high and selective affinity for serotonin 4 (hereinafter referred to as 5-$HT_4$) receptors and capable of activating same, which are useful for the prophylaxis and therapy of various gastrointestinal diseases, central nervous disorders, cardiac function disorders, urinary diseases and the like, optical isomers thereof, pharmaceutically acceptable salts thereof, and use thereof as medicaments.

### Background Art

When control mechanism of gastrointestinal motility fails and prokinetic function is declined, atonic constipation and digestive symptoms such as abdominal distention, anorexia and heartburn emerge. Declined gastrointestinal motility is found with aging, stress or diseases such as chronic gastritis, non-ulcer dyspepsia, reflux esophagitis, peptic ulcer, diabetes and the like, and gastrointestinal prokinetic agents have been used for treatment.

Ever since metoclopramide (The Merck Index, vol. 11, 6063) was developed as a gastrointestinal prokinetic agent, various substituted benzamide derivatives have been synthesized. At present, cisapride (The Merck Index, vol. 11, 2318) and others have been clinically used as gastrointestinal prokinetic agents besides metoclopramide. While benzamide derivatives such as metoclopramide and cisapride have been speculated to show effects via certain receptors in the digestive organs, the actual function of the receptors involved in the promotion of gastrointestinal motility has long been unclarified. Recently, however, 5-$HT_4$ receptor has been identified to be a new serotonin receptor subtype which stimulates adenylate cyclase activity, and benzamide derivatives have been found to promote gastrointestinal motility by activating 5-$HT_4$ receptor in the digestive organs [The Journal of Pharmacology and Experimental Therapeutics, vol. 252, pp. 1378-1386 (1990), European Journal of Pharmacology, vol. 196, pp. 149-155 (1991)]. Having found the action of metoclopramide and cisapride as 5-$HT_4$ receptor agonists, many attempts have been made to use 5-$HT_4$ receptor agonists as gastrointestinal prokinetic agents. The Journal of Pharmacology and Experimental Therapeutics, vol. 264, pp. 240-248 (1993) reports that substituted benzamide (SC53116), which is a 5-$HT_4$ receptor agonist, stimulated gastrointestinal motility. As 5-$HT_4$ receptor agonists, Japanese Patent Unexamined Publication No. 157518/1994 discloses oxadiazole derivatives; Japanese Patent Unexamined Publication No. 10881/1995 discloses oxazabicyclo derivatives; WO 94/12497 discloses endo-N-(8-methyl-8-azabicyclo[3.2.1]oct-3-yl)-1-isopropyl-2(1H)-quinolone-3-carboxamide and acid addition salts thereof; and WO 95/26953 discloses benzoic acid compounds.

The 5-$HT_4$ receptor has been found to be also present in brain (e.g., prefrontal area, nigra, hippocampus and amygdaloid complex), heart, endocrine system and urinary system [British Journal of Pharmacology, vol. 109, pp. 618-624 (1993) and Naunyn-Schmiedeberg's Archives of Pharmacology, vol. 344, pp. 150-159 (1991), Trends in Pharmacological sciences, vol. 13, pp. 141-145 (1992), Pharmacological Reviews, vol. 46, pp. 182-185 (1994)].

In addition, since activation of 5-$HT_4$ receptor in the central nervous system promotes release of acetylcholine in prefrontal area, 5-$HT_4$ receptor is responsible for memory and learning mechanism through release of acetylcholine. Therefore, a 5-$HT_4$ agonist has a potential for a drug for the prophylaxis and treatment of disturbance of memory, dementia and the like. In view of a report documenting that 5-$HT_4$ receptor on GABA neuron is involved in anxiety, it also has a potential for a drug for the prophylaxis and treatment of anxiety. Furthermore, the action of 5-$HT_4$ receptor on heart and urinary system has been reported [Trends in Pharmacological Sciences, vol. 16, pp. 391-398 (1995)].

In view of such disclosures, a compound capable of activating 5-$HT_4$ receptor is considered to be clinically applicable to digestive organs, brain, heart and urinary system. In other words, a 5-$HT_4$ receptor agonist should be useful as a medication for the prophylaxis and treatment of various gastrointestinal diseases (e.g., reflux esophagitis; gastroesophageal reflux such as that accompanying cystic fibrosis; Barrett syndrome; intestinal pseudoileus; acute or chronic gastritis; gastric or duodenal ulcer; Crohn's disease; non-ulcer dyspepsia; ulcerative colitis; postgastrectomy syndrome; postoperative digestive function failure; delayed gastric emptying caused by gastric neurosis, gastroptosis, diabetes, and the like; gastrointestinal disorders such as indigestion, meteorism, abdominal indefinite complaint, and the like; constipation such as atonic constipation, chronic constipation, and that caused by spinal cord injury, pelvic diaphragm failure and the like; and irritable bowel syndrome), central nervous disorders (e.g., schizophrenia, depression, anxiety, disturbance of memory and dementia), cardiac function disorders (e.g., cardiac failure and myocardial ischemia), urinary diseases (e.g., dysuria caused by urinary obstruction, ureterolith, prostatomegaly, spinal cord injury, pelvic diaphragm failure, etc.) and the like.

In addition, a report has documented that 5-$HT_4$ receptor antagonist shows an antagonistic action on analgesic action of cisapride and metoclopramide [Arzneimittel Forschung, vol. 43, pp. 913-918 (1993)]. Thus, a 5-$HT_4$ receptor agonist is expected to be useful as an anti-nociceptor for analgesic use which increases threshold of pain.

While there have been documented some benzoic acid compounds which have N-alkyl substituted cyclic aminoalkyl in the amide moiety and which selectively activate 5-$HT_4$ receptors [British Journal of Pharmacology, vol. 110, pp. 119-126 (1993) and Journal of Medicinal Chemistry, vol, 36, pp. 4121-4123 (1993)], a compound has not been known at all, which has, at an alkyl moiety bonded to nitrogen atom of cyclic amine, amino, (thio)ether, (thio)carbonyl, sulfinyl or sulfonyl.

The above-mentioned substituted benzamide derivatives, such as metoclopramide, have, besides 5-$HT_4$ receptor agonistic effect, dopamine D2 (hereinafter referred to as $D_2$) receptor antagonism or serotonin 3 (hereinafter referred to as 5-$HT_3$) receptor antagonism, and are not entirely satisfactory in terms of efficacy and safety, since they cause side effects such as extrapyramidal disorders due to $D_2$ receptor antagonism, and side effects such as constipation due to 5-$HT_3$ receptor antagonism [Drugs, vol. 41, pp. 574-595 (1991)], and are associated with such problems to be solved.

The compounds disclosed in Japanese Patent Unexamined Publication Nos. 262724/1993, 50883/1989 and 211685/1992 have been investigated as gastrointestinal prokinetic agents, and it has been found that these compounds show not only 5-$HT_4$ receptor activating action but also 5-$HT_3$ receptor antagonism. Therefore, these compounds are again considered to cause side effects such as constipation due to the 5-$HT_3$ receptor antagonism, as mentioned above; and are not satisfactory.

The 5-$HT_4$ receptors have been reported to be widely distributed in the entire digestive tract, and a 5-$HT_4$ receptor agonist having low affinity for $D_2$ receptor and 5-$HT_3$ receptor, but having high and selective affinity for 5-$HT_4$ receptor is expected to make a superior gastrointestinal prokinetic agent.

The benzoic acid compound disclosed in WO 95/26953 has more selective and higher affinity for 5-$HT_4$ receptor as compared to that for $D_2$ receptor and 5-$HT_3$ receptor, and activates same. However, it shows poor absorption by oral administration and low bioavailability. Thus, there is a need for the development of a compound which has selective and high affinity for 5-$HT_4$ receptor, which activates same and which is superior in absorption.

Accordingly, the present invention aims at providing a compound having high and selective affinity for 5-$HT_4$ receptors and capable of activating same in various tissues, which is useful as a medication for the prophylaxis and treatment of various gastrointestinal diseases (e.g., reflux esophagitis; gastroesophageal reflux such as that accompanying cystic fibrosis; Barrett syndrome; intestinal pseudoileus; acute or chronic gastritis; gastric or duodenal ulcer; Crohn's disease; non-ulcer dyspepsia; ulcerative colitis; postgastrectomy syndrome; postoperative digestive function failure; delayed gastric emptying caused by gastric neurosis, gastroptosis, diabetes, and the like; gastrointestinal disorders such as indigestion, meteorism, aodominal indefinite complaint, and the like; constipation such as atonic constipation, chronic constipation, and that caused by spinal cord injury, pelvic diaphragm failure and the like; and irritable bowel syndrome), central nervous disorders (e.g., schizophrenia, depression, anxiety, disturbance of memory and dementia), cardiac function disorders (e.g., cardiac failure and myocardial ischemia), urinary diseases (e.g., dysuria caused by urinary obstruction, ureterolith, prostatomegaly, spinal cord injury, pelvic diaphragm failure, etc.), and the like, and which shows superior absorption.

In view of the above situation, the present inventors have made intensive studies in an attempt to find a compound which has selective and high affinity for 5-$HT_4$ receptor, which is capable of activating same and which is superior in absorption, and found that a new benzoic acid compound, namely, a benzoic acid compound having, at an alkyl moiety bonded to nitrogen atom of cyclic amine of the following formulas (II-a) - (II-i), amino, (thio)ether, (thio)carbonyl, sulfinyl or sulfonyl shows superior selective and high affinity for 5-$HT_4$ receptors, activates same and has superior absorption, which resulted in the completion of the invention.

Disclosure of the Invention

Thus, the present invention provides the following.

(1) Benzoic acid compounds of the formula

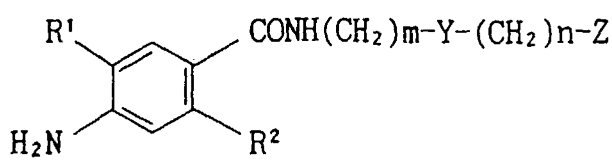

(I)

wherein

$R^1$ is a halogen;
$R^2$ is a lower alkoxy, a substituted lower alkoxy, a cycloalkyloxy or a cycloalkylalkoxy;
m is 1 or 2;

Y is

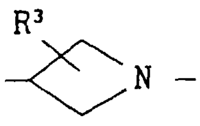

(II-a)

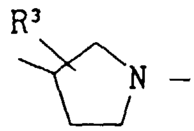

(II-b)

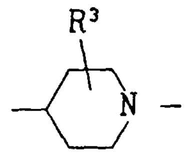

(II-c)

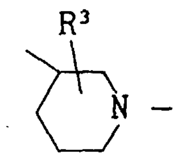

(II-d)

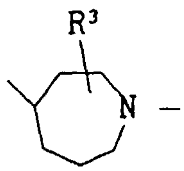

(II-e)

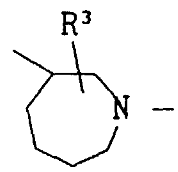

(II-f)

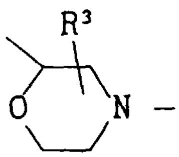

(II-g)

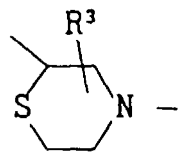

(II-h)

or

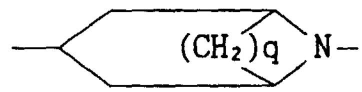

(II-i)

wherein

$R^3$ is hydrogen, hydroxy, lower alkyl or lower alkoxy, and q is 2 or 3;

n is an integer of 1-10; and

Z is a group of the formula

$-N(R^4)(R^5)$,
$-X^1-R^6$ or
$-X^2-R^7$

wherein

$R^4$ and $R^5$ are the same or different and each is hydrogen, lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl, $X^1$ is oxygen atom or sulfur atom, $R^6$ is lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl, $X^2$ is CO, CS, SO or $SO_2$, and $R^7$ is hydrogen, lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl,

optical isomers thereof and pharmaceutically acceptable salts thereof.

(2) Benzoic acid compounds of the formula

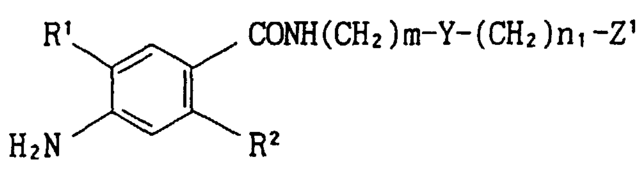

(I-A)

wherein

$R^1$ is a halogen;

$R^2$ is a lower alkoxy, a substituted lower alkoxy, a cycloalkyloxy or a cycloalkylalkoxy;
m is 1 or 2;
Y is

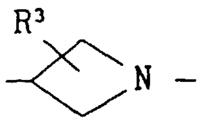
(II-a)

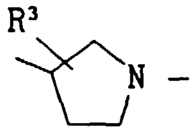
(II-b)

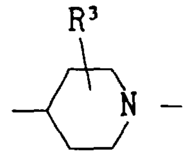
(II-c)

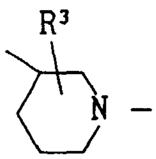
(II-d)

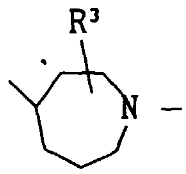
(II-e)

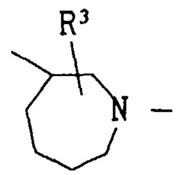
(II-f)

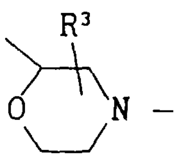
(II-g)

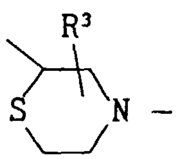
(II-h)

or

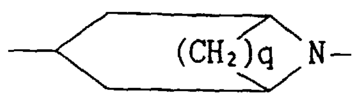
(II-i)

wherein

$R^3$ is hydrogen, hydroxy, lower alkyl or lower alkoxy, and q is 2 or 3;

$n_1$ is an integer of 1-8; and
$Z^1$ is a group of the formula

$-N(R^4)(R^5)$ or
$-X^1-R^6$
wherein
$R^4$ and $R^5$ are the same or different and each is hydrogen, lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl, $X^1$ is oxygen atom or sulfur atom, and $R^6$ is lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl,

optical isomers thereof and pharmaceutically acceptable salts thereof.

(3) Benzoic acid compounds of the formula

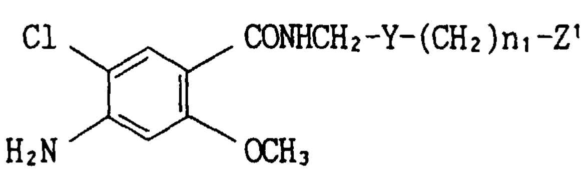
(I-1-A)

wherein Y, $n_1$ and $Z^1$ are as defined above, optical isomers thereof and pharmaceutically acceptable salts thereof.

(4) Benzoic acid compounds of the formula

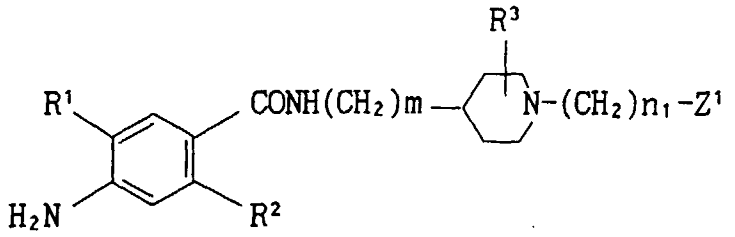

(I-2-A)

wherein $R^1$, $R^2$, $R^3$, m, $n_1$ and $Z^1$ are as defined above, and pharmaceutically acceptable salts thereof.

(5) Benzoic acid compounds of the formula

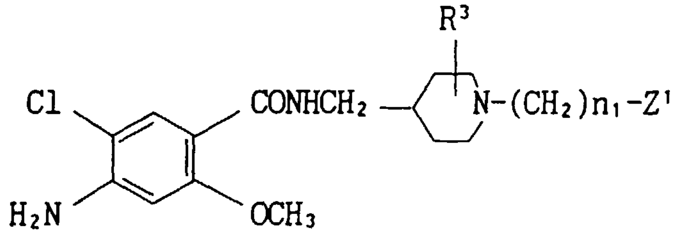

(I-3-A)

wherein $R^3$, $n_1$ and $Z^1$ are as defined above, and pharmaceutically acceptable salts thereof.

(6) Benzoic acid compounds of any one of (2)-(5), wherein $R^4$ and $R^5$ are the same or different and each is hydrogen, lower alkyl, cycloalkylalkyl, aralkyl, substituted aralkyl, heteroarylalkyl or substituted heteroarylalkyl, optical isomers thereof and pharmaceutically acceptable salts thereof.

(7) Benzoic acid compounds of any one of (2)-(5), wherein $R^6$ is aryl, substituted aryl, aralkyl or substituted aralkyl, optical isomers thereof and pharmaceutically acceptable salts thereof.

(8) Benzoic acid compounds of any one of (2)-(7), wherein $n_1$ is an integer of 3 to 8, optical isomers thereof and pharmaceutically acceptable salts thereof.

(9) Benzoic acid compounds of any one of (2)-(7), wherein $n_1$ is an integer of 4 to 6, optical isomers thereof and pharmaceutically acceptable salts thereof.

(10) Benzoic acid compounds of any one of (2)-(6), (8) and (9), which are selected from

4-amino-5-chloro-N-((1-(6-(N-ethyl-N-benzylamino)hexyl)piperidin-4-yl)methyl)-2-methoxybenzamide,
4-amino-5-chloro-N-((1-(5-(benzylamino)pentyl)piperidin-4-yl)methyl)-2-methoxybenzamide,
4-amino-5-chloro-2-methoxy-N-((1-(5-(1-naphthylmethylamino)pentyl)piperidin-4-yl)methyl)benzamide,
4-amino-5-chloro-N-((1-(5-(N-ethyl-N-(4-fluorobenzyl)amino)pentyl)piperidin-4-yl)methyl)-2-methoxybenzamide,
4-amino-5-chloro-2-methoxy-N-((1-(5-(N-n-propyl-N-benzylamino)pentyl)piperidin-4-yl)methyl)benzamide,
4-amino-5-chloro-N-((1-(5-(cyclohexylmethylamino)pentyl)piperidin-4-yl)methyl)-2-methoxybenzamide,
4-amino-5-chloro-N-((1-(4-(N-(3,4-dichlorobenzyl)-N-ethylamino)butyl)piperidin-4-yl)methyl)-2-methoxybenzamide,
4-amino-5-chloro-N-((1-(6-(3,4-dichlorobenzylamino)hexyl)piperidin-4-yl)methyl)-2-methoxybenzamide,
4-amino-5-chloro-N-((1-(6-(N-ethyl-N-(4-methylbenzyl)amino)hexyl)piperidin-4-yl)methyl)-2-methoxybenzamide,
4-amino-5-chloro-N-((1-(6-(N-ethyl-N-(4-nitrobenzyl)amino)hexyl)piperidin-4-yl)methyl)-2-methoxybenzamide,
4-amino-5-chloro-2-methoxy-N-((1-(5-(4-methylbenzylamino)pentyl)piperidin-4-yl)methyl)benzamide,
4-amino-5-chloro-N-((1-(6-(N-ethyl-N-(2-thienylmethyl)amino)hexyl)piperidin-4-yl)methyl)-2-methoxybenzamide,
4-amino-5-chloro-2-methoxy-N-((1-(5-(2-naphthylmethylamino)pentyl)piperidin-4-yl)methyl)benzamide,
4-amino-5-chloro-2-methoxy-N-((1-(6-(4-methoxybenzylamino)hexyl)piperidin-4-yl)methyl)benzamide and
4-amino-5-chloro-2-methoxy-N-((1-(6-(2-thienylmethylamino)hexyl)piperidin-4-yl)methyl)benzamide,
and pharmaceutically acceptable salts thereof.

(11) Benzoic acid compounds of any one of (2)-(5) and (7)-(9), which are selected from

4-amino-N-((1-(5-benzylthiopentyl)piperidin-4-yl)methyl)-5-chloro-2-methoxybenzamide,
4-amino-N-((1-(5-benzyloxypentyl)piperidin-4-yl)methyl)-5-chloro-2-methoxybenzamide,
4-amino-5-chloro-2-methoxy-N-((1-(5-phenoxypentyl)piperidin-4-yl)methyl)benzamide,
4-amino-5-chloro-2-methoxy-N-((1-(5-phenylthiopentyl)piperidin-4-yl)methyl)benzamide,
4-amino-5-chloro-N-((1-(5-(4-chlorobenzyloxy)pentyl)piperidin-4-yl)methyl)-2-methoxybenzamide,
4-amino-5-chloro-2-methoxy-N-((1-(4-phenoxybutyl)piperidin-4-yl)methyl)benzamide and
4-amino-N-((1-(6-benzyloxyhexyl)piperidin-4-yl)methyl)-5-chloro-2-methoxybenzamide,
and pharmaceutically acceptable salts thereof.

(12) Benzoic acid compounds of the formula

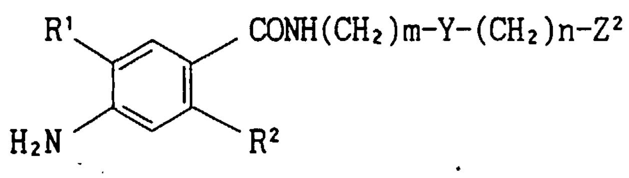

(I-B)

wherein

$R^1$ is a halogen;
$R^2$ is a lower alkoxy, a substituted lower alkoxy, a cycloalkyloxy or a cycloalkylalkoxy;
m is 1 or 2;
Y is

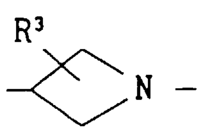

(II-a)

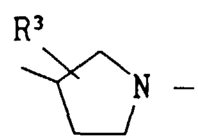

(II-b)

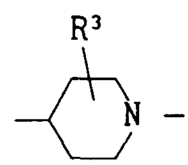

(II-c)

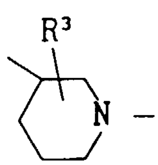

(II-d)

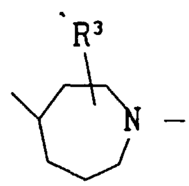

(II-e)

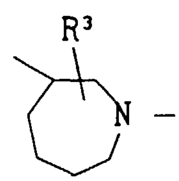

(II-f)

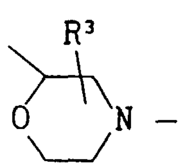

(II-g)

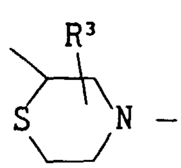

(II-h)

or

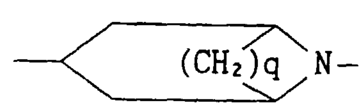

(II-i)

wherein

$R^3$ is hydrogen, hydroxy, lower alkyl or lower alkoxy, and q is 2 or 3;

n is an integer of 1-10; and

$Z^2$ is a group of the formula

$-X^2-R^7$

wherein

$X^2$ is CO, CS, SO or $SO_2$, and $R^7$ is hydrogen, lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl,

optical isomers thereof and pharmaceutically acceptable salts thereof.

(13) Benzoic acid compounds of the formula

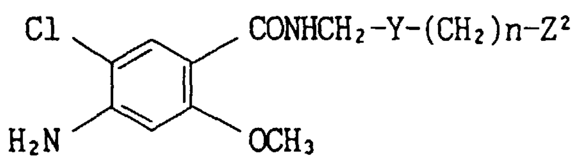

(I-1-B)

wherein Y, n and $Z^2$ are as defined above,
optical isomers thereof and pharmaceutically acceptable salts thereof.
(14) Benzoic acid compounds of the formula

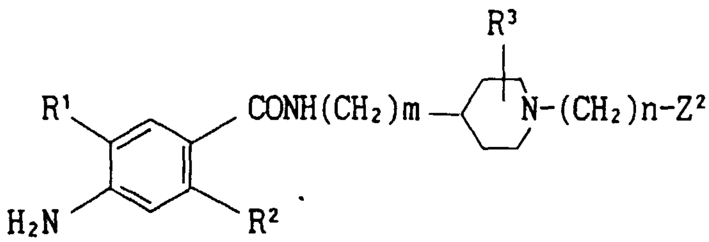

(I-2-B)

wherein $R^1$, $R^2$, $R^3$, m, n and $Z^2$ are as defined above,
and pharmaceutically acceptable salts thereof.
(15) Benzoic acid compounds of the formula

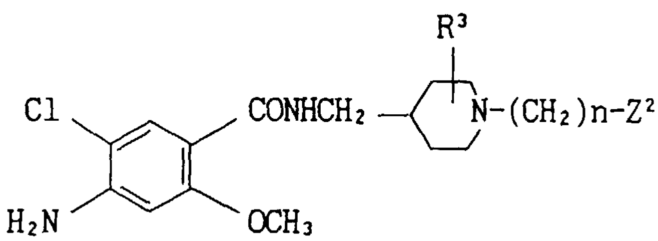

(I-3-B)

wherein $R^3$, n and $Z^2$ are as defined above,
and pharmaceutically acceptable salts thereof.
(16) Benzoic acid compounds of any one of (12)-(15), wherein $X^2$ is CO, and $R^7$ is aryl, substituted aryl, heteroaryl or substituted heteroaryl,
optical isomers thereof and pharmaceutically acceptable salts thereof.
(17) Benzoic acid compounds of any one of (12)-(15), wherein $X^2$ is $SO_2$, and $R^7$ is aryl, substituted aryl, aralkyl or substituted aralkyl,
optical isomers thereof and pharmaceutically acceptable salts thereof.
(18) Benzoic acid compounds of any one of (12)-(17), wherein n is an integer of 3 to 8,

optical isomers thereof and pharmaceutically acceptable salts thereof. (19) Benzoic acid compounds of any one of (12)-(17), wherein n is an integer of 4 to 6,
optical isomers thereof and pharmaceutically acceptable salts thereof.

(20) Benzoic acid compounds of any one of (12)-(16), (18) and (19), which are selected from

4-amino-5-chloro-2-methoxy-N-((1-(6-oxo-6-phenylhexyl)piperidin-4-yl)methyl)benzamide,
4-amino-5-chloro-2-methoxy-N-((1-(7-oxo-7-phenylheptyl)piperidin-4-yl)methyl)benzamide,
4-amino-5-chloro-2-methoxy-N-((1-(6-(1-isopropyl-1H-indol-3-yl)-6-oxohexyl)piperidin-4-yl)methyl)benzamide,
4-amino-5-chloro-2-methoxy-N-((1-(6-(1-ethyl-1H-indol-3-yl)-6-oxohexyl)piperidin-4-yl)methyl)benzamide,
4-amino-5-chloro-2-methoxy-N-((1-(6-(1-naphthyl)-6-oxohexyl)piperidin-4-yl)methyl)benzamide,
4-amino-5-chloro-N-((1-(6-(3-fluoro-4-methoxyphenyl)-6-oxohexyl)piperidin-4-yl)methyl)-2-methoxybenzamide,
4-amino-5-chloro-N-((1-(6-(3-chloro-4-methoxyphenyl)-6-oxohexyl)piperidin-4-yl)methyl)-2-methoxybenzamide,
4-amino-5-chloro-N-((1-(6-(3,4-dimethoxyphenyl)-6-oxohexyl)piperidin-4-yl)methyl)-2-methoxybenzamide,
4-amino-5-chloro-N-((1-(6-(4-hydroxyphenyl)-6-oxohexyl)piperidin-4-yl)methyl)-2-methoxybenzamide,
4-amino-5-chloro-2-methoxy-N-((1-(6-(1-methyl-1H-indol-3-yl)-6-oxohexyl)piperidin-4-yl)methyl)benzamide,
4-amino-5-chloro-2-methoxy-N-((1-(5-(1-methyl-1H-indol-3-yl)-5-oxopentyl)piperidin-4-yl)methyl)benzamide,
4-amino-5-chloro-2-methoxy-N-((1-(7-(1-methyl-1H-indol-3-yl)-7-oxoheptyl)piperidin-4-yl)methyl)benzamide,
4-amino-5-chloro-2-methoxy-N-((1-(5-oxo-5-phenylpentyl)piperidin-4-yl)methyl)benzamide and
4-amino-5-chloro-2-methoxy-N-((1-(6-(1H-indol-3-yl)-6-oxohexyl)piperidin-4-yl)methyl)benzamide,
and pharmaceutically acceptable salts thereof.

(21) Benzoic acid compounds of any one of (12)-(15) and (17)-(19), which are selected from

4-amino-5-chloro-2-methoxy-N-((1-(4-phenylsulfonylbutyl)piperidin-4-yl)methyl)benzamide and
4-amino-5-chloro-2-methoxy-N-((1-(5-phenylsulfonylpentyl)piperidin-4-yl)methyl)benzamide,
and pharmaceutically acceptable salts thereof.

(22) A pharmaceutical composition comprising a benzoic acid compound of any one of (1)-(21), an optical isomer thereof or a pharmaceutically acceptable salt thereof, and a pharmaceutically acceptable additive.
(23) A serotonin 4 receptor agonist comprising a benzoic acid compound of any one of (1)-(21), an optical isomer thereof or a pharmaceutically acceptable salt thereof as an active ingredient.
(24) A gastrointestinal prokinetic agent comprising a benzoic acid compound of any one of (1)-(21), an optical isomer thereof or a pharmaceutically acceptable salt thereof as an active ingredient.
(25) A therapeutic agent for gastrointestinal diseases selected from the group consisting of reflux esophagitis; gastroesophageal reflux such as that accompanying cystic fibrosis; Barrett syndrome; intestinal pseudoileus; acute or chronic gastritis; gastric or duodenal ulcer; Crohn's disease; non-ulcer dyspepsia; ulcerative colitis; postgastrectomy syndrome; postoperative digestive function failure; delayed gastric emptying caused by gastric neurosis, gastroptosis, diabetes, and the like; gastrointestinal disorders such as indigestion, meteorism, abdominal indefinite complaint, and the like; constipation such as atonic constipation, chronic constipation, and that caused by spinal cord injury, pelvic diaphragm failure and the like; and irritable bowel syndrome, which comprises a benzoic acid compound of any one of (1)-(21), an optical isomer thereof or a pharmaceutically acceptable salt thereof as an active ingredient.

The compound of the present invention is characterized by substitution of amino, (thio)ether, (thio)carbonyl, sulfinyl or sulfonyl, at the alkyl moiety bonded to nitrogen atom of cyclic amine of the formulas (II-a) - (II-i), and, with regard to the cyclic amine of the formulas (II-a) - (II-i), bonding between a group of the formula : $-CONH(CH_2)_m-$ which is a bonding site, and carbon atom other than that adjacent to the intercyclic nitrogen atom. Such characteristic chemical structure leads to selective high affinity for 5-$HT_4$ receptor and activation thereof by the compound of the present invention. The compound of the present invention having amino, (thio)ether, (thio)carbonyl, sulfinyl or sulfonyl introduced into the alkyl moiety bonded to nitrogen atom of cyclic amine of the formulas (II-a) - (II-i) characteristically shows superior absorption. In the present invention, "Selectivity" means low affinity for $D_2$ receptors and 5-$HT_3$ receptors and high affinity for 5-$HT_4$ receptors.

Of the above-mentioned symbols, halogen at $R^1$ is exemplified by, for example, fluorine, chlorine, bromine and ibdine, with particular preference given to chlorine.

Lower alkoxy at $R^2$ is a linear or branched alkoxy having 1 to 4 carbon atoms, and exemplified by, for example, methoxy, ethoxy, propoxy, isopropoxy, butoxy, isobutoxy and tert-butoxy, with particular preference given to methoxy.

Substituted lower alkoxy at $R^2$ is substituted by, for example, fluorine, alkoxy (e.g., methoxy, ethoxy and isopropoxy), acyl (e.g., acetyl and propionyl) and cyano. Specific examples of substituted lower alkoxy include, for example, fluoromethoxy, 2- fluoroethoxy, 3-fluoropropoxy, methoxymethoxy, ethoxymethoxy, 2-methoxyethoxy, 2-ethoxyethoxy, 3-methoxypropoxy, 3-ethoxypropoxy, 2-oxopropoxy, 2-oxobutoxy, 3-oxobutoxy, 3-oxopentyloxy, 4-oxopentyloxy, 4-oxohexyloxy, cyanomethoxy, 2-cyanoethoxy and 3-cyanopropoxy.

Cycloalkyloxy at $R^2$ is exemplified by cyclopropyloxy, cyclobutyloxy, cyclopentyloxy, cyclohexyloxy and the like.

Cycloalkylalkoxy at $R^2$ is that wherein linear or branched alkoxy having 1 to 3 carbon atoms (e.g., methoxy, ethoxy, propoxy and isopropoxy) is substituted by cycloalkyl having 3 to 6 carbon atoms, and exemplified by cyclopropylmethoxy, 1-cyclopropylethoxy, 2-cyclopropylethoxy, cyclobutylmethoxy, cyclopentylmethoxy, cyclohexylmethoxy and the like.

Lower alkyl at $R^3$ is a linear or branched alkyl having 1 to 6 carbon atoms, and exemplified by, for example, methyl, ethyl, propyl, isopropyl, butyl, isobutyl, tert-butyl, pentyl and hexyl.

Lower alkoxy at $R^3$ is the same as that at $R^2$, with preference given to methoxy.

Lower alkyl at $R^4$ and $R^5$ is the same as that at $R^3$, with preference given to methyl, ethyl and propyl.

Cycloalkyl at $R^4$ and $R^5$ is that having 3 to 6 carbon atoms, such as cyclopropyl, cyclobutyl, cyclopentyl, cyclohexyl and the like, and preferred are cyclopropyl and cyclohexyl, and particularly preferred is cyclohexyl.

Cycloalkylalkyl at $R^4$ and $R^5$ is linear or branched alkyl having 1 to 4 carbon atoms (e.g., methyl, ethyl, propyl, isopropyl, butyl, isobutyl, tert-butyl and the like) which has been substituted by cycloalkyl at $R^4$ and $R^5$, and examples of cycloalkylalkyl include cyclopropylmethyl, cyclohexylmethyl, 2-cyclohexylethyl, 3-cyclohexylpropyl and the like. Preferred is cyclohexylmethyl.

Aryl at $R^4$ and $R^5$ is phenyl, 1-naphthyl, 2-naphthyl and the like, and preferred is phenyl.

Substituent for substituted aryl at $R^4$ and $R^5$ includes, for example, halogen such as fluorine, chlorine and bromine; lower alkyl having 1 to 4 carbon atoms, such as methyl, ethyl, propyl, isopropyl, butyl, isobutyl and tert-butyl; lower alkoxy having 1 to 4 carbon atoms, such as methoxy, ethoxy, propoxy, isopropoxy, butoxy, isobutoxy and tert-butoxy; hydroxy; nitro; amino; cyano; and alkylenedioxy such as methylenedioxy and ethylenedioxy. Specific examples of substituted aryl include 4-methylphenyl, 2,4-dimethylphenyl, 3,4-dimethylphenyl, 4-ethylphenyl, 2-methoxyphenyl, 3-methoxyphenyl, 4-methoxyphenyl, 3,4-dimethoxyphenyl, 2-chlorophenyl, 3-chlorophenyl, 4-chlorophenyl, 2,3-dichlorophenyl, 2,4-dichlorophenyl, 3,4-dichlorophenyl, 2-fluorophenyl, 3-fluorophenyl, 4-fluorophenyl, 2,4-difluorophenyl, 3,4-difluorophenyl, 3-chloro-4-methoxyphenyl, 2-chloro-4-methylphenyl, 3-chloro-4-methylphenyl, 3-fluoro-4-methoxyphenyl, 4-hydroxyphenyl, 3,4-methylenedioxyphenyl, 4-nitrophenyl, 4-amino-5-chloro-2-methoxyphenyl and the like.

Aralkyl at $R^4$ and $R^5$ is linear or branched alkyl having 1 to 4 carbon atoms (e.g., methyl, ethyl, propyl, isopropyl, butyl, isobutyl, tert-butyl and the like) which has been substituted by aryl at $R^4$ and $R^5$, and examples of aralkyl include benzyl, 2-phenylethyl, 3-phenylpropyl, 4-phenylbutyl, 1-naphthylmethyl, 2-naphthylmethyl and the like. Preferred are benzyl, 1-naphthylmethyl and 2-naphthylmethyl.

Substituent for substituted aralkyl at $R^4$ and $R^5$ is the same as that for substituted aryl at $R^4$ and $R^5$. Preferred are halogen, lower alkyl, lower alkoxy and nitro, with particular preference given to fluorine, chlorine, methyl, methoxy and nitro. Specific examples of substituted aralkyl include 4-fluorobenzyl, 4-methoxybenzyl, 4-chlorobenzyl, 4-methylbenzyl, 4-nitrobenzyl, 3,4-dichlorobenzyl and the like.

Heteroaryl at $R^4$ and $R^5$ is exemplified by pyrrolyl, pyridyl, pyrimidinyl, indolyl, indazolyl, thienyl, furyl, indazolinyl and the like. Preferred are 4-pyridyl, 2-thienyl, 2-pyrimidinyl and 2-indazolinyl.

Substituent for substituted heteroaryl at $R^4$ and $R^5$ is the same as that for substituted aryl at $R^4$ and $R^5$.

Heteroarylalkyl at $R^4$ and $R^5$ is linear or branched alkyl having 1 to 4 carbon atoms (e.g., methyl, ethyl, propyl, isopropyl, butyl, isobutyl, tert-butyl and the like) which has been substituted by heteroaryl at $R^4$ and $R^5$, and examples of heteroarylalkyl include 4-pyridylmethyl, 2-(4-pyridyl)ethyl, 3-(4-pyridyl)propyl, 4-(4-pyridyl)butyl, 2-thienylmethyl and the like. Preferred are 4-pyridylmethyl and 2-thienylmethyl.

Substituent for substituted heteroarylalkyl at $R^4$ and $R^5$ is the same as that for substituted aryl at $R^4$ and $R^5$. Preferred substituent is lower alkyl, particularly methyl. Specific examples of the substituted heteroarylalkyl include 1-methyl-(indol-3-yl)methyl and the like.

Lower alkyl at $R^6$ is the same as that at $R^3$, and preferred are methyl, ethyl, isopropyl, isobutyl and hexyl. Particularly preferred are methyl and ethyl.

Cycloalkyl at $R^6$ is the same as that at $R^4$ and $R^5$, and preferred are cyclopropyl and cyclohexyl.

Cycloalkylalkyl at $R^6$ is the same as that at $R^4$ and $R^5$, and preferred are cyclopropylmethyl and cyclohexylmethyl.

Aryl at $R^6$ is the same as that at $R^4$ and $R^5$, and preferred is phenyl and 1-naphthyl.

Substituent for substituted aryl at $R^6$ is the same as that for substituted aryl at $R^4$ and $R^5$. Preferred substituents are halogen, lower alkyl, lower alkoxy and nitro, with particular preference given to fluorine, chlorine, methyl, methoxy and nitro. Specific examples of substituted aryl include 4-fluorophenyl, 3-methoxyphenyl, 4-chlorophenyl, 4-methylphenyl, 4-methoxyphenyl, 3,4-dimethoxyphenyl, 3,5-dimethoxyphenyl and the like.

Aralkyl at $R^6$ is the same as that at $R^4$ and $R^5$, and preferred are benzyl, 2-phenylethyl, 2-naphthylmethyl and 1-naphthylmethyl.

Substituent for substituted aralkyl at $R^6$ is the same as that for substituted aryl at $R^4$ and $R^5$. Specific examples of substituted aralkyl are 4-fluorobenzyl, 4-chlorobenzyl, 3-methoxybenzyl, 3,4-dimethoxybenzyl, 3,5-dimethoxybenzyl, 3,4-methylenedioxybenzyl, (1,4-benzodioxan-6-yl)methyl and the like.

Heteroaryl at $R^6$ is the same as that at $R^4$ and $R^5$.

Substituent for substituted heteroaryl at $R^6$ is the same as that for substituted aryl at $R^4$ and $R^5$.

Heteroarylalkyl at $R^6$ is the same as that at $R^4$ and $R^5$, and is exemplified by 4-pyridylmethyl, 2-(4-pyridyl)ethyl, 2-thienylmethyl and the like.

Substituent for substituted heteroarylalkyl at $R^6$ is the same as that for substituted aryl at $R^4$ and $R^5$.

Lower alkyl at $R^7$ is the same as that at $R^3$.

Cycloalkyl at $R^7$ is the same as that at $R^4$ and $R^5$.

Cycloalkylalkyl at $R^7$ is the same as that at $R^4$ and $R^5$.

Aryl at $R^7$ is the same as that at $R^4$ and $R^5$.

Substituent for substituted aryl at $R^7$ includes, for example, halogen such as fluorine, chlorine and bromine; lower alkyl having 1 to 4 carbon atoms, such as methyl, ethyl, propyl, isopropyl, butyl, isobutyl and tert-butyl; lower alkoxy having 1 to 4 carbon atoms, such as methoxy, ethoxy, propoxy, isopropoxy, butoxy, isobutoxy and tert-butoxy; haloalkyl such as trifluoromethyl; hydroxy; nitro; amino; acylamino such as acetylamino; cyano; alkylenedioxy such as methylenedioxy and ethylenedioxy; and the like. Preferred are lower alkyl having 1 to 4 carbon atoms, lower alkoxy having 1 to 4 carbon atoms, haloalkyl, halogen, hydroxy, amino, acylamino and alkylenedioxy. Particularly preferred are methyl, ethyl, methoxy, fluorine, chlorine, hydroxy, amino and methylenedioxy. Specific examples of substituted aryl include 4-methylphenyl, 2,4-dimethylphenyl, 3,4-dimethylphenyl, 4-ethylphenyl, 2-methoxyphenyl, 3-methoxyphenyl, 4-methoxyphenyl, 3,4-dimethoxyphenyl, 2-chlorophenyl, 3-chlorophenyl, 4-chlorophenyl, 2,3-dichlorophenyl, 2,4-dichlorophenyl, 3,4-dichlorophenyl, 2-fluorophenyl, 3-fluorophenyl, 4-fluorophenyl, 2,4-difluorophenyl, 3,4-difluonophenyl, 3-chloro-4-methoxyphenyl, 2-chloro-4-methylphenyl, 3-chloro-4-methylphenyl, 3-fluoro-4-methoxyphenyl, 4-hydroxyphenyl, 3,4-methylenedioxyphenyl, 4-nitrophenyl, 4-amino-5-chloro-2-methoxyphenyl and the like.

Aralkyl at $R^7$ is the same as that at $R^4$ and $R^5$, and preferred is 1-naphthylmethyl.

Substituent for substituted aralkyl at $R^7$ is the same as that for substituted aryl at $R^7$. Examples of substituted aralkyl include 4-fluorobenzyl, 4-chlorobenzyl, 3,4-dichlorobenzyl, 4-methoxybenzyl, 4-methylbenzyl, 4-nitrobenzyl and the like.

Heteroaryl at $R^7$ is, for example, pyrrolyl, pyridyl, indolyl, indazolyl, thienyl, furyl, thiazolyl, oxazolyl, benzothienyl, benzofuryl, benzimidazolyl, benzothiazolyl, benzisothiazolyl, benzoxazolyl or benzisoxazolyl. Preferred are 2-thienyl, 2-benzo[b]thienyl, 3-benzo[b]thienyl, 1H-indol-3-yl, 3-benzisothiazolyl and 3-benzisoxazolyl.

Substituent for substituted heteroaryl at $R^7$ includes, for example, halogen such as fluorine, chlorine and bromine; lower alkyl having 1 to 4 carbon atoms, such as methyl, ethyl, propyl, isopropyl, butyl, isobutyl and tert-butyl; lower alkoxy having 1 to 4 carbon atoms, such as methoxy, ethoxy, propoxy, isopropoxy, butoxy, isobutoxy and tert-butoxy; cycloalkylalkyl such as cyclopropylmethyl and cyclohexylmethyl; aralkyl such as benzyl; hydroxy; nitro; amino; and cyano. Preferred are lower alkyl having 1 to 4 carbon atoms, lower alkoxy having 1 to 4 carbon atoms, halogen, cycloalkylalkyl and aralkyl, with particular preference given to methyl, ethyl, propyl, isopropyl, methoxy, chlorine, cyclohexylmethyl and benzyl. Specific examples of substituted heteroaryl include 1-methyl-1H-indol-3-yl, 1-ethyl-1H-indol-3-yl, 1-propyl-1H-indol-3-yl, 1-isopropyl-1H-indol-3-yl, 1-cyclohexylmethyl-1H-indol-3-yl, 1-benzyl-1H-indol-3-yl, 1,5-dimethyl-1H-indol-3-yl, 1-methyl-5-chloro-1H-indol-3-yl, 1-methyl-5-methoxy-1H-indol-3-yl, 2-methyl-3-benzo[b]furyl, 1,2-dimethyl-1H-indol-3-yl, 1-butyl-1H-indol-3-yl and the like.

Heteroarylalkyl at $R^7$ is linear or branched alkyl having 1 to 4 carbon atoms (e.g., methyl, ethyl, propyl, isopropyl, butyl, isobutyl, tert-butyl and the like) which has been substituted by heteroaryl at $R^7$, and examples of heteroarylalkyl include 4-pyridylmethyl, 2-(4-pyridyl)ethyl, 3-(4-pyridyl)propyl, 4-(4-pyridyl)butyl, 2-thienylmethyl, (1H-indol-3-yl)methyl and the like.

Substituent for substituted heteroarylalkyl at $R^7$ is the same as that for substituted aryl at $R^7$. Preferred substituent includes, for example, lower alkyl, particularly methyl. Specific examples of substituted heteroarylalkyl include 1-methyl-(1H-indol-3-yl)methyl and the like.

Preferable examples of the group of the formula : $-Y-(CH_2)n-Z$ include 1-(3-aminopropyl)piperidin-4-yl, 1-(4-aminobutyl)piperidin-4-yl, 1-(5-aminopentyl)piperidin-4-yl, 1-(6-aminohexyl)piperidin-4-yl, 1-(5-dimethylaminopentyl)piperidin-4-yl, 1-(5-diethylaminopentyl)piperidin-4-yl, 1-(6-diethylaminohexyl)piperidin-4-yl, 1-(5-cyclohexylaminopentyl)piperidin-4-yl, 1-(4-cyclohexylmethylaminobutyl)piperidin-4-yl, 1-(5-cyclohexylmethylaminopentyl)piperidin-4-yl, 1-(6-cyclohexylmethylaminohexyl)piperidin-4-yl, 1-(3-benzylaminopropyl)piperidin-4-yl, 1-(4-benzylaminobutyl)piperidin-4-yl, 1-(5-benzylaminopentyl)piperidin-4-yl, 1-(6-benzylaminohexyl)piperidin-4-yl, 1-(4-(4-fluorobenzylamino)butyl)piperidin-4-yl, 1-(5-(4-fluorobenzylamino)pentyl)piperidin-4-yl, 1-(4-(4-chlorobenzylamino)butyl)piperidin-4-yl, 1-(5-(4-chlorobenzylamino)pentyl)piperidin-4-yl, 1-(4-(4-methoxybenzylamino)butyl)piperidin-4-yl, 1-(5-(4-methoxybenzylamino)pentyl)piperidin-4-yl, 1-(6-(4-methoxybenzylamino)hexyl)piperidin-4-yl, 1-(4-(4-methylbenzylamino)butyl)piperidin-4-yl, 1-(5-(4-methylbenzylamino)pentyl)piperidin-4-yl, 1-(6-(4-methylbenzylamino)hexyl)piperidin-4-yl, 1-(5-(4-nitrobenzylamino)pentyl)piperidin-4-yl, 1-(6-(4-nitrobenzylamino)hexyl)piperidin-4-yl, 1-(3-(3,4-dichlorobenzylamino)propyl)piperidin-4-yl, 1-(4-(3,4-dichlorobenzylamino)butyl)piperidin-4-yl, 1-(5-(3,4-dichlorobenzylamino)pentyl)piperidin-4-yl, 1-(6-(3,4-dichlorobenzylamino)hexyl)piperidin-4-yl, 1-(5-(2-thienylmethylamino)pentyl)piperidin-4-yl, 1-(6-(2-thienylmethylamino)hexyl)piperidin-4-yl, 1-(4-(4-pyridylmethylamino)butyl)piperidin-4-yl, 1-(5-(4-pyridylmethylamino)pentyl)piperidin-4-yl, 1-(5-(1-naphthylmethylamino)pentyl)piperidin-4-yl, 1-(6-(1-naphthylmethylamino)hexyl)piperidin-4-yl, 1-(5-(2-naphthylmethylamino)pentyl)piperidin-4-yl, 1-(6-(2-naphthylmethylamino)hexyl)piperidin-4-yl, 1-(4-(N-methyl-N-benzylamino)butyl)piperidin-4-yl, 1-(5-(N-methyl-N-benzylamino)pentyl)piperidin-4-yl, 1-(3-(N-ethyl-N-benzylamino)propyl)piperidin-4-yl, 1-(4-(N-ethyl-N-benzylamino)butyl)piperidin-4-yl, 1-(5-(N-ethyl-N-benzylamino)pentyl)piperidin-4-yl, 1-(6-(N-ethyl-N-benzylamino)hexyl)piperidin-4-yl, 1-(5-(N-n-propyl-N-benzylamino)pentyl)piperidin-4-yl, 1-(5-(N-ethyl-N-cyclohexylamino)pentyl)piperidin-4-yl, 1-(4-(N-ethyl-N-(cyclohexylmethyl)amino)butyl)piperidin-4-yl, 1-(5-(N-ethyl-N-(cyclohexylmethyl)amino)pentyl)piperidin-4-yl, 1-(4-(N-ethyl-N-(4-fluorobenzyl)amino)butyl)piperidin-4-yl, 1-(5-(N-ethyl-N-(4-fluorobenzyl)amino)pentyl)piperidin-4-yl, 1-(4-(N-ethyl-N-(4-chlorobenzyl)amino)butyl)piperidin-4-yl, 1-(5-(N-ethyl-N-(4-chlorobenzyl)amino)pentyl)piperidin-4-yl, 1-(5-(N-ethyl-N-(4-methylbenzyl)amino)pentyl)piperidin-4-yl, 1-(6-(N-ethyl-N-(4-methylbenzyl)amino)hexyl)piperidin-4-yl, 1-(5-(N-ethyl-N-(4-methoxybenzyl)amino)pentyl)piperidin-4-yl, 1-(6-(N-ethyl-N-(4-methoxybenzyl)amino)hexyl)piperidin-4-yl, 1-(5-(N-ethyl-N-(4-nitrobenzyl)amino)pentyl)piperidin-4-yl, 1-(6-(N-ethyl-N-(4-nitrobenzyl)amino)hexyl)piperidin-4-yl, 1-(3-(N-ethyl-N-(3,4-dichlorobenzyl)amino)propyl)piperidin-4-yl, 1-(4-(N-ethyl-N-(3,4-dichlorobenzyl)amino)butyl)piperidin-4-yl, 1-(5-(N-ethyl-N-(3,4-dichlorobenzyl)amino)pentyl)piperidin-4-yl, 1-(6-(N-ethyl-N-(3,4-dichlorobenzyl)amino)hexyl)piperidin-4-yl, 1-(4-(N-ethyl-N-(2-thienylmethyl)amino)butyl)piperidin-4-yl, 1-(5-(N-ethyl-N-(2-thienylmethyl)amino)pentyl)piperidin-4-yl, 1-(6-(N-ethyl-N-(2-thienylmethyl)amino)hexyl)piperidin-4-yl, 1-(4-(N-ethyl-N-(4-pyridylmethyl)amino)butyl)piperidin-4-yl, 1-(5-(N-ethyl-N-(4-pyridylmethyl)amino)pentyl)piperidin-4-yl, 1-(5-(N-ethyl-N-(1-naphthylmethyl)amino)pentyl)piperidin-4-yl, 1-(6-(N-ethyl-N-(1-naphthylmethyl)amino)hexyl)piperidin-4-yl, 1-(5-(N-ethyl-N-(2-naphthylmethyl)amino)pentyl)piperidin-4-yl, 1-(6-(N-ethyl-N-(2-naphthylmethyl)amino)hexyl)piperidin-4-yl , 1-(5-(N-methyl-N-phenylamino)pentyl)piperidin-4-yl, 1-(6-(N-methyl-N-phenylamino)hexyl)piperidin-4-yl, 1-(3-phenoxypropyl)piperidin-4-yl, 1-(4-phenoxybutyl)piperidin-4-yl, 1-(5-phenoxypentyl)piperidin-4-yl, 1-(6-phenoxyhexyl)piperidin-4-yl, 1-(3-(4-fluorophenoxy)propyl)piperidin-4-yl, 1-(4-(4-fluorophenoxy)butyl)piperidin-4-yl, 1-(5-(4-fluorophenoxy)pentyl)piperidin-4-yl, 1-(4-(4-chlorophenoxy)butyl)piperidin-4-yl, 1-(5-(4-chlorophenoxy)pentyl)piperidin-4-yl, 1-(4-(4-methylphenoxy)butyl)piperidin-4-yl, 1-(5-(4-methylphenoxy)pentyl)piperidin-4-yl, 1-(3-benzyloxypropyl)piperidin-4-yl, 1-(4-benzyloxybutyl)piperidin-4-yl, 1-(5-benzyloxpentyl)piperidin-4-yl, 1-(6-benzyloxyhexyl)piperidin-4-yl, 1-(3-phenylthiopropyl)piperidin-4-yl, 1-(4-phenythiobutyl)piperidin-4-yl, 1-(5-phenylthiopentyl)piperidin-4-yl, 1-(6-phenylthiohexyl)piperidin-4-yl, 1-(3-(4-fluorophenylthio)propyl)piperidin-4-yl, 1-(4-(4-fluorophenylthio)butyl)piperidin-4-yl, 1-(5-(4-fluorophenylthio)pentyl)piperidin-4-yl, 1-(4-(4-chlorophenylthio)butyl)piperidin-4-yl, 1-(5-(4-chlorophenylthio)pentyl)piperidin-4-yl, 1-(4-(4-methylphenylthio)butyl)piperidin-4-yl, 1-(5-(4-methylphenylthio)pentyl)piperidin-4-yl, 1-(3-benzylthiopropyl)piperidin-4-yl, 1-(4-benzylthiobutyl)piperidin-4-yl, 1-(5-benzylthiopentyl)piperidin-4-yl, 1-(6-benzylthiohexyl)piperidin-4-yl, 1-(5-(4-chlorobenzyloxy)pentyl)piperidin-4-yl, 1-(5-(2-phenylethyloxy)pentyl)piperidin-4-yl, 1-(5-(2-naphthylmethoxy)pentyl)piperidin-4-yl, 1-(5-cyclohexylmethoxypentyl)piperidin-4-yl, 1-(4-(3-methoxyphenoxy)butyl)piperidin-4-yl, 1-(5-(3-methoxyphenoxy)pentyl)piperidin-4-yl, 1-(4-(3-methoxybenzyloxy)butyl)piperidin-4-yl, 1-(5-(3-methoxybenzyloxy)pentyl)piperidin-4-yl, 1-(4-(3,4-dimethoxyphenoxy)butyl)piperidin-4-yl, 1-(5-(3,4-dimethoxyphenoxy)pentyl)piperidin-4-yl, 1-(4-(3,4-dimethoxybenzyloxy)butyl)piperidin-4-yl, 1-(5-(3,4-dimethoxybenzyloxy)pentyl)piperidin-4-yl, 1-(4-(3,5-dimethoxyphenoxy)butyl)piperidin-4-yl, 1-(5-(3,5-dimethoxy-phen-oxy)pen-tyl)piperidin-4-yl, 1-(4-(3,5-dimethoxybenzyloxy)butyl)piperidin-4-yl, 1-(5-(3,5-dimethoxybenzyloxy)pentyl)piperidin-4-yl, 1-(5-(4-chlorophenoxy)pentyl)piperidin-4-yl, 1-(5-(4-fluorophenoxy)pentyl)piperidin-4-yl, 1-(4-(1-naphthyloxy)butyl)piperidin-4-yl, 1-(5-(1-naphthyloxy)pentyl)piperidin-4-yl, 1-(4-(3,4-methylenedioxyphenylmethoxy)butyl)piperidin-4-yl, 1-(5-(3,4-methylenedioxyphenylmethoxy)pentyl)piperidin-4-yl, 1-(4-((1,4-benzodioxan-6-yl)methoxy)butyl)piperidin-4-yl, 1-(5-((1,4-benzodioxan-6-yl)methoxy)pentyl)piperidin-4-yl, 1-(5-(1-naphthylthio)pentyl)piperidin-4-yl, 1-(5-(2-pyrimidinylamino)pentyl)piperidin-4-yl, 1-(3-oxo-3-phenylpropyl)piperidin-4-yl, 1-(4-oxo-4-phenylbutyl)piperidin-4-yl, 1-(5-oxo-5-phenylpentyl)piperidin-4-yl, 1-(6-oxo-6-phenylhexyl)piperidin-4-yl, 1-(7-oxo-7-phenylheptyl)piperidin-4-yl, 1-(8-oxo-8-phenyloctyl)piperidin-4-yl, 1-(6-(2-fluorophenyl)-6-oxohexyl)piperidin-4-yl, 1-(6-(3-fluorophenyl)-6-oxohexyl)piperidin-4-yl, 1-(6-(4-fluorophenyl)-6-oxohexyl)piperidin-4-yl, 1-(6-(2,3-difluorophenyl)-6-oxohexyl)piperidin-4-yl, 1-(6-(2,4-difluorophenyl)-6-oxohexyl)piperidin-4-yl, 1-(6-(2,5-difluorophenyl)-6-oxohexyl)piperidin-4-yl, 1-(6-(2,6-difluorophenyl)-6-oxohexyl)piperidin-4-yl, 1-(6-(3,4-difluorophenyl)-6-oxohexyl)piperidin-4-yl, 1-(6-(3,5-difluorophenyl)-6-oxohexyl)piperidin-4-yl, 1-(6-(2-chlorophenyl)-6-oxohexyl)piperidin-4-yl, 1-(6-(3-chlorophenyl)-6-oxohexyl)piperidin-4-yl, 1-(6-(4-chlorophenyl)-6-oxohexyl)piperidin-4-yl, 1-(7-(4-chlorophenyl)-7-oxoheptyl)piperidin-4-yl, 1-(6-(2,3-dichlorophenyl)-6-oxohexyl)piperidin-4-yl, 1-(6-(2,4-dichlorophenyl)-6-oxohexyl)piperidin-4-yl, 1-(6-(2,5-dichlorophenyl)-6-oxohexyl)piperidin-4-yl, 1-(6-(2,6-dichlorophenyl)-6-oxohexyl)piperidin-4-yl, 1-(6-(3,4-dichlorophenyl)-6-oxohexyl)piperidin-4-yl, 1-(6-(3,5-dichlorophenyl)-6-oxohexyl)piperidin-4-yl, 1-(6-(4-bromophenyl)-6-oxohexyl)piperidin-4-yl, 1-(6-(2-methylphenyl)-6-oxohexyl)piperidin-4-yl, 1-(6-(3-methylphenyl)-6-oxohexyl)piperidin-4-yl, 1-(6-(4-methylphenyl)-6-oxohexyl)piperidin-4-yl, 1-(6-(2-ethylphenyl)-6-oxohexyl)piperidin-4-yl, 1-(6-(3-ethylphenyl)-6-oxohexyl)piperidin-4-yl, 1-(6-(4-ethylphenyl)-6-oxohexyl)piperidin-4-yl, 1-(6-oxo-6-(2-propylphenyl)hexyl)piperidin-4-yl, 1-(6-oxo-6-(3-propylphenyl)hexyl)piperidin-4-yl, 1-(6-oxo-6-(4-propylphenyl)hexyl)piperidin-4-yl, 1-(6-(2-isopropylphenyl)-6-oxohexyl)piperidin-4-yl, 1-(6-(3-isopropylphenyl)-6-oxohexyl)piperidin-4-yl, 1-(6-(4-isopropylphenyl)hexyl)piperidin-4-yl, 1-(6-(4-butylphenyl)-6-oxohexyl)piperidin-4-yl, 1-(6-(2,3-dimethylphenyl)-6-oxohexyl)piperidin-4-yl, 1-(6-(2,4-dimethylphenyl)-6-oxohexyl)piperidin-4-yl, 1-(6-(2,5-dimethylphenyl)-6-oxohexyl)piperidin-4-yl, 1-(6-(2,6-dimethylphenyl)-6-oxohexyl)piperidin-4-yl, 1-(6-(3,4-dimethylphenyl)-6-oxohexyl)piperidin-4-yl, 1-(6-(3,5-dimethylphenyl)-6-oxohexyl)piperidin-4-yl, 1-(6-(2-methoxyphenyl)-6-oxohexyl)piperidin-4-yl, 1-(6-(3-methoxyphenyl)-6-oxohexyl)piperidin-4-yl, 1-(6-(4-methoxyphenyl)-6-oxohexyl)piperidin-4-yl, 1-(6-(3-fluoro-4-methoxyphenyl)-6-oxohexyl)piperidin-4-yl, 1-(6-(3-chloro-4-methoxyphenyl)-6-oxohexyl)piperidin-4-yl, 1-(6-(3-bromo-4-methoxyphenyl)-6-oxohexyl)piperidin-4-yl, 1-(6-(3-fluoro-4-methylphenyl)-6-oxohexyl)piperidin-4-yl, 1-(6-(3-chloro-4-methylphenyl)-6-oxohexyl)piperidin-4-yl, 1-(6-(3-bromo-4-methylphenyl)-6-oxohexyl)piperidin-4-yl, 1-(6-(4-methoxy-2-methylphenyl)-6-oxohexyl)piperidin-4-yl, 1-(6-(4-methoxy-3-methylphenyl)-6-oxohexyl)piperidin-4-yl, 1-(6-(3,4-dimethoxyphenyl)-6-oxohexyl)piperidin-4-yl, 1-(6-(4-hydroxyphenyl)-6-oxohexyl)piperidin-4-yl, 1-(6-(3,4-dihydroxyphenyl)-6-oxohexyl)piperidin-4-yl, 1-(6-(2-aminophenyl)-6-oxohexyl)piperidin-4-yl, 1-(6-(3-aminophenyl)-6-oxohexyl)piperidin-4-yl, 1-(6-(4-aminophenyl)-6-oxohexyl)piperidin-4-yl, 1-(6-(2-acetaminophenyl)-6-oxohexyl)piperidin-4-yl, 1-(6-(3-acetaminophenyl)-6-oxohexyl)piperidin-4-yl, 1-(6-(4-acetaminophenyl)-6-oxohexyl)piperidin-4-yl, 1-(6-(2-nitrophenyl)-6-oxohexyl)piperidin-4-yl, 1-(6-(3-nitrophenyl)-6-oxohexyl)piperidin-4-yl, 1-(6-(4-nitrophenyl)-6-oxohexyl)piperidin-4-yl, 1-(6-(2-trifluoromethylphenyl)-6-oxohexyl)piperidin-4-yl, 1-(6-(3-trifluoromethylphenyl)-6-oxohexyl)piperidin-4-yl, 1-(6-(4-trifluoromethylphenyl)-6-oxohexyl)piperidin-4-yl, 1-(6-oxo-6-(2-thienyl)hexyl)piperidin-4-yl, 1-(6-oxo-6-(3-thienyl)hexyl)piperidin-4-yl, 1-(6-oxo-6-(2-benzo[b]thienyl)hexyl)piperidin-4-yl, 1-(6-oxo-6-(3-benzo[b]thienyl)hexyl)piperidin-4-yl, 1-(6-(1-methyl-1H-indol-3-yl)-6-oxohexyl)piperidin-4-yl, 1-(6-(1-ethyl-1H-indol-3-yl)-6-oxohexyl)piperidin-4-yl, 1-(6-(1-propyl-1H-indol-3-yl)-6-oxohexyl)piperidin-4-yl, 1-(6-(1-isopropyl-1H-indol-3-yl)-6-oxohexyl)piperidin-4-yl, 1-(6-(1-benzyl-1H-indol-3-yl)-6-oxohexyl)piperidin-4-yl, 1-(6-(1-cyclohexylmethyl-1H-indol-3-yl)-6-oxohexyl)piperidin-4-yl, 1-(6-(1H-indol-3-yl)-6-oxohexyl)piperidin-4-yl, 1-(7-(1-methyl-1H-indol-3-yl)-7-oxoheptyl)piperidin-4-yl, 1-(6-(1-naphthyl)-6-oxohexyl)piperidin-4-yl, 1-(6-(2-naphthyl)-6-oxohexyl)piperidin-4-yl, 1-(6-(2-furyl)-6-oxohexyl)piperidin-4-yl, 1-(6-(3-furyl)-6-oxohexyl)piperidin-4-yl, 1-(6-(2-benzo[b]furyl)-6-oxohexyl)piperidin-4-yl, 1-(6-(3-benzo[b]furyl)-6-oxohexyl)piperidin-4-yl, 1-(6-oxo-6-(2-pyridyl)hexyl)piperidin-4-yl, 1-(6-oxo-6-(3-pyridyl)hexyl)piperidin-4-yl, 1-(6-oxo-6-(4-pyridyl)hexyl)piperidin-4-yl, 1-(6-(3,4-methylenedioxyphenyl)-6-oxohexyl)piperidin-4-yl, 1-(4-phenylsulfonylbutyl)piperidin-4-yl, 1-(5-phenylsulfonylpentyl)piperidin-4-yl, 1-(6-phenylsulfonylhexyl)piperidin-4-yl, 1-(5-phenylsulfinylpentyl)piperidin-4-yl, 1-(6-(4-amino-5-chloro-2-methoxyphenyl)-6-oxohexyl)piperidin-4-yl, 1-(6-(1,5-dimethyl-1H-indol-3-yl)-6-oxohexyl)piperidin-4-yl, 1-(6-(5-chloro-1-methyl-1H-indol-3-yl)-6-oxohexyl)piperidin-4-yl, 1-(6-(5-methoxy-1-methyl-1H-indol-3-yl)-6-oxohexyl)piperidin-4-yl, 1-(6-(3-benzisothiazolyl)-6-oxohexyl)piperidin-4-yl, 1-(6-(3-benzisoxazolyl)-6-oxohexyl)piperidin-4-yl, 1-(5-(1-methyl-1H-indol-3-yl)-5-oxopentyl)piperidin-4-yl, 1-(6-(2-methyl-3-benzo[b]furyl)-6-oxohexyl)piperidin-4-yl, 1-(6-(3-chlorophenyl)-6-oxohexyl)piperidin-4-yl, 1-(6-(2-chlorophenyl)-6-oxohexyl)piperidin-4-yl, 1-(6-(2-chloro-4-methylphenyl)-6-oxohexyl)piperidin-4-yl, 1-(6-(3-chloro-4-methylphenyl)-6-oxohexyl)piperidin-4-yl, 1-(6-(2,3-dichlorophenyl)-6-oxohexyl)piperidin-4-yl, 1-(6-(2-methoxyphenyl)-6-oxohexyl)piperidin-4-yl, 1-(6-(3-methoxyphenyl)-6-oxohexyl)piperidin-4-yl, 1-(6-(2-fluorophenyl)-6-oxohexyl)piperidin-4-yl, 1-(6-(3-fluorophenyl)-6-oxohexyl)piperidin-4-yl, 1-(6-(4-nitrophenyl)-6-oxohexyl)piperidin-4-yl, 1-(3-oxo-3-phenylpropyl)pyrrolidin-3-yl, 1-(4-oxo-4-phenylbutyl)pyrrolidin-3-yl, 1-(5-oxo-5-phenylpentyl)pyrrolidin-3-yl, 1-(6-oxo-6-phenylhexyl)pyrrolidin-3-yl, 1-(7-oxo-7-phenylheptyl)pyrrolidin-3-yl, 1-(8-oxo-8-phenyloctyl)pyrrolidin-3-yl, 1-(6-(1,2-dimethyl-1H-indol-3-yl)-6-oxohexyl)piperidin-4-yl, 1-(6-(1-butyl-1H-indol-3-yl)-6-oxohexyl)piperidin-4-yl, 1-(7-(3-chlorophenyl)-7-oxoheptyl)piperidin-4-yl, 4-methoxy-1-(4-(1-naphthylsulfonyl)butyl)piperidin-4-yl, 1-(5-(1-naphthylsulfonyl)pentyl)piperidin-4-yl, 4-methoxy-1-(4-(1-naphthylmethylsulfonyl)butyl)piperidin-4-yl, 4-methoxy-1-(5-(1-naphthylmethylsulfonyl)pentyl)piperidin-4-yl, 1-(7-(1-methyl-1H-indol-3-yl)-7-oxoheptyl)piperidin-4-yl, 4-hydroxy-1-(6-phenyl-6-oxohexyl)piperidin-4-yl, 4-methoxy-1-(6-phenyl-6-oxohexyl)piperidin-4-yl, 4-methoxy-1-(4-phenylsulfonylbutyl)piperidin-4-yl and the like.

Of these, preferred are 1-(6-(N-benzyl-N-ethylamino)hexyl)piperidin-4-yl, 1-(6-(benzylamino)hexyl)piperidin-4-yl, 1-(5-(1-naphthylmethyl)aminopentyl)piperidin-4-yl, 1-(5-(N-ethyl-N-(4-fluorobenzyl)amino)pentyl)piperidin-4-yl, 1-(5-(N-benzyl-N-propylamino)pentyl)piperidin-4-yl, 1-(5-(cyclohexylmethylamino)pentyl)piperidin-4-yl, 1-(4-(N-(3,4-dichlorobenzyl)-N-ethylamino)butyl)piperidin-4-yl, 1-(6-(3,4-dichlorobenzylamino)hexyl)piperidin-4-yl, 1-(6-(N-ethyl-N-(4-methylbenzyl)amino)hexyl)piperidin-4-yl, 1-(6-(N-ethyl-N-(4-nitro-benzyl)amino)hexyl)piperidin-4-yl, 1-(5-(4-methylbenzylamino)pentyl)piperidin-4-yl, 1-(6-(N-ethyl-N-(2-thienylmethyl)amino)hexyl)piperidin-4-yl, 1-(5-(2-naphthylmethylamino)pentyl)piperidin-4-yl, 1-(6-(4-methoxybenzylamino)hexyl)piperidin-4-yl, 1-(6-(N-(2-thienylmethyl)amino)hexyl)piperidin-4-yl, 1-(5-benzylthiopentyl)piperidin-4-yl, 1-(5-benzyloxypentyl)piperidin-4-yl, 1-(5-phenoxypentyl)piperidin-4-yl, 1-(5-phenylthiopentyl)piperidin-4-yl, 1-(5-(4-chlorobenzyloxy)pentyl)piperidin-4-yl, 1-(4-phenoxybutyl)piperidin-4-yl, 1-(6-phenyl-6-oxohexyl)piperidin-4-yl, 1-(7-phenyl-7-oxoheptyl)piperidin-4-yl, 1-(6-(1-naphthyl)-6-oxohexyl)piperidin-4-yl, 1-(6-(3-fluoro-4-methoxyphenyl)-6-oxohexyl)piperidin-4-yl, 1-(6-(3-chloro-4-methoxyphenyl)-6-oxohexyl)piperidin-4-yl, 1-(6-(3,4-dimethoxyphenyl)-6-oxohexyl)piperidin-4-yl, 1-(6-(4-hydroxyphenyl)-6-oxohexyl)piperidin-4-yl, 1-(6-(1-naphthyl)-6-oxohexyl)piperidin-4-yl, 1-(6-(1-methyl-1H-indol-3-yl)-6-oxohexyl)piperidin-4-yl, 1-(5-(1-methyl-1H-indol-3-yl)-5-oxopentyl)piperidin-4-yl, 1-(7-(1-methyl-1H-indol-3-yl)-7-oxoheptyl)piperidin-4-yl, 1-(6-(1-ethyl-1H-indol-3-yl)-6-oxohexyl)piperidin-4-yl, 1-(6-(1-isopropyl-1H-indol-3-yl)-6-oxohexyl)piperidin-4-yl, 1-(5-phenyl-5-oxopentyl)piperidin-4-yl, 1-(6-(1H-indol-3-yl)-6-oxohexyl)piperidin-4-yl, 1-(4-phenylsulfonylbutyl)piperidin-4-yl and 1-(5-phenylsulfonylpentyl)piperidin-4-yl.

The pharmaceutically acceptable salts of the compound of the present invention are, for example, acid addition salt and quaternary ammonium salt. Examples of the acid addition salt include, for example, hydrochloride, sulfate, hydrobromide, phosphate, nitrate, methanesulfonate, ethanesulfonate, fumarate, maleate, benzoate, citrate, malate, mandelate, p-toluenesulfonate, acetate, succinate, malonate, lactate, salicylate, gallate, picrate, carbonate, ascorbate, trifluoroacetate and tartrate. Examples of quaternary ammonium salt include, for example, quaternary ammonium salts with lower alkyl halide (e.g., methyl iodide, methyl bromide, ethyl iodide, ethyl bromide and the like), lower alkylsulfonate (e.g., methyl methanesulfonate, ethyl methanesulfonate and the like), and lower alkyl arylsulfonate (e.g., methyl p-toluenesulfonate and the like). The N-oxide compounds at the formulas (II-a) to (II-i) of the formula (I) are also encompassed in the compounds of the present invention. The compounds of the present invention may be hydrates (e.g., monohydrate, 1/2 hydrate and 3/2 hydrate) or solvates.

When the compound of the present invention and pharmaceutically acceptable salts thereof have asymmetric carbon, at least two kinds of optical isomers exist. The present invention also encompasses such optical isomers and mixtures thereof. In addition, geometric isomers of cis-compounds and trans-compounds, as well as mixtures thereof are also encompassed in the present invention.

The compound of the present invention can be produced by the following methods.

Method 1 : The compound of the formula (I) can be synthesized by the following route.

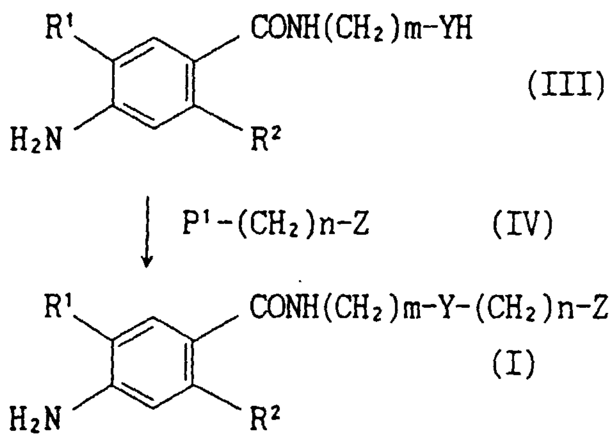


(III)

(I)

wherein $P^1$ is a leaving group such as halogen (e.g., chlorine, bromine, iodine, etc.) or sulfonyloxy (e.g., methanesulfonyloxy, p-toluenesulfonyloxy, trifluoromethanesulfonyloxy, etc.), and other symbols are as defined above.

That is, the compound (I) is produced by reacting compound (III) with compound (IV) in a suitable solvent in the presence of a base.

The solvent to be used for the reaction includes, for example, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene and xylene. The base to be used may be, for example, sodium carbonate, potassium carbonate, sodium hydrogencarbonate, potassium hydrogencarbonate, sodium hydroxide, potassium hydroxide, triethylamine, diisopropylethylamine, pyridine, sodium hydride, sodium methoxide, potassium tert-butoxide and the like. While the reaction temperature varies depending on the kind of solvent to be used, it is generally from 0°C to 140°C, and the reaction time which varies depending on the reaction temperature is generally 1-24 hr.

Method 2 : The compound (III) which is an intermediate shown in Method 1 can be synthesized by the following route.

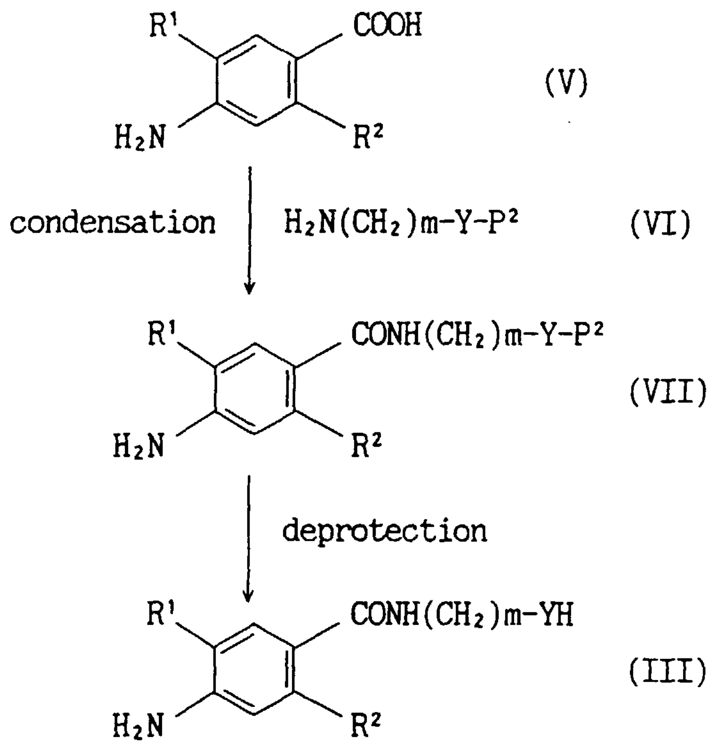


wherein $P^2$ is an urethane type amino-protecting group such as tert-butoxycarbonyl, benzyloxycarbonyl, isopropyloxycarbonyl and the like, and other symbols are as defined above.

That is, compound (III) can be produced by condensing a carboxylic acid represented by compound (V) or its reactive derivative with compound (VI) in a suitable solvent to give compound (VII) and deprotection.

When compound (V) is a free carboxylic acid, condensation is carried out using a routine condensing agent. Examples of the condensing agent include, for example, dicyclohexylcarbodiimide, 1-ethyl-3-(3-dimethylaminopropyl)carbodiimide hydrochloride, carbonyldiimidazole and N-methyl-2-chloropyridinium iodide. This reaction is preferably carried out in the presence of an organic base such as 1-hydroxybenzotriazole, N-methylmorpholine and the like. The solvent to be used includes, for example, dimethylformamide, dimethyl sulfoxide, methylene chloride, tetrahydrofuran, acetonitrile, dioxane and benzene. While the reaction temperature varies depending on the kind of solvent to be used, it is generally from -20°C to 50°C, and the reaction time which varies depending on the reaction temperature is generally 1-24 hr.

When the compound (V) is a reactive derivative of carboxylic acid such as acid halide, acid anhydride, mixed acid anhydride and ester, condensation is generally carried out in a suitable solvent in the presence of a base as necessary. Examples of the base to be used as necessary include, for example, sodium carbonate, potassium carbonate, sodium hydrogencarbonate, potassium hydrogencarbonate, sodium hydroxide, potassium hydroxide, triethylamine, diisopropylethylamine and pyridine. The solvent to be used includes, for example, methylene chloride, chloroform, ethyl acetate, diethyl ether, tetrahydrofuran, dioxane, acetonitrile, benzene, toluene and mixed solvents thereof. While the reaction temperature varies depending on the kind of solvent to be used, it is generally from -30°C to 50°C, and the reaction time which varies depending on the reaction temperature is generally 1-24 hr.

The deprotection of compound (VII) can be carried out by a method generally used for removing amino-protecting group. For example, when $P^2$ is tert-butoxycarbonyl, the compound is treated with an acid in a suitable solvent as necessary. The acid to be used in this reaction may be any as long as amide bonding is not hydrolyzed, and may be, for example, hydrogen chloride, sulfuric acid, trifluoroacetic acid or trifluoromethanesulfonic acid. The solvent to be used as necessary includes, for example, methanol, ethanol, propanol, isopropyl alcohol, tetrahydrofuran, acetone, ethyl acetate, methylene chloride, chloroform, dioxane, water and mixed solvents thereof. The reaction temperature is from ice-cooling to refluxing temperature of the solvent, preferably room temperature, and the reaction time is from 30 min to 5 hr.

Method 3 : The compound (I) can be also synthesized by the following route.

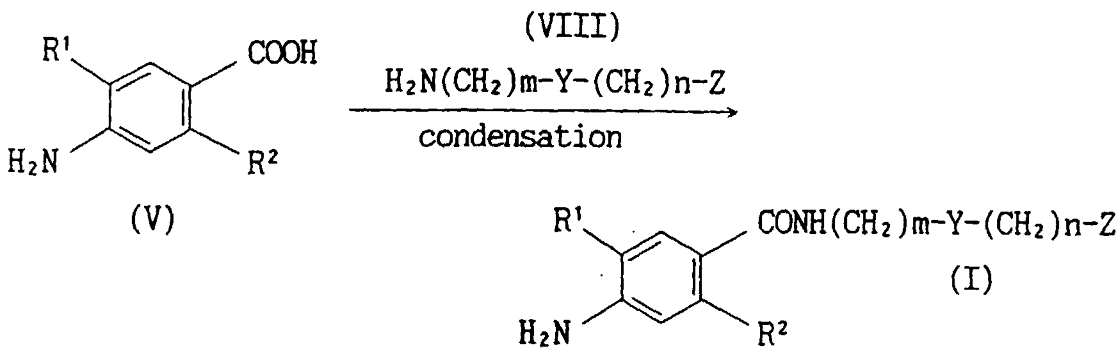


(V)

(I)

wherein each symbol is as defined above.

That is, the compound (I) can be produced by condensing carboxylic acid represented by compound (V) or reactive derivative thereof with compound (VIII) in a suitable solvent.

When compound (V) is a free carboxylic acid, the condensation is carried out using a routine condensing agent. Examples of the condensing agent include dicyclohexylcarbodiimide, 1-ethyl-3-(3-dimethylaminopropyl)carbodiimide hydrochloride, carbonyldiimidazole, N-methyl-2-chloropyridinium iodide and the like. The instant reaction is preferably carried out by condensing in the presence of an organic base such as 1-hydroxybenzotriazole and N-methylmorpholine. The solvent to be used may be, for example, dimethylformamide, dimethyl sulfoxide, methylene chloride, tetrahydrofuran, acetonitrile, dioxane, benzene and the like. While the reaction temperature varies depending on the kind of solvent to be used, it is generally from - 20°C to 50°C, and the reaction time which varies depending on the reaction temperature is generally 1-24 hr.

When compound (V) is reactive derivative of carboxylic acid such as acid halide, acid anhydride, mixed acid anhydride and ester, the condensation is carried out in a suitable solvent in the presence of a base as necessary. The base to be used as necessary is, for example, sodium carbonate, potassium carbonate, sodium hydrogencarbonate, potassium hydrogencarbonate, sodium hydroxide, potassium hydroxide, triethylamine, diisopropylethylamine or pyridine. Examples of the solvent to be used include methylene chloride, chloroform, ethyl acetate, diethyl ether, tetrahydrofuran, dioxane, acetonitrile, benzene, toluene and mixed solvent thereof. While the reaction temperature varies depending on the kind of solvent to be used, it is generally from -30°C to 50°C, and the reaction time which varies depending on reaction temperature is generally 1-24 hr.

Method 4 : A compound (I) wherein Z is a group of the formula $-N(R^4)(R^5)$ wherein $R^4$ is lower alkyl, cycloalkyl, cycloalkylalkyl, aralkyl, substituted aralkyl, heteroarylalkyl or substituted heteroarylalkyl, and $R^5$ is hydrogen, can be synthesized by the following route.

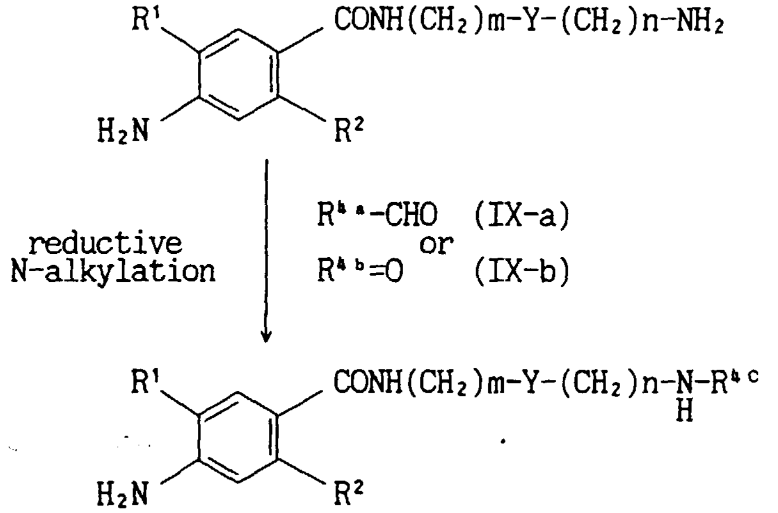


(I-a)

(I-b)

wherein $R^{4a}$ is hydrogen, alkyl having 1 to 5 carbon atoms, cycloalkylalkyl, aralkyl, substituted aralkyl, heteroarylalkyl or substituted heteroarylalkyl, $R^{4b}$ is alkyl having 1 to 6 carbon atoms or cycloalkyl, $R^{4c}$ is alkyl having 1 to 6 carbon atoms, cycloalkyl, cycloalkylalkyl, aralkyl, substituted aralkyl, heteroarylalkyl or substituted heteroarylalkyl, and other symbols are as defined above.

That is, compound (I-b) can be produced by subjecting compound (I-a) to reductive N-alkylation with compound (IX-a) or compound (IX-b) in a suitable solvent.

The solvent to be used for the reductive N-alkylation includes, for example, water, methanol, ethanol, propanol, ethyl acetate, tetrahydrofurn, dioxane, dimethylformamide and acetic acid. The reductive N-alkylating agent to be used includes, for example, sodium cyanoborohydride, sodium borohydride, formic acid and sodium formate. While the reaction temperature varies depending on the kind of solvent to be used, it is from 0°C to 80°C, and the reaction time which varies depending on the reaction temperature is generally 1-24 hr.

Method 5 : A compound (I) wherein Z is a group of the formula $-N(R^4)(R^5)$ wherein $R^4$ and $R^5$ are the same or different and each is lower alkyl, cycloalkyl, cycloalkylalkyl, aralkyl, substituted aralkyl, heteroarylalkyl or substituted heteroarylalkyl can be synthesized by the following route.

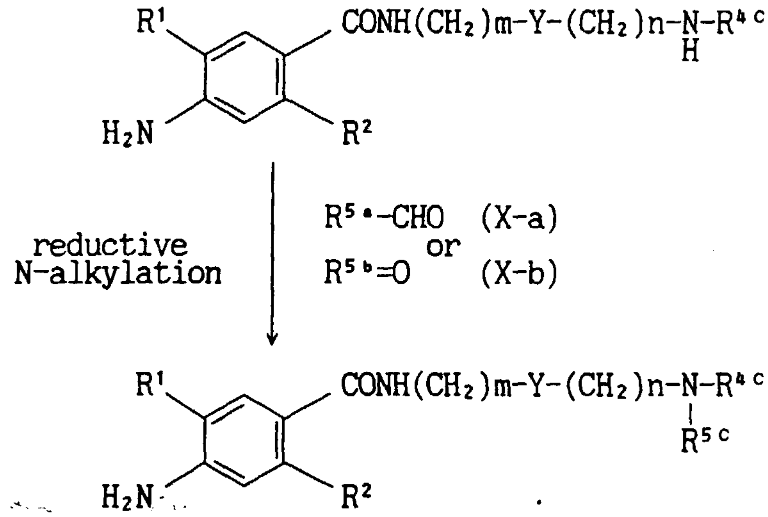


(I-b)

(I-c)

wherein $R^{5a}$ is hydrogen, alkyl having 1 to 5 carbon atoms, cycloalkylalkyl, aralkyl, substituted aralkyl, heteroarylalkyl or substituted heteroarylalkyl, $R^{5b}$ is alkyl having 1 to 6 carbon atoms or cycloalkyl, $R^{5c}$ is alkyl having 1 to 6 carbon atoms, cycloalkyl, cycloalkylalkyl, aralkyl, substituted aralkyl, heteroarylalkyl or substituted heteroarylalkyl, and other symbols are as defined above.

That is, compound (I-c) can be produced by subjecting compound (I-b) to reductive N-alkylation with compound (X-a) or compound (X-b) in a suitable solvent.

The solvent to be used for the reductive N-alkylation includes, for example, water, methanol, ethanol, propanol, ethyl acetate, tetrahydrofuran, dioxane, dimethylformamide and acetic acid. The reductive N-alkylating agent to be used includes, for example, sodium cyanoborohydride, sodium borohydride, formic acid and sodium formate. While the reaction temperature varies depending on the kind of solvent to be used, it is from 0°C to 80°C, and the reaction time which varies depending on the reaction temperature is generally 1-24 hr.

Method 6 : A compound (I) wherein Z is a group of the formula $-NH(R^4)$ or $-N(R^4)(R^5)$ can be synthesized by the following route.

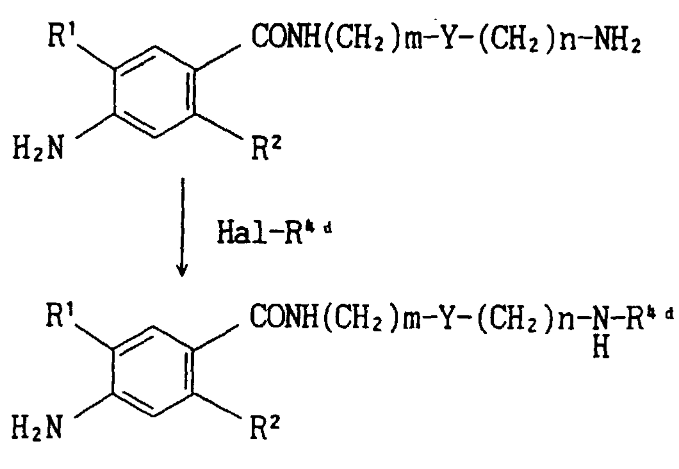


(I-a)

(I-h)

wherein Hal is a halogen such as chlorine, bromine and iodine, $R^{4d}$ is lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl, and other symbols are as defined above.

That is, the compound (I-h) can be produced by subjecting compound (I-a) to alkylation with Hal-$R^{4d}$.

The solvent to be used for this reaction includes, for example, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene and xylene. Examples of the base to be used include, for example, sodium carbonate, potassium carbonate, sodium hydrogencarbonate, potassium hydrogencarbonate, sodium hydroxide, potassium hydroxide, triethylamine, diisopropylethylamine and pyridine. While the reaction temperature varies depending on the kind of solvent to be used, it is generally from 0°C to 140°C, and the reaction time which varies depending on the reaction temperature is generally 1-24 hr.

By then reacting the obtained compound (I-h) with Hal-$R^{5d}$ under the above-mentioned conditions, compound (I-i) of the formula

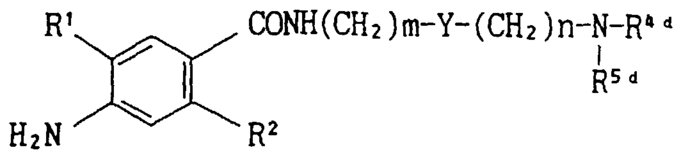

(I-i)

wherein $R^{5d}$ is lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl, and other symbols are as defined above, can be obtained.

Method 7: The compound (I-a) can be synthesized by the following route.

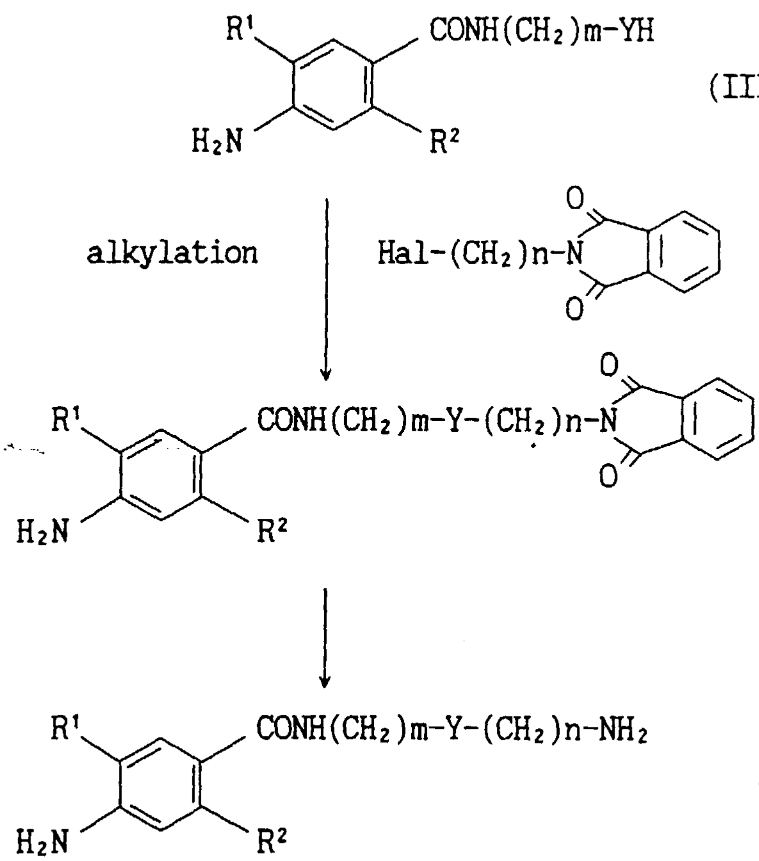


(III)

(XI)

(XII)

(I-a)

wherein each symbol is as defined above.

That is, the compound (III) is subjected to alkylation with compound (XI) in a suitable solvent in the presence of a

base to give compound (XII) and treating the obtained compound in a suitable solvent in the presence of a base to give compound (I-a).

The solvent to be used for the alkylation of compound (III) includes, for example, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene, xylene and the like. The base to be used may be, for example, sodium carbonate, potassium carbonate, sodium hydrogencarbonate, potassium hydrogencarbonate, sodium hydroxide, potassium hydroxide, triethylamine, diisopropylethylamine or pyridine. While the reaction temperature varies depending on the kind of solvent to be used, it is generally from 0°C to 140°C, and the reaction time which varies depending on the reaction temperature is generally 1-24 hr.

The solvent to be used for the reaction from compound (XII) to compound (I-a) includes, for example, methanol, ethanol, propanol, isopropyl alcohol and butanol. The base to be used may be, for example, hydrazine hydrate, methylhydrazine, phenylhydrazine and methylamine. While the reaction temperature varies depending on the kind of solvent to be used, it is generally from 50°C to the boiling point of the solvent to be used, and the reaction time which varies depending on the reaction temperature is generally 1-10 hr.

Method 8 : The compound (XII) can be also synthesized by the following route.

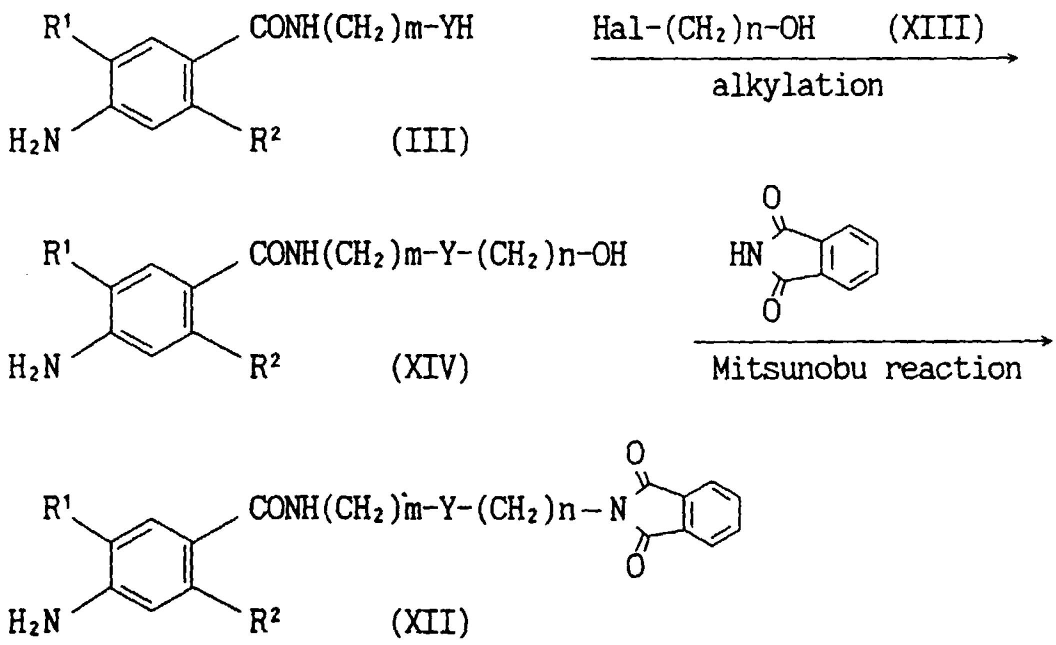


wherein each symbol is as defined above.

That is, compound (XII) can be produced by subjecting compound (III) to alkylation with compound (XIII) in a suitable solvent in the presence of a base to give compound (XIV) and subjecting this compound to Mitsunobu reaction [Synthesis, p. 1 (1981)] with phthalimide in a suitable solvent.

The solvent to be used for the alkylation includes, for example, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene and xylene. Examples of the base to be used include sodium carbonate, potassium carbonate, sodium hydrogencarbonate, potassium hydrogencarbonate, sodium hydroxide, potassium hydroxide, triethylamine, diisopropylethylamine, pyridine and the like. While the reaction temperature varies depending on the kind of solvent to be used, it is generally from 0°C to 140°C, and the reaction time which varies depending on the reaction temperature is generally 1-24 hr.

The reagent used for the Mitsunobu reaction includes, for example, azodicarboxylate (e.g., ethyl azodicarboxylate and the like)-triphenylphosphine.

The solvent to be used for the Mitsunobu reaction includes, for example, tetrahydrofuran and dioxane. The reaction temperature is generally from -20°C to 40°C, and the reaction time which varies depending on the reaction temperature is generally 1-24 hr.

Method 9 : The compound (IV) which is an intermediate shown in Method 1 can be synthesized by the following route.

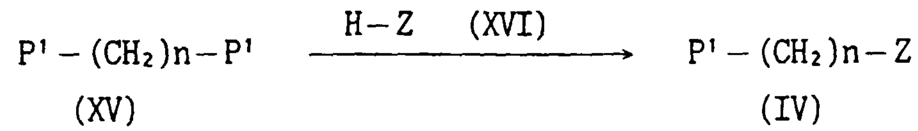


(XV) (IV)

wherein each symbol is as defined above.

That is, compound (IV) can be produced by reacting compound (XV) with compound (XVI) in a suitable solvent in the presence of a base.

The solvent to be used for the reaction includes, for example, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene and xylene. Examples of the base to be used include, for example, sodium hydride, sodium carbonate, potassium carbonate, sodium hydrogencarbonate, potassium hydrogencarbonate, sodium hydroxide, potassium hydroxide, triethylamine, diisopropylethylamine and pyridine. While the reaction temperature varies depending on the kind of solvent to be used, it is generally from 0°C to 140°C, and the reaction time which varies depending on the reaction temperature is generally 1-24 hr.

Method 10 :

A compound (VIII) wherein Z is a group of the formula -NH($R^4$) or -N($R^4$)($R^5$) wherein $R^4$ and $R^5$ are the same or different and each is lower alkyl, cycloalkyl, cycloalkylalkyl, aralkyl, substituted aralkyl, heteroarylalkyl or substituted heteroarylalkyl, can be synthesized by the following route.

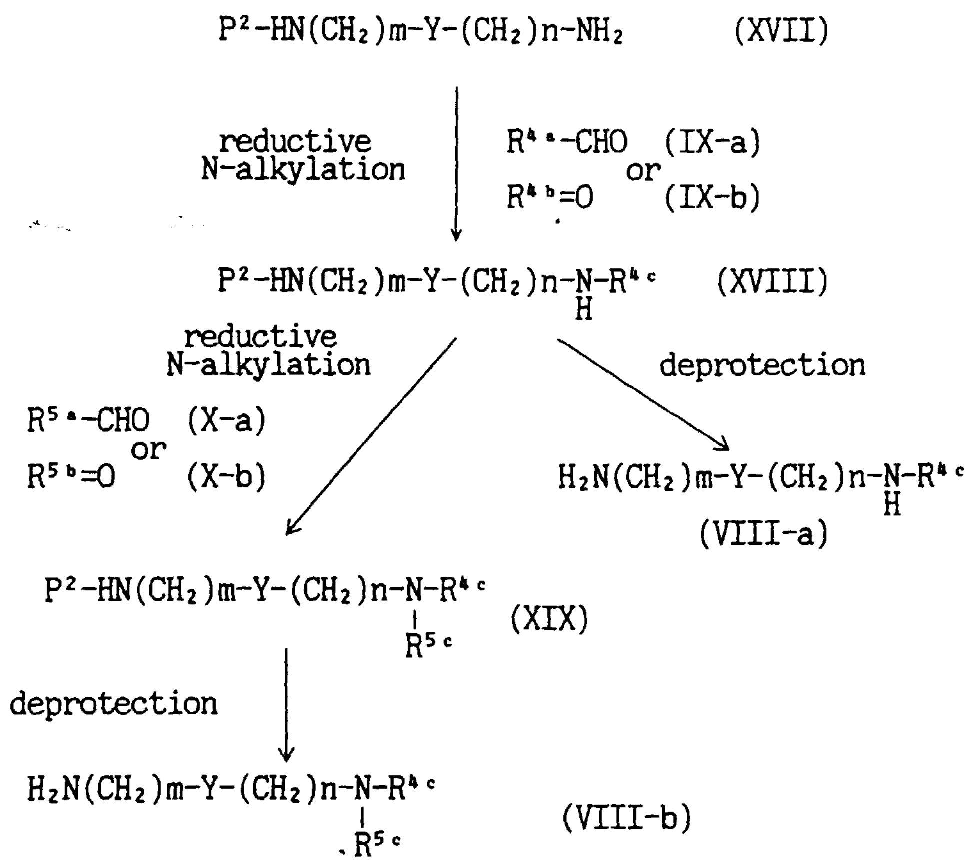


(XVII) (IX-a) or (IX-b) (XVIII) (X-a) or (X-b) (VIII-a) (XIX) (VIII-b)

wherein each symbol is as defined above.

That is, compound (VIII-a) can be produced by subjecting compound (XVII) to reductive N-alkylation with compound (IX-a) or compound (IX-b) in a suitable solvent to give compound (XVIII) and then deprotection. Also, compound (VIII-b) can be produced by subjecting compound (XVIII) to reductive N-alkylation with compound (X-a) or compound (X-b) in a suitable solvent to give compound (XIX) and then deprotection thereof.

The solvent to be used for the reductive N-alkylation of compound (XVII) and compound (XVIII) includes, for example, water, methanol, ethanol, propanol, ethyl acetate, tetrahydrofuran, dioxane, dimethylformamide and acetic acid. The reductive N-alkylating agent to be used includes, for example, sodium cyanoborohydride, sodium borohydride, formic acid and sodium formate. While the reaction temperature varies depending on the kind of solvent to be used, it is from 0°C to 80°C, and the reaction time which varies depending on the reaction temperature is generally 1-24 hr.

The deprotection of amino of compound (XVIII) and compound (XIX) can be carried out by a method generally used for removing amino-protecting group. For example, when $P^2$ is tort-butoxycarbonyl, the compound is treated with an acid in a suitable solvent as necessary. The acid to be used in this reaction may be any as long as amide bonding is not hydrolyzed, and may be, for example, hydrogen chloride, sulfuric acid, trifluoroacetic acid or trifluoromethanesulfonic acid. The solvent to be used as necessary includes, for example, methanol, ethanol, propanol, isopropyl alcohol, tetrahydrofuran, acetone, ethyl acetate, methylene chloride, chloroform, dioxane, water and mixed solvents thereof. The reaction temperature is from ice-cooling to refluxing temperature of the solvent, preferably room temperature, and the reaction time is from 30 min to 5 hr.

<u>Method 11 :</u> A compound (VIII) wherein Z is a group of the formula $-X^1-R^6$ can be synthesized by the following route.

$P^2-HN(CH_2)m-Y-(CH_2)n-X^1H$ (XX)

$R^6-Hal$ (XXI)

$P^2-HN(CH_2)m-Y-(CH_2)n-X^1-R^6$ (XXII)

deprotection

$H_2N(CH_2)m-Y-(CH_2)n-X^1-R^6$ (VIII-c)

wherein each symbol is as defined above.

That is, compound (VIII-c) can be produced by reacting compound (XX) with compound (XXI) in a suitable solvent in the presence of a base to give compound (XXII) and deprotection thereof.

The solvent to be used for the reaction includes, for example, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene and xylene. Examples of the base to be used include, for example, sodium hydride, sodium carbonate, potassium carbonate, sodium hydrogencarbonate, potassium hydrogencarbonate, sodium hydroxide, potassium hydroxide, triethylamine, diisopropylethylamine and pyridine. While the reaction temperature varies depending on the kind of solvent to be used, it is generally from 0°C to 140°C, and the reaction time which varies depending on the reaction temperature is generally 1-24 hr.

The deprotection of amino of compound (XXII) can be carried out by a method generally used for removing amino-protecting group. For example, when $P^2$ is tert-butoxycarbonyl, the compound is treated with an acid in a suitable solvent as necessary. The acid to be used in this reaction may be any as long as amide bonding is not hydrolyzed, and may be, for example, hydrogen chloride, sulfuric acid, trifluoroacetic acid or trifluoromethanesulfonic acid. The solvent to be used as necessary includes, for example, methanol, ethanol, propanol, isopropyl alcohol, tetrahydrofuran, acetone, ethyl acetate, methylene chloride, chloroform, dioxane, water and mixed solvents thereof. The reaction temperature is from ice-cooling to refluxing temperature of the solvent, preferably room temperature, and the reaction time is from 30 min to 5 hr.

Method 12: The compound (XVII) which is the starting compound in Method 10 can be synthesized by the following route.

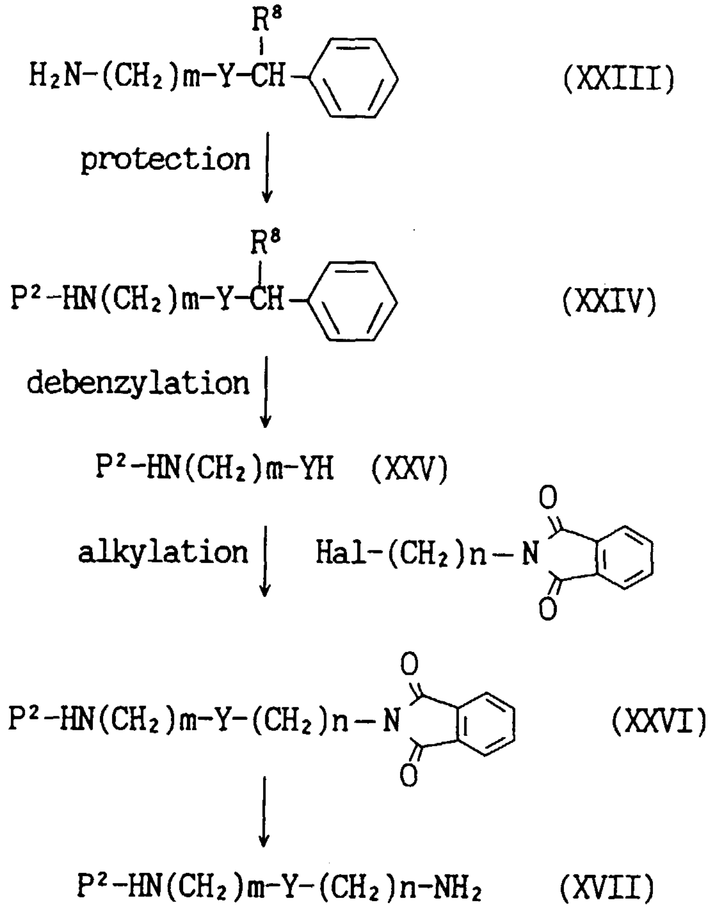


(XXIII)

(XXIV)

(XXV)

(XI)

(XXVI)

(XVII)

wherein $R^8$ is hyrogen or lower alkyl, and other symbols are as defined above.

That is, the compound (XXV) can be produced by reacting a starting compound (XXIII) with an amino-protecting reagent in a suitable solvent to give compound (XXIV), and subjecting the resulting compound to debenzylation in a suitable solvent in the presence of a catalyst under a hydrogen atmosphere or using a suitable hydrogen source. Then, the compound (XXV) is subjected to alkylation with compound (XI) in a suitable solvent in the presence of a base to give compound (XXVI), and the resulting compound is reacted in a suitable solvent in the presence of a base to give compound (XVII).

The amino-protecting reagent to be used for protecting compound (XXIII) includes, for example, di-tert-butyl dicarbonate, 2-tert-butoxycarbonyloxyimino-2-phenylacetonitrile and benzyloxycarbonyl chloride. The solvent to be used includes, for example, tetrahydrofuran, acetone, ethyl acetate, methylene chloride, chloroform, dioxane, dimethylformamide, water and mixed solvents thereof. While the reaction temperature varies depending on the kind of solvent to be used, it is generally from -20°C to 50°C, and the reaction time which varies depending on the reaction temperature is generally 1-24 hr.

The solvent to be used for debenzylation of compound (XXIV) includes, for example, methanol, ethanol, propanol, isopropyl alcohol, butanol, formic acid, acetic acid, water, tetrahydrofuran, dimethyl sulfoxide and mixed solvents thereof. The catalyst to be used includes, for example, palladium-carbon, Raney-nickel and platinum oxide. The hydrogen source to be used includes, for example, hydrazine hydrate, cyclohexene, 1,4-cyclohexadiene, formic acid and ammonium formate. While the reaction temperature varies depending on the kind of solvent to be used, it is generaly from 0°C to the boiling point of the solvent to be used, and the reaction time which varies depending on the reaction temperature is generally 1-6 hr.

The solvent to be used for alkylation of compound (XXV) includes, for example, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene, xylene and the like. The base to be used may be, for example, sodium carbonate, potassium carbonate, sodium hydrogencarbonate, potassium hydrogencarbonate, sodium hydroxide, potassium hydroxide, triethylamine, diisopropylethylamine or pyridine.

While the reaction temperature varies depending on the kind of solvent to be used, it is generally from 0°C to 140°C, and the reaction time which varies depending on the reaction temperature is generally 1-24 hr.

The solvent to be used for the reaction from compound (XXVI) to compound (XVII) includes, for example, methanol, ethanol, propanol, isopropyl alcohol and butanol. The base to be used may be, for example, hydrazine hydrate, methylhydrazine, phenylhydrazine and methylamine. While the reaction temperature varies depending on the kind of solvent to be used, it is generally from 50°C to the boiling point of the solvent to be used, and the reaction time which varies depending on the reaction temperature is generally 1-10 hr.

Method 13 : The compound (XX) which is a starting compound used in Method 11 can be synthesized by the following route.

$P^2$-HN$(CH_2)$m-YH (XXV)

alkylation $\downarrow$ Hal-$(CH_2)$n-$X^1$H (XXVII)

$P^2$-HN$(CH_2)$m-Y-$(CH_2)$n-$X^1$H (XX)

wherein each symbol is as defined above.

That is, compound (XX) can be produced by subjecting compound (XXV) to alkylation with compound (XXVII) in a suitable solvent in the presence of a base.

The solvent to be used for the alkylation includes, for example, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene and xylene. Examples of the base to be used include, for example, sodium carbonate, potassium carbonate, sodium hydrogencarbonate, potassium hydrogencarbonate, sodium hydroxide, potassium hydroxide, triethylamine, diisopropylethylamine and pyridine. While the reaction temperature varies depending on the kind of solvent to be used, it is generally from 0°C to 140°C, and the reaction time which varies depending on the reaction temperature is generally 1-24 hr.

Method 14 : A compound (VIII) which is an intermediate shown in Method 3 can be synthesized by the following route.

$P^2$-HN$(CH_2)$m-YH (XXV)

alkylation $\downarrow$ Hal-$(CH_2)$n-Z (XXVIII)

$P^2$-HN$(CH_2)$m-Y-$(CH_2)$n-Z (XXIX)

deprotection $\downarrow$

$H_2N(CH_2)$m-Y-$(CH_2)$n-Z (VIII)

wherein each symbol is as defined above.

That is, compound (VIII) can be produced by subjecting compound (XXV) to alkylation with compound (XXVIII) in

a suitable solvent in the presence of a base to give compound (XXIX) and deprotection thereof.

The solvent to be used for the alkylation includes, for example, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene and xylene. Examples of the base to be used include, for example, sodium carbonate, potassium carbonate, sodium hydrogencarbonate, potassium hydrogencarbonate, sodium hydroxide, potassium hydroxide, triethylamine, diisopropylethylamine and pyridine. While the reaction temperature varies depending on the kind of solvent to be used, it is generally from 0°C to 140°C, and the reaction time which varies depending on the reaction temperature is generally 1-24 hr.

The deprotection of amino of compound (XXIX) can be carried out by a method generally used for removing amino-protecting group. For example, when $P^2$ is tert-butoxycarbonyl, the compound is treated with an acid in a suitable solvent as necessary. The acid to be used in this reaction may be, for example, hydrogen chloride, sulfuric acid, trifluoroacetic acid or trifluoromethanesulfonic acid. The solvent to be used as necessary includes, for example, methanol, ethanol, propanol, isopropyl alcohol, tetrahydrofuran, acetone, ethyl acetate, methylene chloride, chloroform, dioxane, water and mixed solvents thereof. The reaction temperature is from ice-cooling to refluxing temperature of the solvent, preferably room temperature, and the reaction time is from 30 min to 5 hr.

Method 15 : A compound (I) wherein Z is a group of the formula $-CS-R^7$ can be synthesized by the following route.

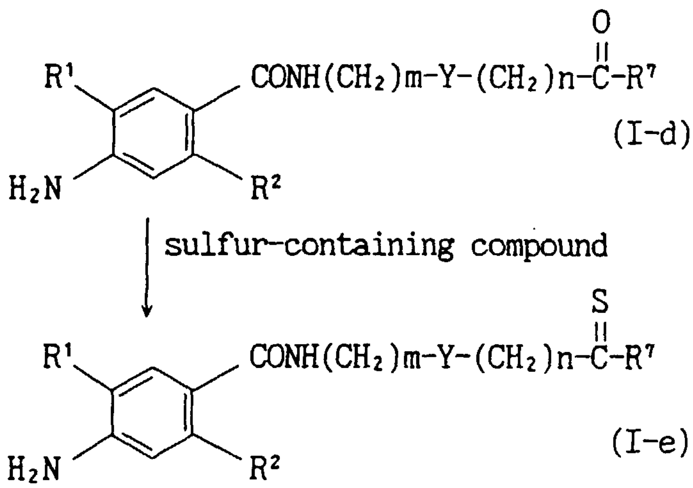


wherein each symbol is as defined above.

That is, compound (I-e) can be produced by reacting compound (I-d) with sulfur-containing compound in a suitable solvent in the presence of an acid or base as necessary.

Examples of the sulfur-containing compound include, for example, hydrogen sulfide, phosphorus pentasulfide, sodium sulfide, potassium sulfide and Lawesson's reagent. The solvent to be used includes, for example, methanol, ethanol, acetic acid, dimethylformamide, dimethyl sulfoxide, methylene chloride, chloroform, ethyl acetate, diethyl ether, tetrahydrofuran, dioxane, acetonitrile, benzene, toluene and mixed solvents thereof. The acid to be used as necessary includes, for example, hydrochloric acid, acetic acid, sulfuric acid, phosphoric acid and hydrofluoric acid. Examples of the base to be used as necessary include, for example, ammonia, methylamine, ethylamine, dimethylamine, diethylamine, pyrrolidine, piperidine, piperazine and morpholine. While the reaction temperature varies depending on the kind of solvent to be used, it is generally from -30°C to 100°C, and the reaction time which varies depending on the reaction temperature is generally 1-24 hr.

Method 16 : A compound (I) wherein Z is a group of the formula $-SO-R^7$ or $-SO_2-R^7$ can be synthesized by the following route.

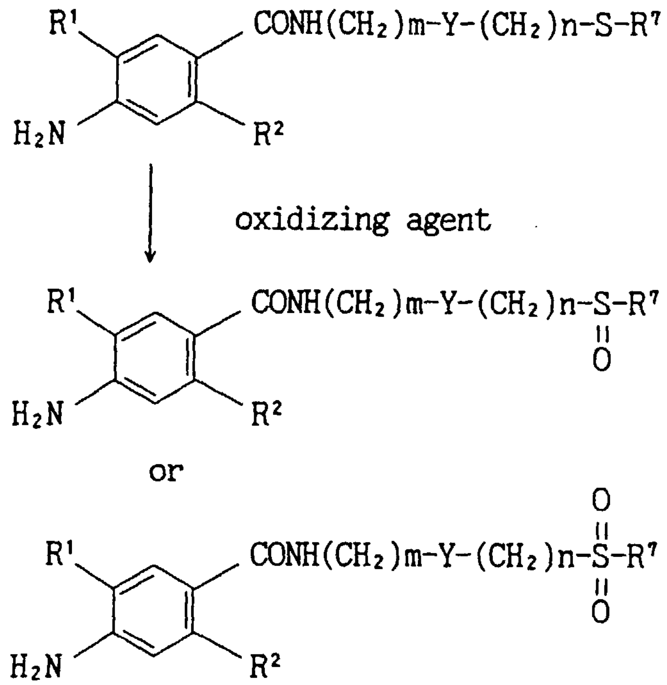


(XXX)

(I-f)

(I-g)

wherein each symbol is as defined above.

That is, compound (I-f) or compound (I-g) can be produced by reacting compound (XXX) with 1 or 2 equivalent(s) of an oxidizing agent in a suitable solvent in the presence of an acid as necessary.

The oxidizing agent to be used for the reaction includes, for example, ruthenium tetraoxide, chromic acid, permanganate, m-chloroperbenzoic acid and aqueous hydrogen peroxide solution. The solvent to be used for the oxidation includes, for example, formic acid, acetic acid, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene and xylene. Examples of the acid to be used as necessay include, for example, hydrochloric acid, sulfuric acid, p-toluenesulfonic acid and benzoic acid. While the reaction temperature varies depending on the kind of oxidizing agent to be used, it is generally from 0°C to 140°C, and the reaction time which varies depending on the reaction temperature is generally 1-24 hr.

Method 17: A quaternary ammonium salt of the compound of the formula (I) can be synthesized by the following route.

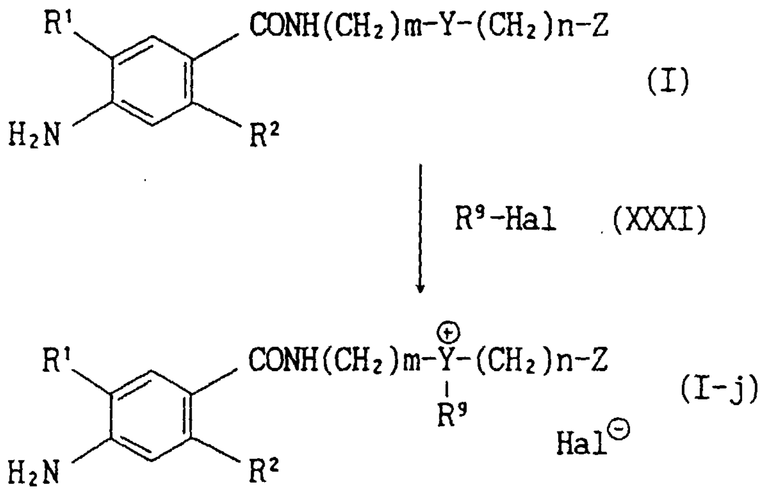


(I)

(I-j)

wherein $R^9$ is lower alkyl and the formula

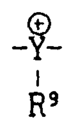

wherein each symbol is as defined above, means a group of the formula

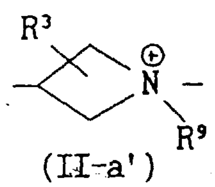

(II-a')

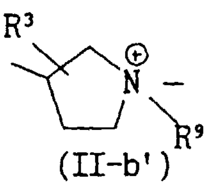

(II-b')

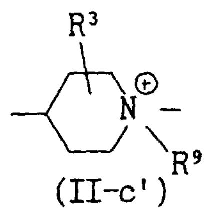

(II-c')

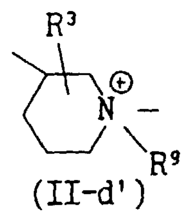

(II-d')

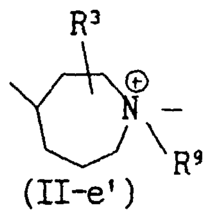

(II-e')

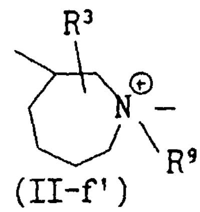

(II-f')

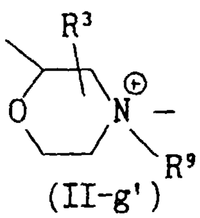

(II-g')

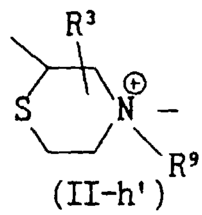

(II-h')

or

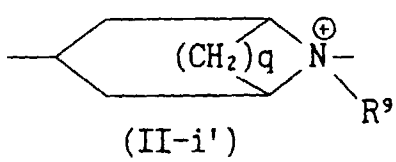

(II-i')

wherein each symbol is as defined above, and other symbols are as defined above.

That is, the compound (I-j) can be produced by reacting compound (I) with compound (XXXI) in a suitable solvent.

The solvent to be used includes, for example, methylene chloride, 1,2-dichloroethane, chloroform, methanol, ethanol, propanol, isopropyl alcohol, butanol, tetrahydrofuran, diethyl ether, dioxane, benzene, toluene and xylene. While

the reaction temperature varies depending on the kind of solvent to be used, it is generally 0 - 40°C, and the reaction time which varies depending on the reaction temperature is generally 1-24 hr.

Method 18 : Of the intermediate compounds (IV) shown in Method 1, the compound of the following formula

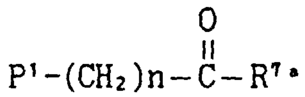

(XXXII)

wherein $R^{7a}$ is phenyl or substituted phenyl and other symbols are as defined above, can be synthesized by the following route.

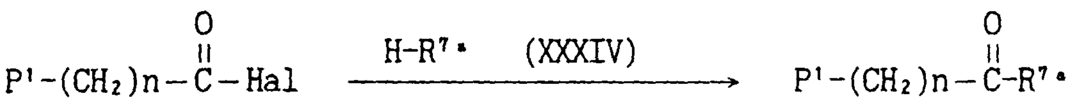


(XXXIII) (XXXII)

wherein each symbol is as defined above.

That is, compound (XXXII) can be produced by subjecting compound (XXXIII) derived from carboxylic acid to Friedel-Crafts reaction with compound (XXXIV) in the presence of an acid.

The acid to be used for Friedel-Crafts reaction includes, for example, aluminum chloride, aluminum bromide, titanium chloride, sulfuric acid, zinc chloride, iron chloride, hydrogen fluoride and phosphoric acid. The organic solvent to be used for Friedel-Crafts reaction includes, for example, tetrahydrofuran, diethyl ether, ethylene glycol dimethyl ether, dimethylformamide, dimethyl sulfoxide, methylene chloride, 1,2-dichloroethane, chloroform, acetonitrile, nitromethane and carbon disulfide. The reaction may be carried out without solvent on demand. While the reaction temperature varies depending on the kind of solvent to be used, it is generally from -20°C to 100°C, and the reaction time which varies depending on the reaction temperature is generally 1-24 hr.

Method 19 : Of the intermediate compounds (IV) shown in Method 1, the compound of the following formula

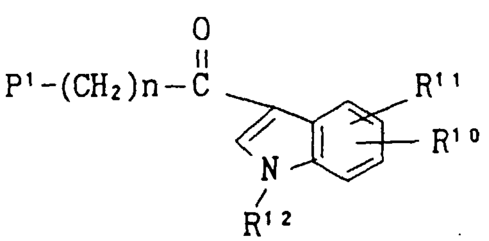

(XXXV)

wherein $R^{10}$ and $R^{11}$ are the same or different and each is hydrogen, halogen, lower alkyl, lower alkoxy, nitro and the like, $R^{12}$ is hydrogen, lower alkyl, cycloalkylalkyl, aralkyl and the like, and other symbols are as defined above, can be synthesized by the following route.

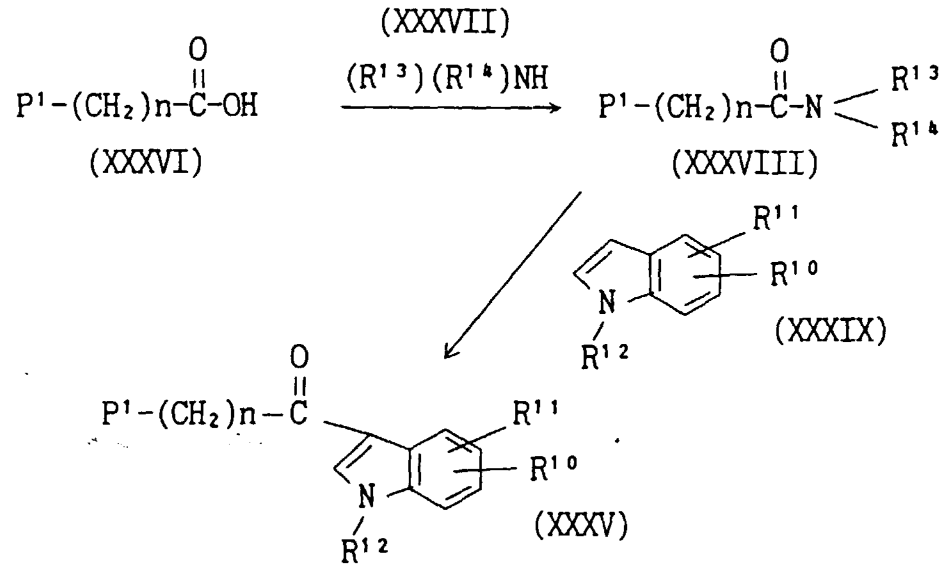

(XXXVI)
(XXXVIII)
(XXXIX)
(XXXV)

wherein $R^{13}$ and $R^{14}$ are respectively lower alkyl or form a ring such as piperidine and the like together with the adjacent nitrogen atom, and other symbols are as defined above.

That is, compound (XXXV) can be produced by reacting compound (XXXVII) and a carboxylic acid represented by compound (XXXVI) or its reactive derivative in a suitable solvent to give compound (XXXVIII) and reacting this compound with compound (XXXIX) in a suitable solvent in the presence of phosphorus oxychloride.

When compound (XXXVI) is a free carboxylic acid, the reaction is carried out using a routine condensing agent. Examples of the condensing agent include, for example, dicyclohexylcarbodiimide, 1-ethyl-3-(3-dimethylaminopropyl)carbodiimide hydrochloride, carbonyldiimidazole and N-methyl-2-chloropyridinium iodide. This reaction is preferably carried out in the presence of an organic base such as 1-hydroxybenzotriazole, N-methylmorpholine and the like. The solvent to be used includes, for example, dimethylformamide, dimethyl sulfoxide, methylene chloride, tetrahydrofuran, acetonitrile, dioxane and benzene. While the reaction temperature varies depending on the kind of solvent to be used, it is generally from -20°C to 50°C, and the reaction time which varies depending on the reaction temperature is generally 1-24 hr.

When compound (XXXVI) is a reactive derivative of carboxylic acid, such as acid halide, acid anhydride, mixed acid anhydride and ester, the condensation is generally carried out in a suitable solvent in the presence of a base as necessary. Examples of the base to be used as necessary include, for example, sodium carbonate, potassium carbonate, sodium hydrogencarbonate, potassium hydrogencarbonate, sodium hydroxide, potassium hydroxide, triethylamine, diisopropylethylamine and pyridine. The solvent to be used includes, for example, methylene chloride, chloroform, ethyl acetate, diethyl ether, tetrahydrofuran, dioxane, acetonitrile, benzene, toluene and mixed solvents thereof. While the reaction temperature varies depending on the kind of solvent to be used, it is generally from -30°C to 50°C, and the reaction time which varies depending on the reaction temperature is generally 1-24 hr.

The reaction of compound (XXXVIII) and compound (XXXIX) is carried out according to the Vilsmeier reaction described in Journal of Organic Chemistry, vol. 30, p. 2534 (1965). The solvent to be used for the reaction includes, for example, chloroform, methylene chloride and dichloroethane. While the reaction temperature varies depending on the kind of solvent to be used, it is generally from room temperature to refluxing temperature of the solvent, and the reaction time which varies depending on the reaction temperature is generally 1-12 hr.

Method 20 : Of the intermediate compounds (IV) shown in Method 1, the compound of the following formula

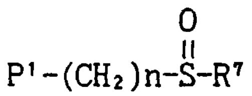

(XL-a)

or

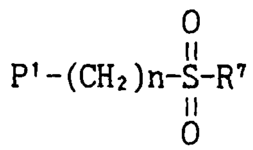

(XL-b)

wherein each symbol is as defined above, can be synthesized by the following route.

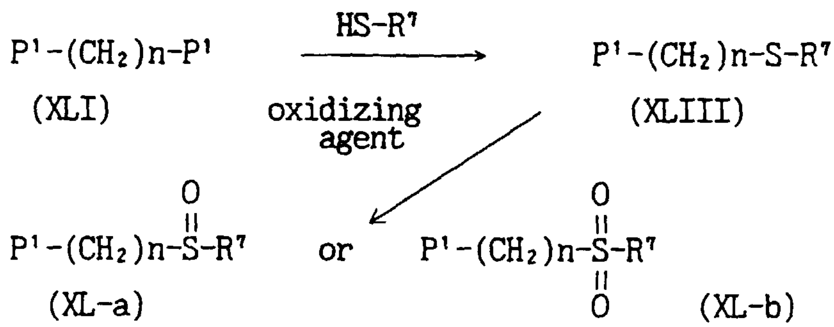


(XLI) (XLIII) (XL-a) or (XL-b)

wherein each symbol is as defined above.

That is, compound (XL-a) or compound (XL-b) can be produced by reacting compound (XLI) with compound (XLII) in a suitable solvent in the presence of a base to give compound (XLIII) and oxidizing the compound using 1 or 2 equivalent(s) of an oxidizing agent in the presence of an acid as necessary.

The solvent to be used for the reaction of compound (XLI) and compound (XLII) includes, for example, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene and xylene. Examples of the base to be used include, for example, sodium hydride, sodium carbonate, potassium carbonate, sodium hydrogencarbonate, potassium hydrogencarbonate, sodium hydroxide, potassium hydroxide, triethylamine, diisopropylethylamine and pyridine. While the reaction temperature varies depending on the kind of solvent to be used, it is generally from 0°C to 140°C, and the reaction time which varies depending on the reaction temperature is generally 1-24 hr.

The oxydizing agent to be used for the oxidation includes, for example, ruthenium tetraoxide, chromic acid, permanganate, m-chloroperbenzoic acid and aqueous hydrogen peroxide solution. The solvent to be used for oxidation includes, for example, formic acid, acetic acid, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene and xylene. Examples of the acid to be used as necessay include, for example, hydrochloric acid, sulfuric acid, p-toluenesulfonic acid and benzoic acid. While the reaction temperature varies depending on the kind of oxidizing agent to be used, it is generally from 0°C to 140°C, and the reaction time which varies depending on the reaction temperature is generally 1-24 hr.

The optical isomer of the compound of the present invention wherein Y is a formula of (II-b) can be produced from, for example, the optically active carboxylic acid ester obtained by the method described in Journal of Medicinal Chemistry, vol. 33, pp. 71-77 (1990), or by the route shown in the following Method 21.

Method 21 : A compound (XXIII) wherein, for example, m is 1, Y is a group of the formula (II-b), $R^3$ is hydrogen, $R^8$ is methyl, and the absolute configuration at the 3-position of pyrrolidine is R-configuration can be synthesized by the following route.

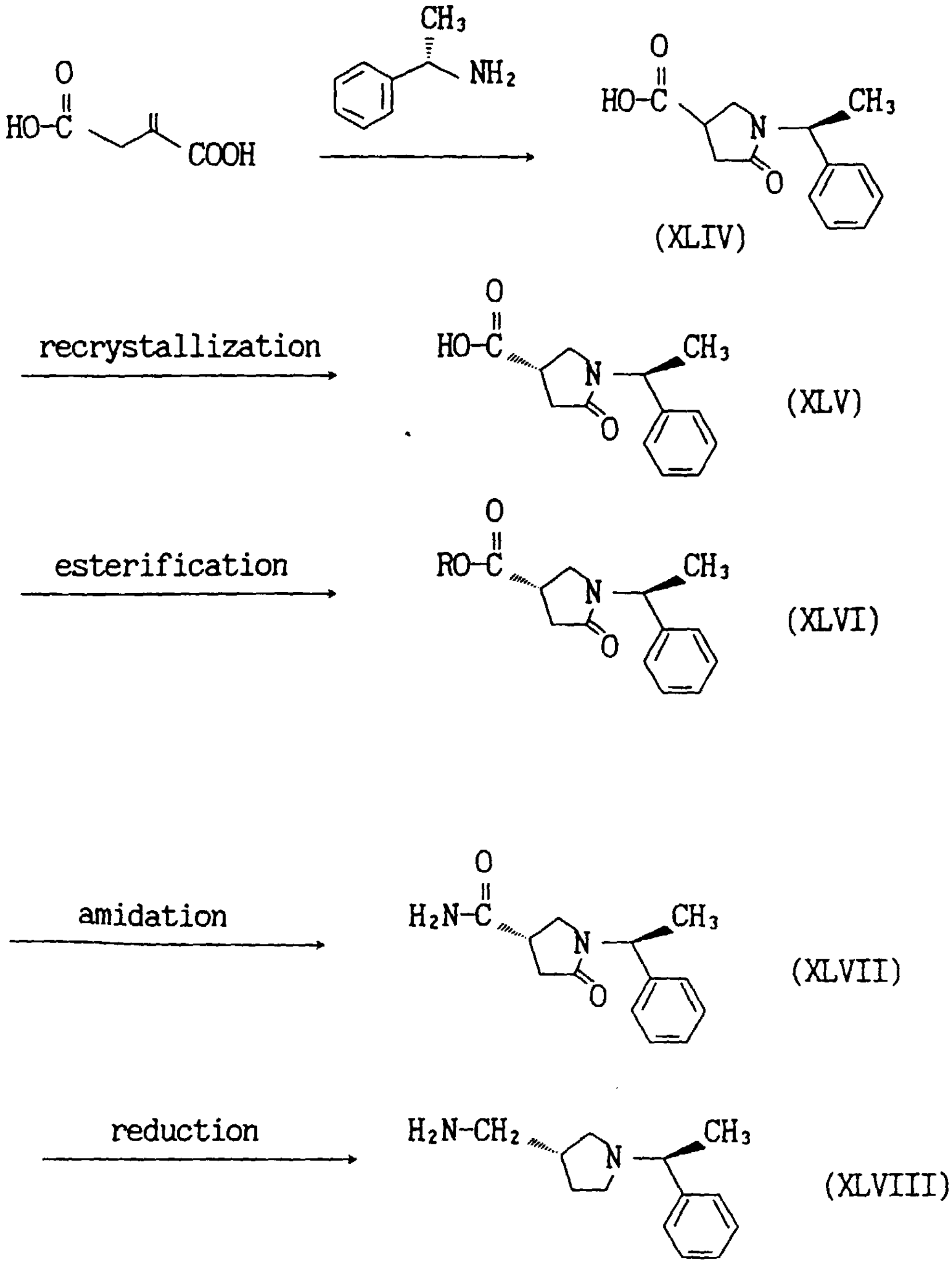


(XLIV)

(XLV)

(XLVI)

(XLVII)

(XLVIII)

wherein R is lower alkyl.

That is, itaconic acid is used as a starting compound and reacted with (S)-1-phenylethylamine without solvent or in a suitable solvent to give compound (XLIV), followed by repetitive recrystallization in a suitable solvent to give an optically pure diastereomer compound (XLV). This compound is reacted in an alcohol solvent in the presence of an acid catalyst to give compound (XLVI), which is subjected to amidation in a suitable solvent in the presence of ammonia gas to give compound (XLVII), followed by reaction with a suitable reducing agent in a suitable solvent to give compound (XLVIII).

The solvent to be used for the reaction of itaconic acid and (S)-1-phenylethylamine is exemplified by methanol, ethanol, propanol, acetone, ethyl acetate, benzene, toluene, tetrahydrofuran, dioxane, 1,3-dimethylimidazolidinone, dimethylformamide, dimethyl sulfoxide and the like. While the reaction temperature varies depending on the kind of solvent to be used, it is generally from 30°C to the refluxing temperature of the solvent, and the reaction time which varies depending on the reaction temperature is generally 1-24 hr.

The solvent to be used for the recrystallization of compound (XLIV) is exemplified by water, methanol, ethanol, propanol, isopropyl alcohol, butanol, acetone, ethyl acetate, benzene, toluene, dioxane, mixed solvents thereof and the like.

The alcohol solvent to be used for the esterification of compound (XLV) is exemplified by methanol, ethanol, propanol, butanol and the like. The acid catalyst to be used may be, for example, hydrochloric acid, sulfuric acid, p-toluenesulfonic acid or thionyl chloride. The reaction temperature is generally from 0°C to the refluxing temperature of the solvent, and the reaction time which varies depending on the reaction temperature is generally 1-24 hr.

The solvent to be used for the amidation of compound (XLVI) includes, for example, methanol, ethanol, propanol, isopropyl alcohol, butanol, ethyl acetate, benzene, toluene and dioxane. The reaction temperature is generally from -20°C to 50°C, and the reaction time which varies depending on the reaction temperature is generally 1-24 hr.

The solvent to be used for the reduction of compound (XLVII) includes, for example, methanol, ethanol, propanol, isopropyl alcohol, butanol, benzene, toluene, tetrahydrofuran, dioxane and diethyl ether. The reducing agent to be used includes, for example, lithium aluminum hydride, diborane, diisobutylaluminum hydride, sodium borohydride-sulfuric acid and sodium borohydride-boron trifluoride. The reaction temperature is generally from -30°C to the refluxing temperature of the solvent, and the reaction time which varies depending on the reaction temperature is generally 1-24 hr.

The compound wherein the absolute configuration at the 3-position of pyrrolidine is S-configuration can be synthesized in the same manner as above using itaconic acid and (R)-1-phenylethylamine as starting compounds.

The compound of the formula (I) thus obtained can be separated and purified from reaction mixtures by a method known *per se*, such as recrystallization and column chromatography.

When the compound of the formula (I) of the present invention and pharmaceutically acceptable salts thereof have asymmetric carbon, they are generally produced as racemates which can be optically resolved into optical isomers by a conventional method such as preparative recrystallization and chromatography. Also, the use of optically active starting compound results in optical isomers. When a compound has two or more asymmetric carbons, they can be obtained as individual diastereoisomers or mixtures thereof which can be separated by a conventional method such as preparative recrystallization and chromatography.

The compound of the formula (I) can be converted to acid addition salt by treating the compound with an acid such as hydrochloric acid, sulfuric acid, hydrobromic acid, phosphoric acid, nitric acid, methanesulfonic acid, ethanesulfonic acid, fumaric acid, maleic acid, benzoic acid, citric acid, malic acid, mandelic acid, p-toluenesulfonic acid, acetic acid, succinic acid, malonic acid, lactic acid, salicylic acid, gallic acid, piclinic acid, carbonic acid, ascorbic acid, trifluoroacetic acid and tartaric acid in a suitable solvent such as methanol and ethanol.

The compound of the formula (I) can be converted to N-oxide compound by oxidation using 1 to 10 equivalents, preferably from monoequivalent to a slightly excess, of an oxidizing agent such as m-chloroperbenzoic acid, perbenzoic acid, peracetic acid, trifluoroperacetic acid, permaleic acid, sodium bromite, sodium hypochlorite and hydrogen peroxide, in a suitable solvent such as chloroform, methylene chloride, tetrahydrofuran, dioxane, dimethylformamide, acetic acid, water and mixed solvents thereof, at -50°C to room temperature for 5 min to 24 hr. This oxidation reaction can be also carried out in the presence of a catalyst such as sodium tungstate.

When the crystals of the obtained compound of the present invention are anhydrides, the crystals can be converted to hydrates or solvates by a treatment with water, aqueous solvent or other solvent.

The compounds of the present invention, optical isomers thereof and pharmaceutically acceptable salts thereof have selective and high affinity for 5-$HT_4$ receptors, as well as activates same, and are useful as pharmaceutical agents for the prophylaxis and treatment of gastrointestinal diseases (e.g., reflux esophagitis; gastroesophageal reflux such as that accompanying cystic fibrosis; Barrett syndrome; intestinal pseudoileus; acute or chronic gastritis; gastric or duodenal ulcer; Crohn's disease; non-ulcer dyspepsia; ulcerative colitis; postgastrectomy syndrome; postoperative digestive function failure; delayed gastric emptying caused by gastric neurosis, gastroptosis, diabetes, and the like; gastrointestinal disorders such as indigestion, meteorism, abdominal indefinite complaint, and the like; constipation such as atonic constipation, chronic constipation, and that caused by spinal cord injury, pelvic diaphragm failure and the like; and irritable bowel syndrome); central nervous disorders (e.g., schizophrenia, depression, anxiety, disturbance of memory and dementia); cardiac function disorders (e.g., cardiac failure and myocardial ischemia); and urinary diseases (e.g., dysuria caused by urinary obstruction, ureterolith, prostatomegaly, spinal cord injury, pelvic diaphragm failure, etc) and the like. In addition, since they possess anti-nociceptive action, they are useful as anti-nociceptive agents for pain relief which increase the threshold value of pain.

The compounds of the present invention, optical isomers thereof and pharmaceutically acceptable salts thereof can be administered orally or parenterally by inhalation, rectal administration or local administration. They can be used as pharmaceutical compositions or pharmaceutical agents such as powders, granules, tablets, pills, capsules, injections, syrups, emulsions, elixirs, suspensions and solutions, wherein at least one inventive compound is used solely or in combination with pharmaceutically acceptable carriers (e.g., excipients, binders, disintegrators, correctives, corrigents, emulsifying agents, diluents and/or solubilizers).

The pharmaceutical composition can be formulated into a pharmaceutical preparation by a conventional method. In the present specification, "parenteral" includes subcutaneous injection, intravenous injection, intramuscular injection, intraperitoneal injection and transfusion. The preparation for injection, such as a sterile aqueous or oily suspension for injection, can be prepared using a suitable dispersing agent, a wetting agent and a suspending agent, according to a method known in the pertinent field. The sterile preparation for injection may be a sterile injectable solution or suspension in a non-toxic diluent or solvent permitting parenteral administration, such as an aqueous solution. Examples of the vehicle and solvent which can be used include water, Ringer solution, isotonic brine and the like. In addition, sterile nonvolatile oil can be generally used as a solvent or a solvent for suspension. For this end, any nonvolatile oil or fatty acid can be used, inclusive of natural, synthetic or semi-synthetic fatty oil or fatty acid, and natural, synthetic or semi-synthetic mono, di or triglycerides. The suppository for rectal administration can be produced by mixing the drug with suitable non-irritative excipient, such as cocoa butter and polyethylene glycols which are solid at ordinary temperature, liquid at the temperature of the intestinal tract and melted in the rectum to release the drug. The solid dosage form for oral administration includes the above-mentioned ones such as powders, granules, tablets, pills, capsules and the like. In these dosage forms, the active ingredient is admixed with at least one additive such as sucrose, lactose, cellulose sugar, mannitol, maltitol, dextran, starches, agar, arginates, kichins, chitosans, pectins, tragacanth gums, gum arabic, gelatins, collagens, casein, albumin, synthetic or semi-synthetic polymers and glycerides. In these dosage forms, routine additives can be added, which may be inert diluents, lubricants such as magnesium stearate, preservatives such as p-hydroxybenzoate, sorbic acid, antioxidants such as ascorbic acid, $\alpha$-tocopherol and cysteine, disintegrators, binders, tackifiers, buffers, sweeteners, flavors, perfumes and the like. An enteric coating may be applied to tablets and pills. The liquid agents for oral administration may be pharmaceutically acceptable emulsions, syrups, elixirs, suspensions, solutions and the like, which may contain inert diluents (e.g., water), which are generally used in the pertinent field.

While the dose for a certain patient is determined according to age, body weight, general health conditions, sex, diet, administration time, administration route, clearance rate, combination of drugs, the state of the disease for which the patient is then undergoing treatments, and other factors. The compound of the present invention, optical isomers thereof and pharmaceutically acceptable salts thereof show low toxicity and can be used safely. While the daily dose varies depending on the condition and body weight of the patient, the kind of compound, administration route and the like, it is, for example, about 0.01-50 mg/person/day, preferably 0.01-20 mg/person/day, for parenteral administration by a subcutaneous, intravenous, intramuscular or intrarectal route, and about 0.01-150 mg/person/day, preferably 0.1-100 mg/person/day, for oral administration.

## Best Mode for Embodying the Invention

The present invention is specifically described by Preparation Examples, Examples and Formulation Examples, to which the invention is not limited.

### Preparation Example 1

4-(Aminomethyl)piperidine (137 g) was dissolved in toluene (1200 ml), and benzaldehyde (127 g) was added, and the mixture was stirred at refluxing temperature for 6.5 hr. The reaction mixture was cooled to room temperature, and di-tert-butyl dicarbonate (288 g) was dropwise added, and the mixture was stirred at room temperature for 1 hr. The reaction mixture was concentrated under reduced pressure, and 1N potassium hydrogensulfate (1200 ml) was added to the obtained residue, which was followed by stirring at room temperature for 4 hr. The reaction mixture was washed 3 times with isopropyl ether and made alkaline with 10% sodium hydroxide solution. The mixture was extracted with chloroform. The organic layer was washed with saturated aqueous sodium chloride solution, dried over sodium sulfate and concentrated under reduced pressure to give 236.5 g of 4-aminomethyl-1-(tert-butoxycarbonyl)piperidine.

$^1$H-NMR ($CDCl_3$,ppm)$\delta$:1.01-1.30(5H,m), 1.45(9H,s), 1.65-1.75(2H,m), 4.07-4.16(2H,m)

### Preparation Example 2

To a solution of 4-aminomethyl-1-(tert-butoxycarbonyl)piperidine (236.5 g) in dimethylformamide (1200 ml) were added 4-amino-5-chloro-2-methoxybenzoic acid (130 g) and 1-hydroxybenzotriazole (91.4 g). 1-Ethyl-3-(3-dimethylaminopropyl)carbodiimide hydrochloride (129.6 g) was added under ice-cooling, and the mixture was stirred at room temperature for 1 hr. The reaction mixture was concentrated under reduced pressure, and water was added to the obtained residue, which was followed by extraction with chloroform. The organic layer was washed successively with 10% aqueous potassium carbonate solution and saturated aqueous sodium chloride solution and dried. The solvent was evaporated under reduced pressure and the obtained residue was purified by silica gel column chromatography

(chloroform) to give 272 g of 4-amino-5-chloro-2-methoxy-N-((1-tert-butoxycarbonyl)piperidin-4-ylmethyl)benzamide.

$^1$H-NMR ($CDCl_3$,ppm)δ:1.10-1.27(2H,m), 1.45(9H,s), 1.65-1.85(3H,m), 2.62- 2.75(2H,m), 3.27-3.37(2H,m), 3.88(3H,s), 4.61(2H,s), 6.34(1H,s), 7.72-7.82(1H,m), 8.07(1H,s)

Preparation Example 3

4.25 M Hydrochloric acid-dioxane (1000 ml) was dropwise added to a solution (600 ml) of 4-amino-5-chloro-2-methoxy-N-((1-tert-butoxycarbonyl)piperidin-4-ylmethyl)benzamide (272 g) in dioxane, and the mixture was stirred at room temperature for 2 hr. The precipitated crystals were collected by filtration and recrystallized from methanolisopropyl ether to give 211 g of 4-amino-5-chloro-2-methoxy-N-(piperidin-4-ylmethyl)benzamide dihydrochloride.

m.p. 235-239°C

Preparation Example 4

N-(5-Bromopentyl)phthalimide (60 g) was added to a solution of 4-amino-5-chloro-2-methoxy-N-(piperidin-4-ylmethyl)benzamide dihydrochloride (75.1 g) and potassium carbonate (112 g) in dimethylformamide (800 ml), and the mixture was stirred at 75-80°C for 8 hr. Inorganic matter was filtered off and the solvent was evaporated under reduced pressure. The obtained residue was purified by silica gel column chromatography (chloroform:methanol=10:1) to give 60.2 g of 4-amino-5-chloro-N-(1-(5-(2,3-dihydro-1,3-dioxo-1H-isoindol-2-yl)pentyl)piperidin-4-ylmethyl)-2-methoxybenzamide.

m.p. 58-61°C

Preparation Example 5

4-Amino-5-chloro-2-methoxy-N-(piperidin-4-ylmethyl)benzamide dihydrochloride (11.1 g) and N-(3-bromopropyl)phthalimide (8.0 g) were reacted and treated in the same manner as in Preparation Example 4 to give 7.7 g of 4-amino-5-chloro-N-(1-(3-(2,3-dihydro-1,3-dioxo-1H-isoindol-2-yl)propyl)piperidin-4-ylmethyl)-2-methoxybenzamide.

$^1$H-NMR ($CDCl_3$,ppm)δ:1.03-1.95(9H,m), 2.39(2H,t,J=7.3Hz), 2.83-2.90(2H,m), 3.21(2H,t,J=6.6Hz), 3.75(2H,t,J=7.3Hz), 3.89(3H,s), 4.41(2H,s), 6.29(1H,s), 7.66-7.75(3H,m), 7.80-7.86(2H,m), 8.09(1H,s)

Preparation Example 6

4-Amino-5-chloro-2-methoxy-N-(piperidin-4-ylmethyl)benzamide dihydrochloride (17.0 g) and N-(4-bromobutyl)phthalimide (15.8 g) were reacted and treated in the same manner as in Preparation Example 4 to give 9.3 g of 4-amino-5-chloro-N-(1-(4-(2,3-dihydro-1,3-dioxo-1H-isoindol-2-yl)butyl)piperidin-4-ylmethyl)-2-methoxybenzamide.

$^1$H-NMR ($CDCl_3$,ppm)δ:1.23-1.38(2H,m), 1.47-1.75(7H,m), 1.84-1.96(2H,m), 2.29-2.38(2H,m), 2.86-2.95(2H,m), 3.31(2H,t,J=6.6Hz), 3.70(2H,t,J=6.6Hz), 3.89(3H,s), 4.42(2H,s), 6.29(1H,s), 7.67-7.76(3H,m), 7.80-7.84(2H,m), 8.09(1H,s)

Preparation Example 7

4-Amino-5-chloro-2-methoxy-N-(piperidin-4-ylmethyl)benzamide dihydrochloride (17.0 g) and N-(6-bromohexyl)phthalimide (17.4 g) were reacted and treated in the same manner as in Preparation Example 4 to give 17.0 g of 4-amino-5-chloro-N-(1-(6-(2,3-dihydro-1,3-dioxo-1H-isoindol-2-yl)hexyl)piperidin-4-ylmethyl)-2-methoxybenzamide.

$^1$H-NMR ($CDCl_3$,ppm)δ:1.23-1.81(13H,m), 2.03-2.15(2H,m), 2.40-2.48(2H,m), 3.02-3.09(2H,m), 3.32(2H,t,J=5.9Hz), 3.67(2H,t,J=6.6Hz), 3.90(3H,s), 4.45(2H,s), 6.32(1H,s), 7.67-7.87(5H,m), 8.08(1H,s)

Preparation Example 8

60% Sodium hydride (1.4 g) was suspended in dimethylformamide (40 ml), and phenol (3 g) was added under ice-coolng with stirring. The mixture was stirred at room temperature for 1 hr. The mixture was again ice-cooled, and 1-bromo-5-chloropentane (5.9 g) was added, which was followed by stirring at room temperature for 1.5 hr. Water was

added to the reaction mixture and the mixture was extracted with ethyl acetate. The organic layer was washed with saturated brine, dried and the solvent was evaporated under reduced pressure to give 5.9 g of 5-phenoxypentyl chloride.

$^1$H-NMR ($CDCl_3$,ppm)δ:1.55-1.67(2H,m), 1.75-1.89(4H,m), 3.55(2H,t,J=6.6Hz), 3.95(2H,t,J=6.6Hz), 6.86-6.95(3H,m), 7.22-7.31(2H,m)

Preparation Example 9

60% Sodium hydride (1.33 g) was suspended in dimethylformamide (40 ml), and benzyl alcohol (3 g) was added under ice-cooilng with stirring. The mixture was stirred at room temperature for 1 hr. The mixture was again ice-cooled, and 1,5-dibromopentane (6.4 g) was added, which was followed by stirring at room temperature for 3 hr. Water was added to the reaction mixture and the mixture was extracted with ethyl acetate. The organic layer was washed with saturated brine, dried and the solvent was evaporated under reduced pressure to give 7.2 g of 5-benzyloxypentyl bromide.

$^1$H-NMR ($CDCl_3$,ppm)δ:1.48-1.91(6H,m), 3.37-3.51(4H,m), 4.49(2H,s), 7.22-7.37(5H,m)

Preparation Example 10

Potassium carbonate (5 g) and 1-bromo-5-chloropentane (4.9 g) were added to a solution of benzylmercaptan (3 g) in dimethylformamide (40 ml), and the mixture was stirred at 70-80°C for 2 hr. Water was added to the reaction mixture and the mixture was extracted with ethyl acetate. The organic layer was washed with saturated brine, dried and the solvent was evaporated under reduced pressure to give 3 g of 5-benzylthiopentyl chloride.

$^1$H-NMR ($CDCl_3$,ppm)δ:1.42-1.91(6H,m), 2.41(2H,t,J=7.3Hz), 3.49(2H,t,J=6.6Hz), 3.70(2H,s), 7.19-7.37(5H,m)

Preparation Example 11

6-Chlorohexyl alcohol (1.4 g), N-methylaniline (1.1 g) and sodium carbonate (1.1 g) were heated at 120°C for 9 hr. Water was added to the reaction mixture and the mixture was extracted with ethyl acetate. The organic layer was washed with saturated brine, dried and the solvent was evaporated under reduced pressure. The obtained residue was purified by silica gel column chromatography to give 1.1 g of 6-(N-methy-N-phenylamino)hexyl alcohol.

$^1$H-NMR ($CDCl_3$,ppm)δ:1.31-1.42(4H,m), 1.51-1.63(4H,m), 2.91(3H,s), 3.30(2H,t,J=7.9Hz), 3.63(2H,t,J=6.6Hz), 6.64-6.70(3H,m), 7.18-7.21(2H,m)

6-(N-Methy-N-phenylamino)hexyl alcohol (1.1 g) and triphenylphosphine (1.3 g) were dissolved in methylene chloride (40 ml), and N-chlorosuccinylimide (0.71 g) was added, which was followed by stirring at room temperature for 1 hr 20 min. The organic layer was washed with saturated brine, dried and the solvent was evaporated under reduced pressure. The obtained residue was purified by silica gel column chromatography to give 0.90 g of 6-(N-methy-N-phenylamino)hexyl chloride.

$^1$H-NMR ($CDCl_3$,ppm)δ:1.31-1.64(6H,m), 1.72-1.82(2H,m), 2.91(3H,s), 3.00(2H,t,J=7.3Hz), 3.52(2H,t,J=6.6Hz), 6.69(3H,d,J=8.0Hz), 7.21(2H,dd,J=1.3,7.2Hz)

Preparation Example 12

60% Sodium hydride (1.16 g) was suspended in dimethylformamide (40 ml), and phenol (3 g) was added under ice-cooilng with stirring. The mixture was stirred at room temperature for 1 hr. The mixture was again ice-cooled, and a solution of 1,6-dibromohexane (7.78 g) in dimethylformamide (10 ml) was dropwise added, which was followed by stirring at room temperature for 2 hr. Water was added to the reaction mixture and the mixture was extracted with ethyl acetate. The organic layer was washed successively with water and saturated brine, dried and the solvent was evaporated under reduced pressure to give 8.2 g of 6-phenoxyhexyl bromide.

$^1$H-NMR ($CDCl_3$,ppm)δ:1.42-1.58(4H,m), 1.70-1.93(4H,m), 3.36-3.42(2H,m), 3.92-3.98(2H,m), 6.86-6.94(3H,m), 7.26(2H,t,J=7.3Hz)

Preparation Example 13

60% Sodium hydride (1.2 g) was suspended in tetrahydrofuran (30 ml), and benzyl alcohol (3.0 g) was added under ice-cooling with stirring. The mixture was stirred at room temperature for 30 min, and again ice-cooled. 1,4-Dibromobutane (6.0 g) was added and the mixture was stirred for 3 hr. Water was added to the reaction mixture and the mixture was extracted with ethyl acetate. The organic layer was washed with saturated brine, dried and the solvent was evaporated under reduced pressure to give 3.8 g of 4-benzyloxybutyl bromide.

$^1$H-NMR ($CDCl_3$,ppm)δ:2.00-2.06(4H,m), 3.40-3.46(4H,m), 4.67(2H,s), 7.27-7.36(5H,m)

Preparation Example 14

4-Chlorobenzyl alcohol (4.3 g), 60% sodium hydride (1.8 g) and 1-bromo-5-chloropentane (6.0 g) were reacted and treated in the same manner as in Example 13 to give 3.6 g of 5-(4-chlorobenzyloxy)pentyl chloride.

$^1$H-NMR ($CDCl_3$,ppm)δ:0.80-0.92(2H,q), 1.43-1.72(4H,m), 1.88-1.92(2H,m), 3.41-3.48(2H,m), 3.48(2H,s), 7.26(2H,dd), 7.30(2H,dd)

Preparation Example 15

60% Sodium hydride (1.16 g) was suspended in dimethylformamide (40 ml), and a solution of cyclohexanemethanol (3 g) in dimethylformamide (10 ml) was dropwise added under ice-cooling with stirring, which was followed by stirring at room temperature for 1 hr. The mixture was again ice-cooled, and a solution of 1-bromo-5-chloropentane (4.88 g) in dimethylformamide (10 ml) was dropwise added, which was followed by stirring at room temperature for 2.5 hr. Water was added to the reaction mixture and the mixture was extracted with ethyl acetate. The organic layer was washed successively with water and saturated brine, dried and the solvent was evaporated under reduced pressure. The obtained residue was purified by silica gel column chromatography (hexane:ethyl acetate=10:1) to give 2.9 g of 5-(cyclohexylmethoxy)pentyl bromide.

$^1$H-NMR ($CDCl_3$,ppm)δ:0.83-1.94(17H,m), 3.19(2H,d,J=6.6Hz), 3.39(2H,t,J=6.6Hz), 3.54(2H,t,J=7.3Hz)

Preparation Example 16

Potassium carbonate (5.64 g) and 1,4-dibromobutane (5.88 g) were added to a solution of thiophenol (3 g) in dimethylformamide (40 ml), and the mixture was stirred at 50°C for 3 hr. Water was added to the reaction mixture and the mixture was extracted with ethyl acetate. The organic layer was washed with saturated brine, dried and the solvent was evaporated under reduced pressure to give 6.67 g of 4-phenylthiobutyl bromide.

$^1$H-NMR ($CDCl_3$,ppm)δ:1.74-1.85(2H,m), 1.97-2.04(2H,m), 2.94(2H,t,J=6.6Hz), 3.40(2H,t,J=6.6Hz), 7.14-7.35(5H,m)

Preparation Example 17

Thiophenol (3 g), potassium carbonate (5.64 g) and 1,6-dibromohexane (6.64 g) were reacted and treated in the same manner as in Preparation Example 16 to give 7.4 g of 6-phenylthiohexyl bromide.

$^1$H-NMR ($CDCl_3$,ppm)δ:1.40-1.49(4H,m), 1.59-1.72(2H,m), 1.81-1.89(2H,m), 2.93(2H,t,J=6.6Hz), 3.40(2H,t,J=6.6Hz), 7.15-7.34(5H,m)

In the same manner as in the above-mentioned Preparation Example 9, the following compounds were obtained.

Preparation Example 18

6-Benzyloxyhexyl chloride
$^1$H-NMR ($CDCl_3$,ppm)δ:1.38-1.80(8H,m), 3.47(2H,t,J=6.3Hz), 3.53(2H,t,J=7.0Hz), 4.50(2H,s), 7.26-7.35(5H,m)

Preparation Example 19

5-(2-phenylethyloxy)pentyl chloride
$^1$H-NMR (CDCl$_3$,ppm)δ:1.41-1.85(6H,m), 1.88(2H,t,J=7.3Hz), 3.44(2H,t,J=6.3Hz), 3.51(2H,t,J=6.6Hz), 3.62(2H,t,J=7.2Hz), 7.19-7.31(5H,m)

Preparation Example 20

5-(2-naphthylmethoxy)pentyl chloride
$^1$H-NMR (CDCl$_3$,ppm)δ:1.49-1.93(6H,m), 3.49-3.55(4H,m), 4.66(2H,s), 7.42-7.84(7H,m)

In the same manner as in the above-mentioned Preparation Example 16, the following compounds were obtained.

Preparation Example 21

5-phenylthiopentyl bromide
$^1$H-NMR (CDCl$_3$,ppm)δ:1.49-1.89(6H,m), 2.92(2H,t,J=7.2Hz), 3.51(2H,t,J=6.6Hz), 7.13-7.34(5H,m)

Preparation Example 22

6-Bromohexanoyl chloride (16.2 g) was dissolved in methylene chloride (50 ml), and aluminum chloride (10.6 g) was added, which was followed by stirring at room temperature for 30 min. The reaction mixture was ice-cooled, and benzene (5.0 g) was dropwise added, which was followed by stirring for 4 hr. Water was added to the reaction mixture and the mixture was extracted with ethyl acetate. The organic layer was washed with saturated brine, dried and the solvent was evaporated under reduced pressure. The obtained residue was purified by silica gel column chromatography (hexane:ethyl acetate=3:1) to give 18.2 g of 6-bromo-1-phenyl-1-hexanone.

m.p. 35-36°C

Preparation Example 23

Aluminum chloride (1.23 g) was added to a solution of 7-bromoheptanoyl chloride (2 g) in benzene (20 ml) under ice-cooling, which was followed by stirring at room temperature for 1 hr. Ice water and conc. hydrochloric acid were added to the reaction mixture, and the mixture was extracted with ethyl acetate. The organic layer was washed with water and saturated brine, dried and the solvent was evaporated under reduced pressure. The obtained residue was purified by silica gel column chromatography to give 0.93 g of 7-bromo-1-phenyl-1-heptanone.

$^1$H-NMR (CDCl$_3$,ppm)δ:1.35-1.56(4H,m), 1.71-1.93(4H,m), 2.98(2H,t,J=7.3Hz), 3.41(2H,t,J=6.6Hz), 7.43-7.49(2H,m), 7.53-7.59(1H,m), 7.95(2H,d,J=7.3Hz)

Preparation Example 24

Aluminum chloride (1.74 g) was added to a solution of 8-bromooctanoyl chloride (3 g) in benzene (20 ml) under ice-cooling, and the mixture was stirred at room temperature for 1.5 hr. Ice water and conc. hydrochloric acid were added to the reaction mixture, and the mixture was extracted with ethyl acetate. The organic layer was washed with water and saturated brine, dried and the solvent was evaporated under reduced pressure. The obtained residue was purified by silica gel column chromatography to give 1.36 g of 8-bromo-1-phenyl-1-octanone.

$^1$H-NMR (CDCl$_3$,ppm)δ:1.30-1.51(6H,m), 1.70-1.91(4H,m), 2.97(2H,t,J=7.3Hz), 3.40(2H,t,J=6.6Hz), 7.43-7.49(2H,m), 7.52-7.58(1H,m), 7.95(2H,d,J= 7.3Hz)

Preparation Example 25

Aluminum chloride (1.3 g) was added to a solution of 6-bromohexanoyl chloride (2 g) in toluene (20 ml) under ice-cooling, and the mixture was stirred at room temperature for 2 hr. Ice water and conc. hydrochloric acid were added to the reaction mixture, and the mixture was extracted with ethyl acetate. The organic layer was washed with water and saturated brine, dried and the solvent was evaporated under reduced pressure to give 2.76 g of 6-bromo-1-(4-methylphenyl)-1-hexanone.

$^1H$-NMR ($CDCl_3$,ppm)δ:1.47-1.58(2H,m), 1.74-1.82(2H,m), 1.86-1.97(2H,m), 2.41(3H,s), 2.96(2H,t,J=7.3Hz), 3.42(2H,t,J=6.6Hz), 7.25(2H,d,J=8.6Hz), 7.85(2H,d,J=8.6Hz)

Preparation Example 26

6-Bromohexanoyl chloride (2.0 g) was added to o-xylene (20 ml), and aluminum chloride (1.4 g) was added at -20°C, which was followed by stirring for 30 min. Water was added to the reaction mixture and the mixture was extracted with ethyl acetate. The organic layer was washed with saturated brine, dried and the solvent was evaporated under reduced pressure to give 2.6 g of 6-bromo-1-(3,4-dimethylphenyl)-1-hexanone.

m.p. 61-63°C

Preparation Example 27

Anisole (1.16 g), 6-bromohexanoyl chloride (2.3 g) and aluminum chloride (1.5 g) were reacted and treated in the same manner as in Preparation Example 22 to give 3.07 g of 6-bromo-1-(4-methoxyphenyl)-1-hexanone.

m.p. 40-41°C

Preparation Example 28

6-Bromohexanoyl chloride (1.83 g), 1,2-dimethylcatechol (1.08 g) and aluminum chloride (1.83 g) were reacted and treated in the same manner as in Preparation Example 22 to give 2.62 g of 6-bromo-1-(3,4-dimethoxyphenyl)-1-hexanone.

m.p. 38-40°C

Preparation Example 29

Aluminum chloride (1.3 g) was added to a solution of 6-bromohexanoyl chloride (2 g) in chlorobenzene (20 ml) under ice-cooling and the mixture was stirred at room temperature for 2 hr. Ice water and conc. hydrochloric acid were added to the reaction mixture, and the mixture was extracted with ethyl acetate. The organic layer was washed with water and saturated brine, dried and the solvent was evaporated under reduced pressure. The obtained residue was purified by silica gel column chromatography to give 1.38 g of 6-bromo-1-(4-chlorophenyl)-1-hexanone.

$^1H$-NMR ($CDCl_3$,ppm)δ:1.47-1.59(2H,m), 1.71-1.82(2H,m), 1.87-1.97(2H,m), 2.96(2H,t,J=6.6Hz), 3.43(2H,t,J=6.6Hz), 7.43(2H,d,J=8.6Hz), 7.89(2H,d,J=8.6Hz)

Preparation Example 30

7-Bromoheptanoyl chloride (3.2 g) was added to chlorobenzene (30 ml), and aluminum chloride (1.6 g) was added under ice-cooling, which was followed by stirring for 3 hr. Water was added to the reaction mixture and the mixture was extracted with ethyl acetate. The organic layer was washed with saturated brine, dried and the solvent was evaporated under reduced pressure. The obtained residue was purified by silica gel column chromatography to give 2.2 g of 7-bromo-1-(4-chlorophenyl)-1-heptanone.

$^1H$-NMR ($CDCl_3$,ppm)δ:1.24-1.92(4H,m), 2.30(2H,t,J=7.2Hz), 2.94(2H,t,J=7.2Hz), 3.37-3.43(4H,m), 7.43(2H,dd,J=2.0,4.0Hz), 7.89(2H,dd,J=2.0,4.0Hz)

Preparation Example 31

Aluminum chloride (1.31 g) was added to a solution of 6-bromohexanoyl chloride (2 g) in 1,2-dichlorobenzene (20 ml) under ice-cooling and the mixture was stirred at 60-70°C for 1.5 hr. Ice water and conc. hydrochloric acid were added to the reaction mixture, and the mixture was extracted with ethyl acetate. The organic layer was washed with water and saturated brine, dried and the solvent was evaporated under reduced pressure. The obtained residue was purified by silica gel column chromatography to give 0.47 g of 6-bromo-1-(3,4-dichlorophenyl)-1-hexanone.

$^1H$-NMR ($CDCl_3$,ppm)δ:1.47-1.58(2H,m), 1.72-1.97(4H,m), 2.96(2H,t,J=7.3Hz), 3.42(2H,t,J=6.6Hz),

7.55(1H,d,J=8.6Hz), 7.78(1H,dd,J=2.0,8.6Hz), 8.03(1H,d,J=2.0Hz)

Preparation Example 32

Aluminum chloride (1.3 g) was added to a solution of 6-bromohexanoyl chloride (2 g) in fluorobenzene (20 ml) under ice-cooling and the mixture was stirred at room temperature for 2 hr. Ice water and conc. hydrochloric acid were added to the reaction mixture, and the mixture was extracted with ethyl acetate. The organic layer was washed with water and saturated brine, dried and the solvent was evaporated under reduced pressure to give 2.47 g of 6-bromo-1-(4-fluorophenyl)-1-hexanone.

$^1$H-NMR ($CDCl_3$,ppm)δ:1.48-1.59(2H,m), 1.72-1.83(2H,m), 1.87-1.97(2H,m), 2.97(2H,t,J=7.3Hz), 3.43(2H,t,J=6.6Hz), 7.10-7.16(2H,m), 7.95-8.01(2H,m)

Preparation Example 33

Aluminum chloride (1.31 g) was added to a solution of 6-bromohexanoyl chloride (2 g) in 1,3-difluorobenzene (20 ml) under ice-cooling and the mixture was stirred at room temperature for 2 hr. Ice water and conc. hydrochloric acid were added to the reaction mixture, and the mixture was extracted with ethyl acetate. The organic layer was washed with water and saturated brine, dried and the solvent was evaporated under reduced pressure. The obtained residue was purified by silica gel column chromatography to give 1.16 g of 6-bromo-1-(2,4-difluorophenyl)-1-hexanone.

$^1$H-NMR ($CDCl_3$,ppm)δ:1.46-1.59(2H,m), 1.72-1.79(2H,m), 1.86-1.97(2H,m), 2.94-3.00(2H,m), 3.43(2H,t,J=6.6Hz), 6.83-6.99(2H,m), 7.88-7.97(1H,m)

Preparation Example 34

6-Bromohexanoyl chloride (2.0 g), 2-chloroanisole (1.17 g) and aluminum chloride (1.12 g) were reacted and treated in the same manner as in Preparation Example 22 to give 0.86 g of 6-bromo-1-(3-chloro-4-methoxyphenyl)-1-hexanone.

m.p. 104-106°C

Preparation Example 35

6-Bromohexanoyl chloride (2.0 g), 2-fluoroanisole (1.18 g) and aluminum chloride (1.31 g) were reacted and treated in the same manner as in Preparation Example 22 to give 2.65 g of 6-bromo-1-(3-fluoro-4-methoxyphenyl)-1-hexanone.

m.p. 47-48°C

Preparation Example 36

6-Bromohexanoyl chloride (2.0 g), phenol (0.88 g) and aluminum chloride (1.4 g) were reacted and treated in the same manner as in Preparation Example 22 to give 0.51 g of 6-bromo-1-(4-hydroxyphenyl)-1-hexanone.

$^1$H-NMR ($CDCl_3$,ppm)δ:1.45-1.60(2H,m), 1.71-1.85(2H,m), 1.88-2.00(2H,m), 2.94(2H,t), 3.41(2H,t), 5.74(1H,s), 6.89(2H,d), 7.90(2H,d)

Preparation Example 37

6-Bromohexanoyl chloride (2.1 g) and thiophene (0.84 g) were dissolved in benzene (20 ml) and tin chloride (1.3 ml) was added under ice-cooling, which was followed by stirring for 1 hr. Water was added to the reaction mixture and the mixture was extracted with ethyl acetate. The organic layer was washed with saturated brine, dried and the solvent was evaporated under reduced pressure to give 3.1 g of 6-bromo-1-(2-thienyl)-1-hexanone.

$^1$H-NMR ($CDCl_3$,ppm)δ:1.46-2.08(6H,m), 2.93(2H,t,J=7.2Hz), 3.42(2H,t,J=7.2Hz), 7.13(1H,t,J=4.6Hz), 7.64(1H,d,J=4.6Hz), 7.73(1H,d,J=4.0Hz)

Preparation Example 38

1-Methyl-1H-indol (1.0 g) and N,N-dimethyl-5-bromopentylamide (1.7 g) were dissolved in chloroform (20 ml) and phosphorus oxychloride (0.85 ml) was added, which was followed by refluxing under heating for 7 hr. Saturated aqueous sodium hydrogencarbonate solution was added to the reaction mixture and the mixture was extracted with chloroform. The organic layer was washed with saturated brine, dried and the solvent was evaporated under reduced pressure to give 1.2 g of 6-bromo-1-(1-methyl-1H-indol-3-yl)-1-hexanone.

$^1$H-NMR ($CDCl_3$,ppm)δ:1.48-1.60(2H,m), 1.75-2.00(4H,m), 2.83-2.90(2H,t), 3.50(2H,dt), 3.82(3H,s), 7.27-7.32(3H,m), 7.69(1H,s), 8.35-8.39(1H,m)

Preparation Example 39

6-Bromohexanoyl chloride (2 g) and benzo[b]thiophene (1.26 g) were dissolved in methylene chloride (20 ml), and aluminum chloride (1.31 g) was added under ice-cooling, which was followed by stirring at room temperature for 1.5 hr. Ice water and conc. hydrochloric acid were added to the reaction mixture, and the mixture was extracted with ethyl acetate. The organic layer was washed with water and saturated brine, dried and the solvent was evaporated under reduced pressure. The obtained residue was purified by silica gel column chromatography and recrystallized from ethyl acetate-hexane to give 0.45 g of 6-bromo-1-(2-benzo[b]thienyl)-1-hexanone.

$^1$H-NMR ($CDCl_3$,ppm)δ:1.51-1.63(2H,m), 1.77-1.99(4H,m), 3.03(2H,t,J=7.3Hz), 3.43(2H,t,J=6.6Hz), 7.38-7.50(2H,m), 7.86-7.91(2H,m), 7.96(1H,s)

Preparation Example 40

The mother liquor of recrystallization of Preparation Example 39 was concentrated under reduced pressure. The obtained residue was recrystallized from ethyl acetate-hexane to give 0.42 g of 6-bromo-1-(3-benzo[b]thienyl)-1-hexanone.

$^1$H-NMR ($CDCl_3$,ppm)δ:1.51-1.62(2H,m), 1.77-1.99(4H,m), 3.02(2H,t,J=7.3Hz), 3.44(2H,t,J=6.6Hz), 7.39-7.52(2H,m), 7.87(1H,d,J=7.9Hz), 8.28(1H,s), 8.77(1H,d,J=7.9Hz)

Preparation Example 41

6-Bromohexanoyl chloride (2.0 g), 1,2-methylenedioxybenzene (1.18 g) and aluminum chloride (1.31 g) were reacted and treated in the same manner as in Preparation Example 22 to give 1.22 g of 6-bromo-1-(3,4-methylenedioxyphenyl)-1-hexanone.

$^1$H-NMR ($CDCl_3$,ppm)δ:1.46-1.98(6H,m), 2.91(2H,t,J=6.6Hz), 3.42(2H,t,J=7.3Hz), 6.03(2H,s), 6.85(1H,d,J=7.9Hz), 7.43(1H,d,J=2.0Hz), 7.56(1H,dd,J=2.0,7.9Hz)

Preparation Example 42

6-Bromohexanoyl chloride (1.8 g), naphthalene (1.0 g) and aluminum chloride (1.18 g) and carbon disulfide as solvent were reacted and treated in the same manner as in Preparation Example 22 to give 0.84 g of 6-bromo-1-(1-naphthyl)-1-hexanone as an oily substance.

$^1$H-NMR ($CDCl_3$,ppm)δ:1.47-1.98(6H,m), 3.07(2H,t,J=7.3Hz), 3.43(2H,t,J=6.6Hz), 7.46-7.61(3H,m), 7.82-7.99(3H,m), 8.55(1H,d)

Preparation Example 43

30% Aqueous hydrogen peroxide solution (27.6 ml) was dropwise added under ice-cooling to a solution of 4-phenylthiobutyl chloride (22.2 g) obtained in the sane manner as in Preparation Example 16 in formic acid (150 ml), which was followed by stirring at room temperature for 1 hr. The reaction mixture was poured into ice water, neutralized with sodium hydroxide solution and extracted with chloroform. The organic layer was washed with saturated brine, dried and the solvent was evaporated under reduced pressure. The obtained residue was purified by silica gel column chromatography (chloroform) and further recrystallized from hexane to give 21 g of 4-phenylsulfonylbutyl chloride.

m.p. 54-55°C

Preparation Example 44

Benzenethiol (3.0 g), 1-bromo-5-chloropentane (5.7 g) and potassium carbonate (4.15 g) were suspended in N,N-dimethylformamide (50 ml), and the mixture was stirred at 50°C for 5 hr. After cooling, water was added to the reaction mixture, and the mixture was extracted with ethyl acetate. The organic layer was washed with saturated brine, dried and the solvent was evaporated under reduced pressure. Thus, 5-phenylthiopentyl chloride was obtained. This compound was dissolved in formic acid (50 ml) and 2 equivalents of 30% aqueous hydrogen peroxide solution was added thereto, and the mixture was stirred at room temperature for 6 hr. Water was added to the reaction mixture, and the mixture was extracted with ethyl acetate. The organic layer was washed with saturated brine, dried and the solvent was evaporated under reduced pressure to give 2.11 g of 5-phenylsulfonylpentyl chloride.

$^1$H-NMR ($CDCl_3$,ppm)δ:1.47-1.84(6H,m), 3.09(2H,t,J=6.0Hz), 3.50(2H,t,J=6.6Hz), 7.55-7.94(5H,m)

Preparation Example 45

5-Phenylthiopentyl chloride (1.84 g) was dissolved in dichloromethane (50 ml), and one equivalent of m-chloroperbenzoic acid was added thereto, which was followed by stirring at room temperature for 6 hr. Water was added to the reaction mixture, and the mixture was extracted with ethyl acetate. The organic layer was washed with saturated brine, dried and the solvent was evaporated under reduced pressure. The obtained residue was purified by silica gel column chromatography to give 1.20 g of 5-phenylsulfinylpentyl chloride.

$^1$H-NMR ($CDCl_3$,ppm)δ:1.49-1.87(6H,m), 2.80(2H,t,J=6.0Hz), 3.51(2H,t,J=6.6Hz), 7.49-7.64(5H,m)

Preparation Example 46

1-Methyl-1H-indol (3.5 g), N,N-dimethyl-4-bromobutylamide (4.4 g) and phosphorus oxychloride (6 ml) were reacted and treated in the same manner as in Preparation Example 38 to give 3.73 g of 5-bromo-1-(1-methyl-1H-indol-3-yl)-1-pentanone.

$^1$H-NMR ($CDCl_3$,ppm)δ:1.84-2.00(4H,m), 2.87(2H,t,J=6.6Hz), 3.58(2H,t,J=5.9Hz), 3.84(3H,s), 7.28-7.35(3H,m), 7.71(1H,s), 8.35-8.39(1H,m)

Preparation Example 47

Tin tetrachloride (1.15 ml) was added to a solution of 6-bromohexanoyl chloride (2 g) and 2-methylbenzo[b]furan (1.24 g) in carbon disulfide (30 ml) under ice-cooling, the mixture was stirred at room temperature for 3 hr. Ice water and conc. hydrochloric acid were added to the reaction mixture, and the mixture was extracted with ethyl acetate. The organic layer was washed with water and saturated brine, dried and the solvent was evaporated under reduced pressure. The obtained residue was purified by silica gel column chromatography to give 2.2 g of 6-bromo-1-(2-methyl-3-benzo[b]furyl)-1-hexanone.

$^1$H-NMR ($CDCl_3$,ppm)δ:1.52-1.63(2H,m), 1.76-2.04(4H,m), 2.78(3H,s), 2.97(2H,t,J=7.3Hz), 3.44(2H,t,J=6.6Hz), 7.29-7.36(2H,m), 7.42-7.47(1H,m), 7.88-7.92(1H,m)

Preparation Example 48

A solution (30 ml) of 1-bromo-5-chloropentane (7.0 g) in tetrahydrofuran was gently added dropwise under refluxing to a suspension (30 ml) of magnesium (0.92 g) in tetrahydrofuran. After confirmation of disappearance of magnesium, a solution (30 ml) of 3-chlorobenzaldehyde (5.3 g) in tetrahydrofuran was dropwise added. After cooling, water was added to the reaction mixture, and the mixture was extracted with ethyl acetate. The organic layer was washed with saturated brine, dried and the solvent was evaporated under reduced pressure. The obtained residue was purified by silica gel column chromatography to give 6-bromo-1-(3-chlorophenyl)-1-hexanol, which was dissolved in chloroform (50 ml). Thereto was added 15 equivalents of manganese dioxide, and the mixture was stirred at room temperature for 48 hr. The reaction mixture was filtered, and the residue was purified by silica gel column chromatography to give 6-bromo-1-(3-chlorophenyl)-1-hexanone.

$^{1}$H-NMR ($CDCl_3$,ppm)δ:1.47-1.86(6H,m), 2.97(2H,t,J=7.3Hz), 3.56(2H,t,J=6.6Hz), 7.37-7.92(4H,m)

Preparation Example 49

1-Methyl-1H-indol (2.80 g) and N,N-dimethyl-6-bromohexylamide (1.56 g) were reacted and treated in the same manner as in Preparation Example 38 to give 0.94 g of 7-bromo-1-(1-methyl-1H-indol-3-yl)-1-heptanone as an oily substance.

$^{1}$H-NMR ($CDCl_3$,ppm)δ:1.35-1.99(8H,m), 2.85(2H,t,J=7.2Hz), 3.53(2H,t,J=7.0Hz), 3.85(1H,s), 7.28-7.35(3H,m), 7.72(1H,s), 8.37-8.39(1H,m)

Preparation Example 50

1H-Indole (2.3 g) and N,N-dimethyl-5-bromopentylamide (4.3 g) were dissolved in chloroform (40 ml) and phosphorus oxychloride (2.3 ml) was added, which was followed by refluxing under heating for 12 hr. Saturated aqueous sodium hydrogencarbonate solution was added to the reaction mixture and the mixture was extracted with chloroform. The organic layer was washed with saturated brine, dried and the solvent was evaporated under reduced pressure. The obtained residue was purified by silica gel column chromatography (hexane:ethyl acetate=5:1) to give 0.9 g of 6-bromo-1-(1H-indol-3-yl)-1-hexanone.

m.p. 156-158°C

Preparation Example 51

1-Ethyl-1H-indol (3.26 g) and N,N-dimethyl-5-bromopentylamide (5.0 g) were reacted and treated in the same manner as in Preparation Example 38 to give 2.77 g of 6-bromo-1-(1-ethyl-1H-indol-3-yl)-1-hexanone.

$^{1}$H-NMR ($CDCl_3$,ppm)δ:1.54(3H,t,J=7.2Hz), 1.76-1.95(6H,m), 2.87(2H,t,J=7.2Hz), 3.43(2H,t,J=6.5Hz), 4.22(2H,q,J=7.3Hz), 7.25-7.39(3H,m), 7.78(1H,s), 8.36-8.39(1H,m)

Preparation Example 52

1-Isopropyl-1H-indol (1.4 g), N,N-dimethyl-5-bromopentylamide (1.95 g) and phosphorus oxychloride (1.63 ml) were reacted and treated in the sane manner as in Preparation Example 38 to give 1.96 g of 6-bromo-1-(1-isopropyl-1H-indol-3-yl)-1-hexanone.

$^{1}$H-NMR ($CDCl_3$,ppm)δ:1.50-1.58(2H,m), 1.59(6H,d,J=6.6Hz), 1.77-1.99(4H,m), 2.90(2H,t,J=7.3Hz), 3.43(2H,t,J=6.6Hz), 4.71(1H,m), 7.26-7.33(2H,m), 7.37-7.42(1H,m), 7.87(1H,s), 8.35-8.40(1H,m)

Preparation Example 53

6-Bromohexanoyl chloride (2 g), 1,3-difluorobenzene (20 ml) and aluminum chloride (1.31 g) were reacted and treated in the same manner as in Preparation Example 22 to give 1.16 g of 6-bromo-1-(2,4-difluorophenyl)-1-hexanone.

$^{1}$H-NMR ($CDCl_3$,ppm)δ:1.47-1.97(6H,m), 2.93-2.30(2H,m), 3.43(2H,t,J=6.6Hz), 6.83-6.99(2H,m), 7.88-7.97(1H,m)

Preparation Example 54

1-Butyl-1H-indol (3.5 g), N,N-dimethyl-5-bromopentylamide (4.49 g) and phosphorus oxychloride (3.75 ml) were reacted and treated in the same manner as in Preparation Example 38 to give 2.59 g of 6-bromo-1-(1-butyl-1H-indol-3-yl)-1-hexanone.

$^{1}$H-NMR ($CDCl_3$,ppm)δ:0.97(3H,t,J=7.3Hz), 1.34-1.98(10H,m), 2.87(2H,t,J=7.3Hz), 3.39-3.45(2H,m), 4.16(2H,t,J=6.6Hz), 7.26-7.39(3H,m), 7.75(1H,s), 8.35-8.41(1H,m)

Preparation Example 55

(1) 6-Bromo-1-phenyl-1-hexanone (2.9 g) and potassium carbonate (3 g) were added to a solution of 4-hydroxy-4-tert-butoxycarbonylaminomethylpiperidine (2.5 g) in dimethylformamide (40 ml), and the mixture was stirred at 60°C for 2 hr. Water was added to the reaction mixture, and the mixture was extracted with chloroform. The organic layer was washed with saturated brine, dried and the solvent was evaporated under reduced pressure. The obtained residue was purified by silica gel column chromatography (chloroform:methanol=10:1) to give 3.74 g of 4-hydroxy-1-(6-oxo-6-phenylhexyl)-4-tert-butoxycarbonylaminomethylpiperidine.

$^1$H-NMR (CDCl$_3$,ppm)δ:1.36-1.82(20H,m), 2.36-2.88(4H,m), 2.60-2.67(2H,m), 2.97(2H,t,J=7.3Hz), 3.14(2H,d,J=6.6Hz), 5.01(1H,br), 7.42-7.59(3H,m), 7.93-7.97(2H,m)

(2) 4-Hydroxy-1-(6-oxo-6-phenylhexyl)-4-tert-butoxycarbonylaminomethylpiperidine (3.6 g) was dissolved in isopropyl alcohol (30 ml) and 15% hydrochloric acid-isopropyl alcohol (10 ml) was added under ice-cooling, which was followed by stirring at 60°C for 3 hr. The solvent was evaporated under reduced pressure to give 2.92 g of 4-aminomethyl-4-hydroxy-1-(6-oxo-6-phenylhexyl)piperidine dihydrochloride.

$^1$H-NMR (DMSO-d$_6$,ppm)δ:1.33-1.41(2H,m), 1.59-1.97(8H,m), 2.80-2.83(2H,m), 3.01-3.09(8H,m), 5.54(1H,br), 7.50-7.67(3H,m), 7.96-7.99(2H,m), 8.13(3H,br), 10.61(1H,br)

Preparation Example 56

(1) 4-Methoxy-4-tert-butoxycarbonylaminomethylpiperidine (2.3 g), 6-bromo-1-phenyl-1-hexanone (2.52 g) and potassium carbonate (2.6 g) were reacted and treated in the same manner as in Preparation Example 55(1) to give 3.35 g of 4-methoxy-1-(6-oxo-6-phenylhexyl)-4-tert-butoxycarbonylaminomethylpiperidine.

$^1$H-NMR (CDCl$_3$,ppm)δ:1.34-1.81(19H,m), 2.26-2.39(4H,m), 2.51-2.59(2H,m), 2.92-2.97(2H,m), 3.16(3H,s), 3.18-3.21(2H,m), 4.69(1H,br), 7.42-7.56(3H,m), 7.93-7.96(2H,m)

(2) 4-Methoxy-1-(6-oxo-6-phenylhexyl)-4-tert-butoxycarbonylaminomethylpiperidine (3.35 g) and 15% hydrochloric acid-isopropyl alcohol (15 ml) web reacted and treated in the same manner as in Preparation Example 55(2) to give 2.25 g of 4-aminomethyl-4-methoxy-1-(6-oxo-6-phenylhexyl)piperidine dihydrochloride.

$^1$H-NMR (DMSO-d$_6$,ppm)δ:1.32-2.07(10H,m), 2.52-3.19(13H,m), 7.53(2H,t,J=7.3Hz), 7.61-7.64(1H,m), 7.96(2H,d,J=9.3Hz), 8.13(2H,br), 8.83(1H,br), 10.85(1H,br)

Preparation Example 57

(1) 4-Methoxy-4-tert-butoxycarbonylaminomethylpiperidine (1.43 g), 4-phenylsulfonylbutyl bromide (1.7 g) and potassium carbonate (1.6 g) were reacted and treated in the same manner as in Preparation Example 55(1) to give 2.58 g of 4-methoxy-1-(4-phenylsulfonylbutyl)-4-tert-butoxycarbonylaminomethylpiperidine.

$^1$H-NMR (CDCl$_3$,ppm)δ:1.42-1.86(17H,m), 2.22-2.50(6H,m), 3.09-3.19(7H,m), 4.68(1H,br), 7.54-7.70(3H,m), 7.89-7.93(2H,m)

(2) 4-Methoxy-1-(4-phenylsulfonylbutyl)-4-tert-butoxycarbonylaminomethylpiperidine (2.58 g) and 15% hydrochloric acid-isopropyl alcohol (15 ml) were reacted and treated in the same manner as in Preparation Example 55(2) to give 2.2 g of 4-aminomethyl-4-methoxy-1-(4-phenylsulfonylbutyl)piperidine dihydrochloride.

$^1$H-NMR (DMSO-d$_6$,ppm)δ:1.32-2.12(8H,m), 2.71-3.42(13H,m), 7.65-7.83(3H,m), 7.86-7.97(2H,m), 8.18(2H,br), 8.83(1H,br)

In the same manner as in Preparation Example 22, the following compounds were produced.

Preparation Example 58

6-bromo-1-(4-ethylphenyl)-1-hexanone
$^1$H-NMR (CDCl$_3$,ppm)δ:1.26(3H,t,J=7.6Hz), 1.48-2.04(6H,m), 2.71(2H,q,J=7.2Hz), 2.97(2H,t,J=7.3Hz), 3.43(2H,t,J=7.0Hz), 7.28(1H,d,J=8.6Hz), 7.88(1H,d,J=8.6Hz)

Preparation Example 59

6-bromo-1-(2-naphthyl)-1-hexanone
m.p. 68-70°C

In the same manner as in Preparation Example 48, the following compound was produced.

Preparation Example 60

7-bromo-1-(3-chlorophenyl)-1-heptanone
$^1$H-NMR ($CDCl_3$,ppm)δ:1.38-1.85(8H,m), 2.95(2H,t,J=7.3Hz), 3.54(2H,t,J=6.6Hz), 7.40-7.92(4H,m)

In the same manner as in Preparation Example 33, the following compound was produced.

Preparation Example 61

6-bromo-1-(2,4-dichlorophenyl)-1-hexanone
$^1$H-NMR ($CDCl_3$,ppm)δ:1.45-1.79(2H,m), 1.83-1.89(2H,m), 1.91-1.94(2H,m), 2.33(2H,t,J=7.2Hz), 2.94(2H,t,J=7.2Hz), 7.30(1H,dd,J=2.0,8.6Hz), 7.42(1H,d,J=8.6Hz), 7.48(1H,d,J=2.0Hz)

In the same manner as in Preparation Example 38, the following compounds were produced.

Preparation Example 62

6-bromo-1-(1-benzyl-1H-indol-3-yl)-1-hexanone
$^1$H-NMR ($CDCl_3$,ppm)δ:1.47-1.62(2H,m), 1.74-1.95(4H,m), 2.85(2H,t,J=8.8Hz), 3.41(2H,t,J=8.8Hz), 5.34(2H,s), 7.13-7.16(2H,m) 7.23-7.33(6H,m), 7.76(1H,s), 8.38-8.42(1H,m)

Preparation Example 63

6-bromo-1-(1,2-dimethyl-1H-indol-3-yl)-1-hexanone
m.p. 54-56°C

In the same manner as in the above Preparation Examples, the following compounds were obtained.

Preparation Example 64

5-(3,4-dimethoxyphenoxy)pentyl chloride
$^1$H-NMR ($CDCl_3$,ppm)δ:1.56-1.68(2H,m), 1.75-1.91(4H,m), 3.57(2H,t,J=6.6Hz), 3.83(3H,s), 3.85(3H,s), 3.92(2H,t,J=6.6Hz), 6.38(1H,dd,J=2.6,8.6Hz), 6.51(1H,d,J=2.6Hz), 6.77(1H,d,J= 8.6Hz)

Preparation Example 65

5-(1-naphthyloxy)pentyl chloride
$^1$H-NMR ($CDCl_3$,ppm)δ:1.64-1.76(2H,m), 1.83-1.99(4H,m), 3.57(2H,t,J=6.6Hz), 4.11(2H,t,J=5.9Hz), 6.75-6.78(1H,m), 7.31-7.51(4H,m), 7.74-7.81(1H,m), 8.24-8.29(1H,m)

Preparation Example 66

4-(benzylthio)butyl chloride
$^1$H-NMR ($CDCl_3$,ppm)δ:1.66-1.88(4H,m), 2.42(2H,t,J=7.3Hz), 3.49(2H,t,J=5.9Hz), 3.70(2H,s), 7.19-7.36(5H,m)

Preparation Example 67

4-(benzylsulfonyl)butyl chloride
m.p. 100-101°C

Preparation Example 68

5-((1,4-benzodioxan-6-yl)methoxy)pentyl chloride
$^1$H-NMR ($CDCl_3$,ppm)δ:1.50-1.99(6H,m), 3.44(2H,t,J=6.3Hz), 3.53(2H,t,J=7.0Hz), 4.24(4H,s), 4.37(2H,s), 6.80-6.90(3H,m)

Preparation Example 69

5-(3-methoxyphenoxy)pentyl chloride
$^1$H-NMR ($CDCl_3$,ppm)δ:1.57-1.92(6H,m), 3.56(2H,t,J=6.6Hz), 3.78(3H,s), 3.95(2H,t,J=6.3Hz), 6.44-6.51(3H,m), 7.11-7.19(1H,m)

Preparation Example 70

5-bromo-1-(1-naphthyl)-1-pentanone
$^1$H-NMR ($CDCl_3$,ppm)δ: 1.86-2.02(4H,m), 3.07(2H,t,J=6.6Hz), 3.44(2H,t,J=6.8Hz), 7.44-7.61(3H,m), 7.81-8.01(3H,m), 8.56(1H,d,J=8.7Hz)

Preparation Example 71

5-benzylsulfonylpentyl chloride
m.p. 91-92°C

Preparation Example 72

5-(4-fluorophenoxy)pentyl bromide
$^1$H-NMR (DMSO-$d_6$,ppm)δ:1.49-1.58(2H,m), 1.70-1.77(2H,m), 1.80-1.89(2H,m), 3.55(2H,t,J=6.1Hz), 3.93(2H,t,J=6.1Hz), 6.90-6.95(2H,m), 7.05-7.13(2H,m)

Preparation Example 73

5-(4-chlorophenoxy)pentyl bromide
$^1$H-NMR ($CDCl_3$,ppm)δ:1.56-1.67(2H,m), 1.73-1.86(2H,m), 1.87-1.99(2H,m), 3.43(2H,t,J=6.0Hz), 3.93(2H,t,J=6.0Hz), 6.79-6.82(2H,m), 7.20-7.23(2H,m)

Preparation Example 74

5-(3,5-dimethoxyphenoxy)pentyl chloride
$^1$H-NMR ($CDCl_3$,ppm)δ:1.55-1.63(2H,m), 1.77-1.87(4H,m), 3.56(2H,t,J=6.6Hz), 3.76(6H,s), 3.95(2H,t,J=6.6Hz), 6.07(3H,s)

Preparation Example 75

3-(phenoxy)propyl chloride
$^1$H-NMR ($CDCl_3$,ppm)δ:2.23(2H,t,J=6.4Hz), 3.74(2H,t,J=6.7Hz), 4.11(2H,t,J=5.9Hz), 6.78-7.32(5H,m)

Preparation Example 76

3-(phenylthio)propyl chloride
$^1$H-NMR ($CDCl_3$,ppm)δ:2.01-2.18(2H,m), 3.07(2H,t,J=7.2Hz), 3.68(2H,t,J=6.4Hz), 7.11-7.40(5H,m)

Preparation Example 77

3-(phenylsulfonyl)propyl chloride
$^1$H-NMR ($CDCl_3$,ppm)δ:2.20-2.35(2H,m), 3.29(2H,t,J=7.7Hz), 3.62(2H,t,J=5.7Hz), 7.65-7.99(5H,m)

Preparation Example 78

3-bromo-1-phenyl-1-propanone
m.p. 50-51°C

Preparation Example 79

p-Toluenesulfonic acid in a catalytic amount and ethylene glycol (2.9 g) were added to a solution of 3-bromo-1-phenyl-1-propanone (10 g) in benzene (100 ml), and the mixture was stirred at refluxing temperature for 70 hr. The reaction mixture was washed successively with 10% aqueous potassium carbonate solution and saturated brine, and dried. The solvent was evaporated under reduced pressure, and the obtained residue was purified by silica gel column chromatography to give 6.0 g of 3,3-ethylenedioxy-3-phenylpropyl bromide. m.p. 57-58°C

In the same manner as in the above-mentioned Preparation Examples, the following compounds were obtained.

Preparation Example 80

4-bromo-1-phenyl-1-butanone
$^1$H-NMR ($CDCl_3$,ppm)δ:2.27-2.36(2H,m), 3.19(2H,t,J=6.6Hz), 3.55(2H,t,J=6.6Hz), 7.44-7.48(2H,m), 7.50-7.60(1H,m), 7.96-8.00(2H,m)

Preparation Example 81

2-(phenylsulfonyl)ethyl bromide
m.p. 58-59°C

Preparation Example 82

2-(phenylthio)ethyl bromide
$^1$H-NMR ($CDCl_3$,ppm)δ:3.25-3.31(2H,m), 3.42-3.49(2H,m), 7.16-7.41(5H,m)

Preparation Example 83

60% Sodium hydride (0.6 g) was suspended in dimethylformamide (10 ml), and piperonyl alcohol (1.5 g) was added under ice-cooilng with stirring. The mixture was stirred at room temperature for 1 hr. The mixture was again ice-cooled, and 1-bromo-5-chloropentane (1.8 g) was added, which was followed by stirring at room temperature for 1.5 hr. Water was added to the reaction mixture and the mixture was extracted with ethyl acetate. The organic layer was washed with saturated brine, dried and the solvent was evaporated under reduced pressure to give 0.6 g of 5-((3,4-methylenedioxyphenyl)methoxy)pentyl chloride.

$^1$H-NMR ($CDCl_3$,ppm)δ:1.42-1.68(4H,m), 1.72-1.82(2H,m), 3.44(2H,t,J=4.3Hz), 3.53(2H,t,J=6.6Hz), 4.39(2H,s), 5.94(2H,s), 6.77(2H,s), 6.84(1H,s)

Example 1

Hydrazine monohydrate (11.3 ml) was added to a solution of 4-amino-5-chloro-N-(1-(5-(2,3-dihydro-1,3-dioxo-1H-isoindol-2-yl)pentyl)piperidin-4-ylmethyl)-2-methoxybenzamide (60 g) in ethanol (0.6 L), and the mixture was refluxed under heating for 4 hr. The precipitated crystals were filtered off, and the solvent was evaporated under reduced pressure. The obtained residue was purified by silica gel column chromatography (chloroform:methanol=10:1) to give 37.0 g of 4-amino-N-(1-(5-aminopentyl)piperidin-4-ylmethyl)-5-chloro-2-methoxybenzamide.

m.p. 60-63°C

Example 2

4-Amino-5-chloro-N-(1-(3-(2,3-dihydro-1,3-dioxo-1H-isoindol-2-yl)propyl)piperidin-4-ylmethyl)-2-methoxybenzamide (7.7 g) as starting compound and hydrazine monohydrate (0.76 ml) were reacted and treated in the same manner as in Example 1 to give 3.6 g of 4-amino-N-(1-(3-aminopropyl)piperidin-4-ylmethyl)-5-chloro-2-methoxybenzamide.

$^1$H-NMR (DMSO-$d_6$)δ:0.96-1.83(9H,m), 2.22-2.29(2H,m), 2.51-2.58(2H,m), 2.78-2.86(2H,m), 3.15(2H,t,J=5.9Hz), 3.31(2H,br), 3.83(3H,s), 5.91(2H,s), 6.48(1H,s), 7.66(1H,s), 7.86-7.93(1H,m)

Example 3

4-Amino-5-chloro-N-(1-(4-(2,3-dihydro-1,3-dioxo-1H-isoindol-2-yl)butyl)piperidin-4-ylmethyl)-2-methoxybenzamide (9.3 g) as starting compound and hydrazine monohydrate (1.4 ml) were reacted and treated in the same manner as in Example 1 to give 6.9 g of 4-amino-N-(1-(4-aminobutyl)piperidin-4-ylmethyl)-5-chloro-2-methoxybenzamide.

m.p. 104-107°C

Example 4

4-Amino-5-chloro-N-(1-(6-(2,3-dihydro-1,3-dioxo-1H-isoindol-2-yl)hexyl)piperidin-4-ylmethyl)-2-methoxybenzamide (15.9 g) as starting compound and hydrazine monohydrate (2.2 ml) were reacted and treated in the same manner as in Example 1 to give 13.5 g of 4-amino-N-(1-(6-aminohexyl)piperidin-4-ylmethyl)-5-chloro-2-methoxybenzamide.

$^1$H-NMR ($CDCl_3$)δ:1.21-1.98(17H,m), 2.28-2.36(2H,m), 2.68(2H,t,J=6.6Hz), 2.91-2.98(2H,m), 3.32(2H,t,J=6.6Hz), 3.89(3H,s), 4.38(2H,s), 6.29(1H,s), 7.71-7.78(1H,m), 8.10(1H,s)

Example 5

Benzaldehyde (0.76 g) was added to a solution of 4-amino-N-(1-(5-aminopentyl)piperidin-4-ylmethyl)-5-chloro-2-methoxybenzamide (2.0 g) in ethanol (50 ml), and the mixture was stirred at refluxing temperature for 3 hr. Sodium borohydride (0.53 g) was added to the reaction mixture under ice-cooling, and the mixture was stirred overnight at room temperature. Water was added to the reaction mixture, and the mixture was extracted with chloroform. The organic layer was washed with saturated brine, dried and the solvent was evaporated under reduced pressure. The obtained residue was purified by silica gel column chromatography (chloroform:methanol:ammonia=50:5:1) to give 1.78 g of 4-amino-N-((1-(5-benzylaminopentyl)piperidin-4-yl)methyl)-5-chloro-2-methoxybenzamide.

$^1$H-NMR($CDCl_3$,ppm) δ:1.22-1.94(14H,m), 2.26-2.33(2H,m), 2.62(2H,t,J=7.3Hz), 2.88-2.96(2H,m), 3.32(2H,t,J=6.6Hz), 3.78(2H,s), 3.88(3H,s), 4.44(2H,s), 6.29(1H,s), 7.20-7.37(5H,m), 7.72-7.78(1H,m), 8.10(1H,s)

Example 6

4-Amino-N-(1-(5-aminopentyl)piperidin-4-ylmethyl)-5-chloro-2-methoxybenzamide (2 g) as starting compound and 4-fluorobenzaldehyde (0.71 g) were reacted and treated in the same manner as in Example 5 to give 2.3 g of 4-amino-5-chloro-N-((1-(5-(4-fluorobenzylamino)pentyl)piperidin-4-yl)methyl)-2-methoxybenzamide.

m.p. 66-68°C

Example 7

4-Amino-N-(1-(5-aminopentyl)piperidin-4-ylmethyl)-5-chloro-2-methoxybenzamide (2 g) as starting compound and 4-methoxybenzaldehyde (0.78 g) were reacted and treated in the same manner as in Example 5 to give 2.15 g of 4-amino-5-chloro-2-methoxy-N-((1-(5-(4-methoxybenzylamino)pentyl)piperidin-4-yl)methyl)benzamide.

$^1$H-NMR($CDCl_3$,ppm) δ:1.25-1.76(12H,m), 1.84-1.94(2H,m), 2.25-2.32(2H,m), 2.57-2.63(2H,m), 2.88-2.94(2H,m), 3.31(2H,t,J=5.9Hz), 3.70(2H,s), 3.79(3H,s), 3.87(3H,s), 4.48(2H,s), 6.28(1H,s), 6.82-6.87(2H,m), 7.20-7.24(2H,m), 7.72-7.77(1H,m), 8.09(1H,s)

Example 8

4-Amino-N-(1-(5-aminopentyl)piperidin-4-ylmethyl)-5-chloro-2-methoxybenzamide (2 g) as starting compound and 4-methylbenzaldehyde (0.69 g) were reacted and treated in the same manner as in Example 5 to give 2.5 g of 4-amino-5-chloro-2-methoxy-N-((1-(5-(4-methylbenzylamino)pentyl)piperidin-4-yl)methyl)benzamide.

$^1$H-NMR($CDCl_3$,ppm) δ:1.25-1.77(12H,m), 1.91-2.00(2H,m), 2.30-2.37(5H,m), 2.58-2.65(2H,m), 2.92-2.99(2H,m),

3.31(2H,t,J=6.6Hz), 3.75(2H,s), 3.88(3H,s), 4.46(2H,s), 6.30(1H,s), 7.10-7.27(4H,m), 7.72-7.79(1H,m), 8.08(1H,s)

Example 9

4-Amino-N-(1-(5-aminopentyl)piperidin-4-ylmethyl)-5-chloro-2-methoxybenzamide (2 g) as starting compound and 4-nitrobenzaldehyde (0.87 g) were reacted and treated in the same manner as in Example 5 to give 2.3 g of 4-amino-5-chloro-2-methoxy-N-((1-(5-(4-nitrobenzylamino)pentyl)piperidin-4-yl)methyl)benzamide.

$^1$H-NMR($CDCl_3$,ppm) δ:1.26-1.79(11H,m), 1.91-2.03(2H,m), 2.23(1H,br), 2.31-2.38(2H,m), 2.58-2.65(2H,m), 2.92-3.00(2H,m), 3.32(2H,t,J=6.6Hz), 3.89(5H,s), 4.49(2H,s), 6.31(1H,s), 7.48-7.53(2H,m), 7.74-7.81(1H,m), 8.06(1H,s), 8.15-8.18(2H,m)

Example 10

4-Amino-N-(1-(5-aminopentyl)piperidin-4-ylmethyl)-5-chloro-2-methoxybenzamide (2 g) as starting compound and 2-thiophenecarboxaldehyde (0.64 g) were reacted and treated in the same manner as in Example 5 to give 2.5 g of 4-amino-5-chloro-2-methoxy-N-((1-(5-(2-thienylmethylamino)pentyl)piperidin-4-yl)methyl)benzamide.

m.p. 67-69°C

Example 11

4-Amino-N-(1-(5-aminopentyl)piperidin-4-ylmethyl)-5-chloro-2-methoxybenzamide (1 g) as starting compound and cyclohexanecarboxaldehyde (0.32 g) were reacted and treated in the same manner as in Example 5 to give 1.2 g of 4-amino-5-chloro-N-((1-(5-(cyclohexylmethylamino)pentyl)piperidin-4-yl)methyl)-2-methoxybenzamide.

$^1$H-NMR($CDCl_3$,ppm) δ:0.83-1.80(22H,m), 1.88-2.00(2H,m), 2.29-2.37(2H,m), 2.48(2H,d,J=6.6Hz), 2.59-2.68(2H,m), 2.69-2.99(3H,m), 3.32(2H,t,J=5.9Hz), 3.90(3H,s), 4.43(2H,s), 6.31(1H,s), 7.71-7.79(1H,m), 8.09(1H,s)

Example 12

4-Amino-N-(1-(5-aminopentyl)piperidin-4-ylmethyl)-5-chloro-2-methoxybenzamide (3 g) as starting compound and 4-pyridinecarboxaldehyde (0.84 g) were reacted and treated in the same manner as in Example 5 to give 1.14 g of 4-amino-5-chloro-2-methoxy-N-((1-(5-(4-pyridylmethylamino)pentyl)piperidin-4-yl)methyl)benzamide.

$^1$H-NMR($CDCl_3$,ppm) δ:1.25-2.00(14H,m), 2.25-2.34(2H,m), 2.61(2H,t,J=7.3Hz), 2.87-2.95(2H,m), 3.32(2H,t,J=6.6Hz), 3.80(2H,s), 3.88(3H,s), 4.44(2H,s), 6.29(1H,s), 7.24-7.28(2H,m), 7.70-7.78(1H,m), 8.10(1H,s), 8.51-8.54(2H,m)

Example 13

4-Amino-N-(1-(5-aminopentyl)piperidin-4-ylmethyl)-5-chloro-2-methoxybenzamide (2.1 g) as starting compound and 1-naphthaldehyde (1.13 g) were reacted and treated in the same manner as in Example 5 to give 1.71 g of 4-amino-5-chloro-2-methoxy-N-((1-(5-(1-naphthylmethylamino)pentyl)piperidin-4-yl)methyl)benzamide.

$^1$H-NMR($CDCl_3$,ppm) δ:1.25-1.75(12H,m), 1.84-1.94(2H,m), 2.26-2.32(2H,m), 2.74(2H,t,J=6.6Hz), 2.87-2.95(2H,m), 3.31(2H,t,J=6.6Hz), 3.87(3H,s),4.22(2H,s), 4.41(2H,s), 6.27(1H,s), 7.38-7.55(4H,m), 7.71-7.77(2H,m), 7.81-7.87(1H,m), 8.09-8.12(2H,m)

Example 14

4-Amino-N-(1-(5-aminopentyl)piperidin-4-ylmethyl)-5-chloro-2-methoxybenzamide (1.98 g) as starting compound and 2-naphthaldehyde (0.74 g) were reacted and treated in the same manner as in Example 5 to give 1.81 g of 4-amino-5-chloro-2-methoxy-N-((1-(5-(2-naphthylmethylamino)pentyl)piperidin-4-yl)methyl)benzamide.

$^1$H-NMR($CDCl_3$,ppm) δ:1.22-1.74(12H,m), 1.83-1.94(2H,m), 2.26-2.33(2H,m), 2.66(2H,t,J=7.3Hz), 2.88-2.94(2H,m), 3.31(2H,t,J=6.6Hz), 3.87(3H,s),3.94(2H,s), 4.40(2H,s), 6.27(1H,s), 7.39-7.49(3H,m), 7.70-7.82(5H,m), 8.10(1H,s)

Example 15

4-Amino-N-(1-(5-aminopentyl)piperidin-4-ylmethyl)-5-chloro-2-methoxybenzamide (1.5 g) as starting compound and 1-methyl-1H-indol-3-carboxaldehyde (0.81 g) were reacted and treated in the same manner as in Example 5 to give 1.0 g of 4-amino-5-chloro-N-((1-(5-((1-methyl-1H-indol-3-ylmethyl)amino)pentyl)piperidin-4-yl)methyl)-2-methoxybenzamide.

$^1$H-NMR($CDCl_3$,ppm) δ:1.18-2.16(14H,m), 2.17-2.23(2H,m), 2.48(2H,t,J=7.3Hz), 2.82-2.88(2H,m), 3.31(2H,t,J=6.6Hz), 3.73(3H,s), 3.77(2H,s), 3.87(3H,s), 4.37(2H,s), 6.26(1H,s), 6.95-7.28(4H,m), 7.64-7.76(2H,m), 8.10(1H,s)

Example 16

4-Amino-N-(1-(3-aminopropyl)piperidin-4-ylmethyl)-5-chloro-2-methoxybenzamide (2 g) as starting compound and 3,4-dichlorobenzaldehyde (0.99 g) were reacted and treated in the same manner as in Example 5 to give 2.7 g of 4-amino-5-chloro-N-((1-(3-(3,4-dichlorobenzylamino)propyl)piperidin-4-yl)methyl)-2-methoxybenzamide.

$^1$H-NMR ($CDCl_3$,ppm)δ:1.24-1.35(2H,m), 1.52-1.96(5H,m), 2.21(1H,br), 2.37(2H,t,J=7.3Hz), 2.63(2H,t,J=6.6Hz), 2.88-2.97(2H,m), 3.31(2H,t,J=5.9Hz), 3.72(2H,s), 3.88(3H,s), 4.44(2H,s), 6.29(1H,s), 7.12-7.19(1H,m), 7.35-7.46(2H,m), 7.72-7.78(1H,m), 8.09(1H,s)

Example 17

4-Amino-N-(1-(6-aminohexyl)piperidin-4-ylmethyl)-5-chloro-2-methoxybenzamide (2.0 g) as starting compound and 4-methylbenzaldehyde (0.66 g) were reacted and treated in the same manner as in Example 5 to give 1.07 g of 4-amino-5-chloro-2-methoxy-N-((1-(6-(4-methylbenzylamino)hexyl)piperidin-4-yl)methyl)benzamide.

$^1$H-NMR($CDCl_3$,ppm) δ:1.21-1.96(16H,m), 2.26-2.34(5H,m), 2.60(2H,t,J=7.3Hz), 2.88-2.97(2H,m), 3.32(2H,t,J=5.9Hz), 3.73(2H,s), 3.89(3H,s), 4.40(2H,s), 6.29(1H,s), 7.09-7.22(4H,m), 7.71-7.78(1H,m), 8.10(1H,s)

Example 18

4-Amino-N-(1-(6-aminohexyl)piperidin-4-ylmethyl)-5-chloro-2-methoxybenzamide (1.5 g) as starting compound and 4-methoxylbenzaldehyde (0.56 g) were reacted and treated in the same manner as in Example 5 to give 1.13 g of 4-amino-5-chloro-N-((1-(6-(4-methoxybenzylamino)hexyl)piperidin-4-yl)methyl)-2-methoxybenzamide.

$^1$H-NMR($CDCl_3$,ppm) δ:1.21-2.00(16H,m), 2.26-2.32(2H,m), 2.60(2H,t,J=7.3Hz), 2.89-2.96(2H,m), 3.32(2H,t,J=5.9Hz), 3.71(2H,s), 3.79(3H,s), 3.88(3H,s), 4.44(2H,s), 6.29(1H,s), 6.83-6.88(2H,m), 7.19-7.24(2H,m), 7.73-7.78(1H,m), 8.09(1H,s)

Example 19

4-Amino-N-(1-(6-aminohexyl)piperidin-4-ylmethyl)-5-chloro-2-methoxybenzamide (2.0 g) as starting compound and 3,4-dichlorobenzaldehyde (0.97 g) were reacted and treated in the same manner as in Example 5 to give 1.42 g of 4-amino-5-chloro-N-((1-(6-(3,4-dichlorobenzylamino)hexyl)piperidin-4-yl)methyl)-2-methoxybenamide.

$^1$H-NMR($CDCl_3$,ppm) δ:1.23-1.77(14H,m), 1.84-1.95(2H,m), 2.26-2.32(2H,m), 2.58(2H,t,J=6.6Hz), 2.88-2.96(2H,m), 3.32(2H,t,J=5.9Hz), 3.73(2H,s),3.89(3H,s), 4.41(2H,s), 6.29(1H,s), 7.15(1H,dd,J=2.0,7.9Hz), 7.37(1H,d,J=7.9Hz), 7.43(1H,d,J=2.0Hz), 7.72-7.78(1H,m), 8.10(1H,s)

Example 20

Acetaldehyde (0.32 ml) and sodium cyanoborohydride (0.36 g) were added to a solution of 4-amino-N-(1-(5-aminopentyl)piperidin-4-ylmethyl)-5-chloro-2-methoxybenzamide (1.0 g) in methanol (20 ml), and the mixture was stirred at room temperature for 1 hr. A 10% aqueous sodium hydroxide solution was added to the reaction mixture, and the mixture was extracted with chloroform. The organic layer was washed with saturated brine, dried and the solvent was

evaporated under reduced pressure. The obtained residue was purified by silica gel column chromatography (chloroform:methanol=5:1) to give 0.55 g of 4-amino-5-chloro-N-((1-(5-diethylaminopentyl)piperidin-4-yl)methyl)-2-methoxybenzamide.

$^1$H-NMR($CDCl_3$,ppm) δ:1.08(6H,t,J=7.3Hz), 1.24-1.77(11H,m), 1.87-1.99(2H,m), 2.28-2.37(2H,m), 2.45-2.54(2H,m), 2.61(4H,q,J=7.3Hz), 2.91-2.98(2H,m), 3.32(2H,t,J=6.0Hz), 3.89(3H,s), 4.53(2H,s), 6.33(1H,s), 7.72-7.80(1H,m), 8.08(1H,s)

Example 21

4-Amino-N-(1-(5-aminopentyl)piperidin-4-ylmethyl)-5-chloro-2-methoxybenzamide (1.0 g) as starting compound, 37% formalin (0.45 ml) and sodium cyanoborohydride (0.34 g) were reacted and treated in the same manner as in Example 20 to give 0.48 g of 4-amino-5-chloro-N-((1-(5-dimethylaminopentyl)piperidin-4-yl)methyl)-2-methoxybenzamide.

$^1$H-NMR($CDCl_3$,ppm) δ: 1.24-1.78(11H,m), 1.83-1.97(2H,m), 2.15-2.35(10H,m), 2.88-2.97(2H,m), 3.32(2H,t,J=6.6Hz), 3.89(3H,s), 4.43(2H,s), 6.29(1H,s), 7.70-7.78(1H,m), 8.10(1H,s)

Example 22

4-Amino-N-(1-(6-aminohexyl)piperidin-4-ylmethyl)-5-chloro-2-methoxybenzamide (1.0 g) as starting compound, acetaldehyde (0.31 ml) and sodium cyanoborohydride (0.38 g) were reacted and treated in the same manner as in Example 20 to give 0.35 g of 4-amino-5-chloro-N-((1-(6-diethylaminohexyl)piperidin-4-yl)methyl)-2-methoxybenzamide.

$^1$H-NMR($CDCl_3$,ppm) δ: 1.02(6H,t,J=7.3Hz), 1.24-1.96(15H,m), 2.26-2.33(2H,m), 2.37-2.44(2H,m), 2.52(4H,q,J=7.3Hz), 2.89-2.96(2H,m), 3.32(2H,t,J=6.6Hz), 3.90(3H,s), 4.40(2H,s), 6.30(1H,s), 7.72-7.78(1H,m), 8.10(1H,s)

Example 23

4-Amino-N-(1-(5-aminopentyl)piperidin-4-ylmethyl)-5-chloro-2-methoxybenzamide (2.0 g) as starting compound, cyclohexanone (0.73 ml) and sodium cyanoborohydride (0.86 g) were reacted and treated in the same manner as in Example 20 to give 1.42 g of 4-amino-5-chloro-N-((1-(5-(cyclohexylamino)pentyl)piperidin-4-yl)methyl)-2-methoxybenzamide.

$^1$H-NMR($CDCl_3$,ppm) δ: 0.99-1.96(24H,m), 2.27-2.45(3H,m), 2.62(2H,t,J=7.3Hz), 2.88-2.97(2H,m), 3.32(2H,t,J=6.6Hz), 3.90(3H,s), 4.39(2H,s), 6.29(1H,s), 7.72-7.77(1H,m), 8.10(1H,s)

Example 24

4-Amino-5-chloro-N-((1-(5-(4-chlorobenzylamino)pentyl)piperidin-4-yl)methyl)-2-methoxybenzamide (1.8 g) as starting compound, acetaldehyde (0.26 ml) and sodium cyanoborohydride (0.29 g) were reacted and treated in the same manner as in Example 20 to give 1.05 g of 4-amino-5-chloro-N-((1-(5-(N-(4-chlorobenzyl)-N-ethylamino)pentyl)piperidin-4-yl)methyl)-2-methoxybenzamide.

$^1$H-NMR ($CDCl_3$,ppm)δ: 1.01(3H,t,J=7.3Hz), 1.20-1.76(11H,m), 1.86-1.98(2H,m), 2.20-2.52(6H,m), 2.88-2.97(2H,m), 3.32(2H,t,J=6.6Hz), 3.49(2H,s), 3.88(3H,s), 4.50(2H,s), 6.30(1H,s), 7.25(4H,s), 7.73-7.81(1H,m), 8.08(1H,s)

Example 25

4-Amino-5-chloro-N-((1-(5-(4-fluorobenzylamino)pentyl)piperidin-4-yl)methyl)-2-methoxybezamide (1.8 g) as starting compound, acetaldehyde (0.25 ml) and sodium cyanoborohydride (0.28 g) were reacted and treated in the same manner as in Example 20 to give 1.0 g of 4-amino-5-chloro-N-((1-(5-(N-ethyl-N-(4-fluorobenzyl)amino)pentyl)piperidin-4-yl)methyl)-2-methoxybenzamide.

$^1$H-NMR ($CDCl_3$,ppm)δ: 1.01(3H,t,J=7.3Hz), 1.22-1.76(11H,m), 1.84-1.97(2H,m), 2.24-2.42(4H,m), 2.47(2H,q,J=7.3Hz), 2.88-2.97(2H,m), 3.32(2H,t,J=5.9Hz), 3.49(2H,s), 3.89(3H,s), 4.44(2H,s), 6.29(1H,s), 6.91-7.00(2H,m), 7.24-7.30(2H,m), 7.71-7.78(1H,m), 8.10(1H,s)

Example 26

4-Amino-5-chloro-N-((1-(5-(3,4-dichlorobenzylamino)pentyl)piperidin-4-yl)methyl)-2-methoxybenzamide (2.0 g) as starting compound, acetaldehyde (0.27 ml) and sodium cyanoborohydride (0.3 g) were reacted and treated in the same manner as in Example 20 to give 0.82 g of 4-amino-5-chloro-N-((1-(5-(N-(3,4-dichlorobenzyl)-N-ethylamino)pentyl)piperidin-4-yl)methyl)-2-methoxybenzamide.

$^1$H-NMR ($CDCl_3$,ppm)δ: 1.01(3H,t,J=7.3Hz), 1.20-1.77(11H,m), 1.82-1.95(2H,m), 2.23-2.32(2H,m), 2.35-2.42(2H,m), 2.48(2H,q,J=7.3Hz), 2.86-2.96(2H,m), 3.32(2H,t,J=6.6Hz), 3.47(2H,s), 3.89(3H,s), 4.39(2H,s), 6.29(1H,s), 7.15(1H,dd,J=2.0,8.0Hz), 7.35(1H,d,J=8.0Hz), 7.43(1H,d,J=2.0Hz), 7.70-7.78(1H,m), 8.11(1H,s)

Example 27

4-Amino-5-chloro-2-methoxy-N-((1-(5-(4-methoxybenzylamino)pentyl)piperidin-4-yl)methyl)benzamide (1.5 g) as starting compound, acetaldehyde (0.22 ml) and sodium cyanoborohydride (0.24 g) were reacted and treated in the same manner as in Example 20 to give 0.8 g of 4-amino-5-chloro-N-((1-(5-(N-ethyl-N-(4-methoxybenzyl)amino)pentyl)piperidin-4-yl)methyl)-2-methoxybenzamide.

$^1$H-NMR ($CDCl_3$,ppm)δ: 1.02(3H,t,J=7.3Hz), 1.20-1.77(11H,m), 1.87-1.98(2H,m), 2.26-2.54(6H,m), 2.89-2.97(2H,m), 3.32(2H,t,J=6.6Hz), 3.50(2H,s), 3.79(3H,s), 3.89(3H,s), 4.46(2H,s), 6.30(1H,s), 6.81-6.87(2H,m), 7.20-7.24(2H,m), 7.73-7.81(1H,m), 8.09(1H,s)

Example 28

4-Amino-5-chloro-2-methoxy-N-((1-(5-(4-methylbenzylamino)pentyl)piperidin-4-yl)methyl)benzamide (1.5 g) as starting compound, acetaldehyde (0.22 ml) and sodium cyanoborohydride (0.25 g) were reacted and treated in the same manner as in Example 20 to give 0.7 g of 4-amino-5-chloro-N-((1-(5-(N-ethyl-N-(4-methylbenzyl)amino)pentyl)piperidin-4-yl)methyl)-2-methoxybenzamide.

$^1$H-NMR ($CDCl_3$,ppm)δ: 1.02(3H,t,J=7.3Hz), 1.20-1.76(11H,m), 1.85-1.98(2H,m), 2.25-2.53(6H,m), 2.87-2.97(2H,m), 3.32(2H,t,J=6.6Hz), 3.46(3H,s), 3.51(2H,s), 3.89(3H,s), 4.44(2H,s), 6.29(1H,s), 7.07-7.23(4H,m), 7.72-7.80(1H,m), 8.09(1H,s)

Example 29

4-Amino-5-chloro-2-methoxy-N-((1-(5-(4-nitrobenzylamino)pentyl)piperidin-4-yl)methyl)benzamide (1.5 g) as starting compound, acetaldehyde (0.21 ml) and sodium cyanoborohydride (0.24 g) were reacted and treated in the same manner as in Example 20 to give 0.6 g of 4-amino-5-chloro-N-((1-(5-(N-ethyl-N-(4-nitrobenzyl)amino)pentyl)piperidin-4-yl)methyl)-2-methoxybenzamide.

$^1$H-NMR ($CDCl_3$,ppm)δ: 1.02(3H,t,J=7.3Hz), 1.21-1.78(11H,m), 1.88-2.02(2H,m), 2.27-2.54(6H,m), 2.91-2.99(2H,m), 3.32(2H,t,J=5.9Hz), 3.62(2H,s), 3.90(3H,s), 4.41(2H,s), 6.30(1H,s), 7.41-7.52(2H,m), 7.70-7.88(1H,m), 8.07-8.18(3H,m)

Example 30

4-Amino-5-chloro-2-methoxy-N-((1-(5-((2-thienylmethyl)amino)pentyl)piperidin-4-yl)methyl)benzamide (1.5 g) as starting compound, acetaldehyde (0.23 ml) and sodium cyanoborohydride (0.25 g) were reacted and treated in the same manner as in Example 20 to give 0.96 g of 4-amino-5-chloro-N-((1-(5-(N-ethyl-N-(2-thienylmethyl)amino)pentyl)piperidin-4-yl)methyl)-2-methoxybenzamide.

$^1$H-NMR ($CDCl_3$,ppm)δ: 1.05(3H,t,J=7.3Hz), 1.23-1.77(11H,m), 1.88-2.00(2H,m), 2.28-2.57(6H,m), 2.91-2.99(2H,m), 3.32(2H,t,J=6.6Hz), 3.80(2H,s), 3.89(3H,s), 4.46(2H,s), 6.30(1H,s), 6.86-6.96(2H,m), 7.18-7.21(1H,m), 7.72-7.80(1H,m), 8.09(1H,s)

Example 31

4-Amino-5-chloro-N-((1-(5-(cyclohexylmethylamino)pentyl)piperidin-4-yl)methyl)-2-methoxybenzamide (1.27 g) as starting compound, acetaldehyde (0.19 ml) and sodium cyanoborohydride (0.22 g) were reacted and treated in the same manner as in Example 20 to give 0.6 g of 4-amino-5-chloro-N-((1-(5-(N-ethyl-N-(cyclohexylmethyl)amino)pentyl)piperidin-4-yl)methyl)-2-methoxybenzamide.

$^1$H-NMR ($CDCl_3$,ppm)δ: 0.75-0.94(2H,m), 0.98(3H,t,J=7.3Hz), 1.09-2.00(22H,m), 2.16(2H,d,J=7.3Hz), 2.28-2.41(4H,m), 2.48(2H,q,J=7.3Hz), 2.91-3.00(2H,m), 3.32(2H,t,J=6.6Hz), 3.90(3H,s), 4.39(2H,s), 6.30(1H,s), 7.72-7.79(1H,m), 8.10(1H,s)

Example 32

4-Amino-5-chloro-2-methoxy-N-((1-(5-((4-pyridylmethyl)amino)pentyl)piperidin-4-yl)methyl)benzamide (0.8 g) as starting compound, acetaldehyde (0.12 ml) and sodium cyanoborohydride (0.14 g) were reacted and treated in the same manner as in Example 20 to give 0.35 g of 4-amino-5-chloro-N-((1-(5-(N-ethyl-N-(4-pyridylmethyl)amino)pentyl)piperidin-4-yl)methyl)-2-methoxybenzamide.

$^1$H-NMR ($CDCl_3$,ppm)δ: 1.02(3H,t,J=7.3Hz), 1.21-1.80(11H,m), 1.90-2.03(2H,m), 2.29-2.54(6H,m), 2.92-3.00(2H,m), 3.29-3.36(2H,m), 3.54(2H,s), 3.90(3H,s), 4.39(2H,s), 6.29(1H,s), 7.25-7.29(2H,m), 7.70-7.80(1H,m), 8.10(1H,s), 8.49-8.52(2H,m)

Example 33

4-Amino-N-((1-(5-benzylaminopentyl)piperidin-4-yl)methyl)-5-chloro-2-methoxybenzamide (2.0 g) as starting compound, propionaldehyde (0.13 ml) and sodium cyanoborohydride (0.35 g) were reacted and treated in the same manner as in Example 20 to give 1.05 g of 4-amino-5-chloro-2-methoxy-N-((1-(5-(N-n-propyl-N-benzylamino)pentyl)piperidin-4-yl)methyl)benzamide.

$^1$H-NMR ($CDCl_3$,ppm)δ: 0.85(3H,t,J=7.3Hz), 1.20-1.75(13H,m), 1.85-1.96(2H,m), 2.24-2.43(6H,m), 2.88-2.96(2H,m), 3.31(2H,t,J=6.6Hz), 3.53(2H,s), 3.87(3H,s), 4.51(2H,s), 6.30(1H,s), 7.16-7.35(5H,m), 7.71-7.79(1H,m), 8.09(1H,s)

Example 34

4-Amino-N-((1-(5-benzylaminopentyl)piperidin-4-yl)methyl)-5-chloro-2-methoxybenzamide (2.0 g) as starting compound, acetone (0.3 ml) and sodium cyanoborohydride (0.35 g) were reacted and treated in the same manner as in Example 20 to give 0.43 g of 4-amino-5-chloro-N-((1-(5-(N-isopropyl-N-benzylamino)pentyl)piperidin-4-yl)methyl)-2-methoxybenzamide.

$^1$H-NMR ($CDCl_3$,ppm)δ: 0.99(6H,d,J=6.6Hz), 1.19-1.75(11H,m), 1.87-1.98(2H,m), 2.24-2.32(2H,m), 2.38(2H,t,J=6.6Hz), 2.83-2.98(3H,m), 3.32(2H,t,J=6.6Hz), 3.52(2H,s), 3.89(3H,s), 4.41(2H,s), 6.28(1H,s), 7.16-7.35(5H,m), 7.71-7.78(1H,m), 8.10(1H,s)

Example 35

4-Amino-N-((1-(5-benzylaminopentyl)piperidin-4-yl)methyl)-5-chloro-2-methoxybenzamide (1.46 g) as starting compound, acetaldehyde (0.19 ml) and sodium cyanoborohydride (0.43 g) were reacted and treated in the same manner as in Example 20 to give 0.8 g of 4-amino-5-chloro-N-((1-(5-(N-ethyl-N-benzylamino)pentyl)piperidin-4-yl)methyl)-2-methoxybenzamide.

$^1$H-NMR ($CDCl_3$,ppm)δ: 1.02(3H,t,J=7.3Hz), 1.20-1.74(11H,m), 1.84-1.93(2H,m), 2.24-2.31(2H,m), 2.37-2.44(2H,m), 2.49(2H,q,J=7.3Hz), 2.87-2.94(2H,m), 3.32(2H,t,J=6.6Hz), 3.54(2H,s), 3.89(3H,s), 4.38(2H,s), 6.28(1H,s), 7.17-7.33(5H,m), 7.71-7.76(1H,m), 8.11(1H,s)

Example 36

4-Amino-5-chloro-2-methoxy-N-((1-(5-(1-naphthylmethylamino)pentyl)piperidin-4-yl)methyl)benzamide (1.43 g) as

starting compound, acetaldehyde (0.16 ml) and sodium cyanoborohydride (0.38 g) were reacted and treated in the same manner as in Example 20 to give 0.75 g of 4-amino-5-chloro-N-((1-(5-(N-ethyl-N-(1-naphthylmethyl)amino)pentyl)piperidin-4-yl)methyl)-2-methoxybenzamide.

$^1$H-NMR ($CDCl_3$,ppm)δ: 1.06(3H,t,J=7.3Hz), 1.16-1.73(11H,m), 1.80-1.89(2H,m), 2.18-2.24(2H,m), 2.44-2.50(2H,m), 2.56(2H,q,J=7.3Hz), 2.83-2.90(2H,m), 3.31(2H,t,J=6.6Hz), 3.88(3H,s), 3.96(2H,s), 4.38(2H,s), 6.27(1H,s), 7.35-7.52(4H,m), 7.71-7.76(2H,m), 7.80-7.85(1H,m), 8.11(1H,s), 8.30-8.34(1H,m)

Example 37

4-Amino-5-chloro-2-methoxy-N-((1-(5-(2-naphthylmethylamino)pentyl)piperidin-4-yl)methyl)benzamide (1.50 g) as starting compound, acetaldehyde (0.18 ml) and sodium cyanoborohydride (0.40 g) were reacted and treated in the same manner as in Example 20 to give 1.1 g of 4-amino-5-chloro-N-((1-(5-(N-ethyl-N-(2-naphthylmethyl)amino)pentyl)piperidin-4-yl)methyl)-2-methoxybenzamide.

$^1$H-NMR ($CDCl_3$,ppm)δ: 1.05(3H,t,J=7.3Hz), 1.20-1.72(11H,m), 1.81-1.90(2H,m), 2.22-2.29(2H,m), 2.42-2.49(2H,m), 2.54(2H,q,J=7.3Hz), 2.85-2.91(2H,m), 3.31(2H,t,J=6.6Hz), 3.69(2H,s), 3.87(3H,s), 4.40(2H,s), 6.27(1H,s), 7.38-7.52(3H,m), 7.72-7.83(5H,m), 8.10(1H,s)

Example 38

4-Amino-5-chloro-N-((1-(5-(cyclohexylmethylamino)pentyl)piperidin-4-yl)methyl)-2-methoxybenzamide (0.79 g) as starting compound, benzaldehyde (0.19 g) and sodium cyanoborohydride (0.23 g) were reacted and treated in the same manner as in Example 20 to give 0.49 g of 4-amino-N-((1-(5-(N-benzyl-N-(cyclohexylmethyl)amino)pentyl)piperidin-4-yl)methyl)-5-chloro-2-methoxybenzamide.

$^1$H-NMR ($CDCl_3$,ppm)δ: 0.72-0.84(2H,m), 1.09-1.80(20H,m), 2.01-2.13(2H,m), 2.16(2H,d,J=7.3Hz), 2.32(2H,t,J=7.3Hz), 2.36-2.44(2H,m), 2.99-3.07(2H,m), 3.32(2H,t,J=5.9Hz), 3.48(2H,s), 3.90(3H,s), 4.40(2H,s), 6.30(1H,s), 7.17-7.33(5H,m), 7.73-7.80(1H,m), 8.10(1H,s)

Example 39

4-Amino-N-((1-(5-benzylaminopentyl)piperidin-4-yl)methyl)-5-chloro-2-methoxybenzamide (1.11 g) as starting compound, benzaldehyde (0.28 g) and sodium cyanoborohydride (0.33 g) were reacted and treated in the same manner as in Example 20 to give 0.3 g of 4-amino-5-chloro-N-((1-(5-dibenzylaminopentyl)piperidin-4-yl)methyl)-2-methoxybenzamide.

$^1$H-NMR ($CDCl_3$,ppm)δ: 1.21-2.07(13H,m), 2.31-2.43(4H,m), 2.94-3.01(2H,m), 3.32(2H,t,J=5.9Hz), 3.53(4H,s), 3.90(3H,s), 4.37(2H,s), 6.29(1H,s),7.18-7.38(10H,m), 7.72-7.78(1H,m), 8.10(1H,s)

Example 40

4-Amino-5-chloro-N-((1-(5-cyclohexylaminopentyl)piperidin-4-yl)methyl)-2-methoxybenzamide (1.1 g) as starting compound, acetaldehyde (0.15 ml) and sodium cyanoborohydride (0.33 g) were reacted and treated in the same manner as in Example 20 to give 1.0 g of 4-amino-5-chloro-N-((1-(5-(N-ethyl-N-cyclohexylamino)pentyl)piperidin-4-yl)methyl)-2-methoxybenzamide.

$^1$H-NMR ($CDCl_3$,ppm)δ: 1.01(3H,t,J=7.3Hz), 1.03-1.96(23H,m), 2.26-2.33(2H,m), 2.39-2.58(5H,m), 2.89-2.96(2H,m), 3.32(2H,t,J=6.6Hz), 3.89(3H,s), 4.40(2H,s), 6.29(1H,s), 7.71-7.77(1H,m), 8.10(1H,s)

Example 41

4-Amino-5-chloro-N-((1-(3-(3,4-dichlorobenzylamino)propyl)piperidin-4-yl)methyl)-2-methoxybenzamide (2.0 g) as starting compound, acetaldehyde (0.27 ml) and sodium cyanoborohydride (0.3 g) were reacted and treated in the same manner as in Example 20 to give 0.7 g of 4-amino-5-chloro-N-((1-(3-(N-(3,4-dichlorobenzyl)-N-ethylamino)propyl) piperidin-4-yl)methyl)-2-methoxybenzamide.

$^1$H-NMR ($CDCl_3$,ppm)δ: 1.01(3H,t,J=7.3Hz), 1.25-1.45(2H,m), 1.59-1.78(5H,m), 1.92-2.04(2H,m), 2.32-2.54(6H,m), 2.92-3.00(2H,m), 3.32(2H,t,J=6.7Hz), 3.48(2H,s), 3.89(3H,s), 4.42(2H,s), 6.30(1H,s), 7.15(1H,dd,J=2.0,7.9Hz), 7.35(1H,d,J=7.9Hz), 7.42(1H,d,J=2.0Hz), 7.71-7.79(1H,m), 8.09(1H,s)

Example 42

4-Amino-5-chloro-N-((1-(4-(3,4-dichlorobenzylamino)butyl)piperidin-4-yl)methyl)-2-methoxybenzamide (1.9 g) as starting compound, acetaldehyde (0.26 ml) and sodium cyanoborohydride (0.3 g) were reacted and treated in the same manner as in Example 20 to give 1.1 g of 4-amino-5-chloro-N-((1-(4-(N-(3,4-dichlorobenzyl)-N-ethylamino)butyl) piperidin-4-yl)methyl)-2-methoxybenzamide.

$^1$H-NMR ($CDCl_3$,ppm)δ: 1.01(3H,t,J=7.3Hz), 1.24-1.77(9H,m), 1.86-1.98(2H,m), 2.26-2.53(6H,m), 2.88-2.96(2H,m), 3.32(2H,t,J=6.6Hz), 3.47(2H,s),3.89(3H,s), 4.41(2H,s), 6.30(1H,s), 7.15(1H,dd,J=2.0,7.9Hz), 7.35(1H,d,J=7.9Hz), 7.44(1H,d,J=2.0Hz), 7.71-7.78(1H,m), 8.10(1H,s)

Example 43

4-Amino-N-((1-(4-benzylaminobutyl)piperidin-4-yl)methyl)-5-chloro-2-methoxybenzamide (0.5 g) as starting compound, acetaldehyde (0.07 ml) and sodium cyanoborohydride (0.15 g) were reacted and treated in the same manner as in Example 20 to give 0.35 g of 4-amino-5-chloro-N-((1-(4-(N-ethyl-N-benzylamino)butyl)piperidin-4-yl)methyl)-2-methoxybenzamide.

$^1$H-NMR ($CDCl_3$,ppm)δ: 1.02(3H,t,J=7.3Hz), 1.19-1.95(11H,m), 2.26-2.32(2H,m), 2.39-2.54(4H,m), 2.89-2.96(2H,m), 3.32(2H,t,J=6.6Hz), 3.54(2H,s), 3.89(3H,s), 4.38(2H,s), 6.29(1H,s), 7.18-7.35(5H,m), 7.71-7.78(1H,m), 8.10(1H,s)

Example 44

4-Amino-N-((1-(6-benzylaminohexyl)piperidin-4-yl)methyl)-5-chloro-2-methoxybenzamide (1.3 g) as starting compound, acetaldehyde (0.16 ml) and sodium cyanoborohydride (0.37 g) were reacted and treated in the same manner as in Example 20 to give 0.25 g of 4-amino-5-chloro-N-((1-(6-(N-ethyl-N-benzylamino)hexyl)piperidin-4-yl)methyl)-2-methoxy benzamide.

$^1$H-NMR ($CDCl_3$,ppm)δ: 1.02(3H,t,J=7.3Hz), 1.23-1.76(13H,m), 1.86-1.97(2H,m), 2.25-2.33(2H,m), 2.37-2.43(2H,m), 2.49(2H,q,J=7.3Hz), 2.91-2.97(2H,m), 3.32(2H,t,J=5.9Hz), 3.54(2H,s), 3.90(3H,s), 4.37(2H,s), 6.29(1H,s), 7.19-7.35(5H,m), 7.70-7.77(1H,m), 8.11(1H,s)

Example 45

4-Amino-5-chloro-2-methoxy-N-((1-(6-(4-methylbenzylamino)hexyl)piperidin-4-yl)methyl)benzamide (0.77 g) as starting compound, acetaldehyde (0.1 ml) and sodium cyanoborohydride (0.23 g) were reacted and treated in the same manner as in Example 20 to give 0.42 g of 4-amino-5-chloro-N-((1-(6-(N-ethyl-N-(4-methylbenzyl)amino)hexyl)piperidin-4-yl)methyl)-2-methoxybenzamide.

$^1$H-NMR ($CDCl_3$,ppm)δ: 1.01(3H,t,J=7.3Hz), 1.21-2.04(15H,m), 2.24-2.31(2H,m), 2.33(3H,s), 2.36-2.42(2H,m), 2.48(2H,q,J=7.3Hz), 2.89-2.96(2H,m), 3.32(2H,t,J=6.6Hz), 3.51(2H,s), 3.89(3H,s), 4.38(2H,s), 6.29(1H,s), 7.10(2H,d,J=7.9Hz), 7.20(2H,d,J=7.9Hz), 7.71-7.77(1H,m), 8.11(1H,s)

Example 46

4-Amino-5-chloro-2-methoxy-N-((1-(6-(4-methoxybenzylamino)hexyl)piperidin-4-yl)methyl)benzamide (0.9 g) as starting compound, acetaldehyde (0.12 ml) and sodium cyanoborohydride (0.26 g) were reacted and treated in the same manner as in Example 20 to give 0.55 g of 4-amino-5-chloro-N-((1-(6-(N-ethyl-N-(4-methoxybenzyl)amino)hexyl)piperidin-4-yl)methyl)-2-methoxybenzamide.

$^1$H-NMR ($CDCl_3$,ppm)δ: 1.01(3H,t,J=7.3Hz), 1.22-1.77(13H,m), 1.86-1.97(2H,m), 2.25-2.32(2H,m), 2.38(2H,t,J=7.3Hz), 2.48(2H,q,J=7.3Hz), 2.90-2.97(2H,m), 3.32(2H,t,J=5.9Hz), 3.49(2H,s), 3.79(3H,s), 3.89(3H,s), 4.39(2H,s), 6.29(1H,s), 6.80-6.86(2H,m), 7.19-7.24(2H,m), 7.71-7.77(1H,m), 8.10(1H,s)

Example 47

4-Amino-5-chloro-2-methoxy-N-((1-(6-(4-nitrobenzylamino)hexyl)piperidin-4-yl)methyl)benzamide (0.89 g) as starting compound, acetaldehyde (0.11 ml) and sodium cyanoborohydride (0.25 g) were reacted and treated in the same manner as in Example 20 to give 0.4 g of 4-amino-5-chloro-N-((1-(6-(N-ethyl-N-(4-nitrobenzyl)amino)hexyl)piperidin-4-yl)methyl)-2-methoxybenzamide.

$^1$H-NMR ($CDCl_3$,ppm)δ: 1.02(3H,t,J=7.3Hz), 1.22-1.94(15H,m), 2.23-2.30(2H,m), 2.41(2H,t,J=6.6Hz), 2.50(2H,q,J=7.3Hz), 2.88-2.95(2H,m), 3.32(2H,t,J=5.9Hz), 3.62(2H,s), 3.90(3H,s), 4.40(2H,s), 6.30(1H,s), 7.49-7.53(2H,m), 7.72-7.78(1H,m), 8.10(1H,s), 8.13-8.17(2H,m)

Example 48

4-Amino-5-chloro-N-((1-(6-(3,4-dichlorobenzylamino)hexyl)piperidin-4-yl)methyl)-2-methoxybenzamide (1.11 g) as starting compound, acetaldehyde (0.13 ml) and sodium cyanoborohydride (0.3 g) were reacted and treated in the same manner as in Example 20 to give 0.75 g of 4-amino-5-chloro-N-((1-(6-(N-(3,4-dichlorobenzyl)-N-ethylamino)hexyl)piperidin-4-yl)methyl)-2-methoxybenzamide.

$^1$H-NMR ($CDCl_3$,ppm)δ: 1.00(3H,t,J=7.3Hz), 1.20-1.95(15H,m), 2.24-2.31(2H,m), 2.38(2H,t,J=6.6Hz), 2.47(2H,q,J=7.3Hz), 2.88-2.96(2H,m), 3.32(2H,t,J=6.6Hz), 3.47(2H,s), 3.89(3H,s), 4.38(2H,s), 6.29(1H,s), 7.15(1H,dd,J=2.0,7.9Hz), 7.35(1H,d,J=7.9Hz), 7.43(1H,d,J=2.0Hz), 7.71-7.77(1H,m), 8.10(1H,s)

Example 49

4-Amino-5-chloro-2-methoxy-N-((1-(6-(2-thienylmethylamino)hexyl)piperidin-4-yl)methyl)benzamide (0.8 g) as starting compound, acetaldehyde (0.12 ml) and sodium cyanoborohydride (0.26 g) were reacted and treated in the same manner as in Example 20 to give 0.8 g of 4-amino-5-chloro-N-((1-(6-(N-ethyl-N-(2-thienylmethyl)amino)hexyl)piperidin-4-yl)methyl)-2-methoxybenzamide.

$^1$H-NMR ($CDCl_3$,ppm)δ: 1.05(3H,t,J=7.3Hz), 1.21-1.96(15H,m), 2.25-2.32(2H,m), 2.40-2.46(2H,m), 2.52(2H,q,J=7.3Hz), 2.89-2.96(2H,m), 3.32(2H,t,J=6.6Hz), 3.80(2H,s), 3.90(3H,s), 4.38(2H,s), 6.29(1H,s), 6.87-6.95(2H,m), 7.18-7.24(1H,m), 7.71-7.77(1H,m), 8.11(1H,s)

Example 50

(1) A solution (150 ml) of 5-(4-tert-butoxycarbonylaminomethylpiperidin-1-yl)pentylamine (5.0 g) and benzaldehyde (2.0 g) in ethanol was stirred at 70°C for 3 hr. After cooling, sodium borohydride (1.40 g) was added, and the mixture was stirred at room temperature for 2 hr. Water was added to the reaction mixture, and the mixture was extracted with ethyl acetate. The organic layer was washed with saturated brine, dried and the solvent was evaporated under reduced pressure. The obtained residue was purified by silica gel column chromatography to give 3.7 g of N-benzyl-5-(4-tert-butoxycarbonylaminomethylpiperidin-1-yl)pentylamine.

$^1$H-NMR ($CDCl_3$,ppm)δ: 1.23-1.88(11H,m), 1.44(9H,s), 2.63(2H,t,J=6.8Hz), 2.60-2.32(4H,m), 3.78(2H,s), 7.23-7.35(5H,m)

(2) A solution of N-benzyl-5-(4-tert-butoxycarbonylaminomethylpiperidin-1-yl)pentylamine (3.7 g) in methyl formate (30 ml) was refluxed under heating for 12 hr. After cooling, methyl formate was evaporated, and tetrahydrofuran (40 ml) and 2M trifluoroborane-dimethyl sulfide solution (19 ml) were added to the residue, which was followed by refluxing under heating for 6 hr. The solvent was evaporated, and ethanol (40 ml) and hydrochloric acid (3 ml) were added to the reaction mixture, which was followed by refluxing under heating for 3 hr. After cooling, water was added to the reaction mixture, and the mixture was extracted with ethyl acetate. The organic layer was washed with saturated brine, dried and the solvent was evaporated under reduced pressure to give 3.60 g of N-benzyl-N-methyl-5-(4-aminomethylpiperidin-1-yl)pentylamine.

$^1$H-NMR ($CDCl_3$,ppm)δ: 1.20-1.88(11H,m), 2.20(3H,s), 2.63(2H,t,J=6.8Hz), 2.60-2.32(4H,m), 3.78(2H,s), 7.25-7.36(5H,m)

(3) A suspension of N-benzyl-N-methyl-5-(4-aminomethylpiperidin-1-yl)pentylamine (3.52 g), 4-amino-5-chloro-2-methoxybenzoic acid (2.34 g), dicyclohexylcarbodiimide (2.39 g), 1-hydroxybenzotriazole (1.57 g) and triethylamine (2.35 g) in N,N-dimethylformamide (40 ml) was stirred at room temperature for 12 hr. The solvent was evaporated, and water was added to the residue, which was followed by extraction with chloroform. The organic layer was washed with saturated brine, dried and the solvent was evaporated under reduced pressure. The obtained residue was purified by silica gel column chromatography to give 0.86 g of 4-amino-5-chloro-2-methoxy-N-((1-(5-(N-benzyl-N-methylamino)pentyl)piperidin-4-yl)methyl)benzamide.

$^1$H-NMR ($CDCl_3$,ppm)δ: 1.22-1.97(13H,m), 2.17(3H,s), 2.27-2.38(4H,m), 2.91-2.96(2H,m), 3.32(2H,t,J=6.6Hz), 3.48(2H,s), 3.89(3H,s), 4.38(2H,s),6.29(1H,s), 7.19-7.34(5H,m), 7.71-7.77(1H,m), 8.10(1H,s)

Example 51

(1) A solution of 4-(3-tert-butoxycarbonylaminomethylpyrrolidin-1-yl)butylamine (2.0 g) and benzaldehyde (0.86 g) in ethanol (50 ml) was stirred at 70°C for 3 hr. After cooling, sodium borohydride was added, and the mixture was stirred at room temperature for 2 hr. Water was added to the reaction mixture, and the mixture was extracted with ethyl acetate. The organic layer was washed with saturated brine, dried and the solvent was evaporated under reduced pressure. The obtained residue was purified by silica gel column chromatography to give 1.74 g of N-benzyl-4-(3-tert-butoxycarbonylaminomethylpyrrolidin-1-yl)butylamine.

$^1$H-NMR ($CDCl_3$,ppm)δ: 1.23-1.55(7H,m), 1.44(9H,s), 1.85-3.68(8H,m), 3.79(2H,s), 7.20-7.42(5H,m)

(2) Methyl formate (10 ml) was added to N-benzyl-5-(3-tert-butoxycarbonylaminomethylpyrrolidin-1-yl)butylamine (1.74 g), and the mixture was refluxed under heating for 12 hr. After cooling, methyl formate was evaporated, and tetrahydrofuran (25 ml) and 2M trifluoroborane-dimethyl sulfide solution (9.3 ml) were added, which was followed by refluxing under heating for 6 hr. The solvent was evaporated, and ethanol and hydrochloric acid were added, which was followed by refluxing under heating for 3 hr. After cooling, water was added to the reaction mixture, and the mixture was extracted with ethyl acetate. The organic layer was washed with saturated brine, dried and the solvent was evaporated under reduced pressure to give 1.06 g of N-benzyl-N-methyl-4-(3-aminomethylpyrrolidin-1-yl)butylamine.

$^1$H-NMR ($CDCl_3$,ppm)δ: 1.23-1.56(7H,m), 2.19(3H,s), 1.85-3.70(8H,m), 3.78(2H,s), 7.20-7.40(5H,m)

(3) A suspension of N-benzyl-N-methyl-4-(3-aminomethylpyrrolidin-1-yl)butylamine (1.06 g), 4-amino-5-chloro-2-methoxybenzoic acid (0.78 g), dicyclohexylcarbodiimide (0.79 g), 2-hydroxybenzotriazole (0.52 g) and triethylamine (1.1 ml) in N,N-dimethylformamide (20 ml) was stirred at room temperature for 12 hr. The solvent was evaporated, and water was added to the residue, which was followed by extraction with chloroform.

The organic layer was washed with saturated brine, dried and the solvent was evaporated under reduced pressure. The obtained residue was purified by silica gel column chromatography to give 0.19 g of 4-amino-5-chloro-2-methoxy-N-((1-(4-(N-methyl-N-benzylamino)butyl) pyrrolidin-3-yl)methyl)benzamide.

$^1$H-NMR ($CDCl_3$,ppm)δ: 1.40-1.65(5H,m), 1.95-2.06(1H,m), 2.16(3H,s), 2.26-2.76(7H,m), 3.38-3.49(4H,m), 3.73(2H,s), 3.86(3H,s), 4.40(2H,s), 6.28(1H,s), 7.20-7.31(5H,m), 7.77-7.84(1H,m), 8.09(1H,s)

Example 52

Potassium carbonate (2.2 g) and 5-phenoxypentyl chloride (1.2 g) were added to a solution of 4-amino-5-chloro-2-methoxy-N-(piperidin-4-ylmethyl)benzamide dihydrochloride (1.5 g) in dimethylformamide (40 ml), an the mixture was stirred at 70-80°C for 7.5 hr. Water was added to the reaction mixture, and the mixture was extracted with chloroform. The organic layer was washed with saturated aqueous sodium chloride solution, dried and the solvent was evaporated under reduced pressure. The obtained residue was purified by silica gel column chromatography (chloroform:methanol=5:1) to give crystals. The obtained crystals were recrystallized from ethyl acetate to give 0.82 g of 4-amino-5-chloro-2-methoxy-N-((1-(5-phenoxypentyl)piperidin-4-yl)methyl)benzamide.

m.p. 129-132°C

Example 53

4-Amino-5-chloro-2-methoxy-N-(piperidin-4-ylmethyl)benzamide dihydrochloride (1.5 g) as starting compound, potassium carbonate (2.8 g) and 5-benzyloxypentyl bromide (2.08 g) were reacted and treated in the same manner as in Example 52 to give 0.82 g of 4-amino-N-((1-(5-benzyloxypentyl)piperidin-4-yl)methyl)-5-chloro-2-methoxybenzamide.

$^1$H-NMR($CDCl_3$,ppm) δ: 1.31-1.79(11H,m), 1.99-2.11(2H,m), 2.38-2.47(2H,m), 2.99-3.07(2H,m), 3.32(2H,t,J=6.6Hz), 3.46(2H,t,J=6.6Hz), 3.89(3H,s), 4.46(2H,s), 4.48(2H,s), 6.31(1H,s), 7.21-7.38(5H,m), 7.72-7.80(1H,m), 8.09(1H,s)

Example 54

4-Amino-5-chloro-2-methoxy-N-(piperidin-4-ylmethyl)benzamide dihydrochloride (1.5 g) as starting compound, potassium carbonate (2.2 g) and 5-benzylthiopentyl chloride (1.4 g) were reacted and treated in the same manner as in Example 52 to give 0.57 g of 4-amino-N-((1-(5-benzylthiopentyl)piperidin-4-yl)methyl)-5-chloro-2-methoxybenzamide.

$^1$H-NMR($CDCl_3$,ppm) δ: 1.29-1.99(13H,m), 2.27-2.34(2H,m), 2.41(2H,t,J=7.3Hz), 2.89-2.98(2H,m), 3.32(2H,t,J=6.6Hz), 3.69(2H,s), 3.90(3H,s), 4.37(2H,s), 6.29(1H,s), 7.20-7.35(5H,m), 7.71-7.77(1H,m), 8.11(1H,s)

Example 55

4-Amino-5-chloro-2-methoxy-N-(piperidin-4-ylmethyl)benzamide dihydrochloride (1.3 g) as starting compound, potassium carbonate (2.0 g) and 6-(N-methyl-N-phenylamino)hexyl chloride (0.8 g) were reacted and treated in the same manner as in Example 52 to give 1.2 g of 4-amino-5-chloro-2-methoxy-N-((1-(6-(N-methyl-N-phenylamino)hexyl)piperidin-4-yl)methyl)benzamide.

$^1$H-NMR($CDCl_3$,ppm) δ: 1.16(2H,m), 1.27(4H,m), 1.54(5H,m), 1.59(2H,m), 1.85(2H,t,J=11.2Hz), 2.25(2H,t,J=7.2Hz), 2.85(3H,s), 3.14(2H,t,J=6.4Hz), 3.27(2H,t,J=6.4Hz), 3.82(3H,s), 5.90(2H,s), 6.48(1H,s), 6.54(1H,t,J=7.3Hz), 6.65(2H,d,J=8.0Hz), 7.13(1H,t,J=9.2Hz), 7.67(1H,s), 7.87(1H,t,J=6.0Hz)

Example 56

Potassium carbonate (0.57 g) and 5-bromo-1-phenyl-1-pentanone (0.80 g) were added to a solution (45 ml) of 4-amino-5-chloro-2-methoxy-N-(piperidin-4-ylmethyl)benzamide dihydrochloride (1.5 g) in dimethylformamide, and the mixture was stirred at 70-80°C for 7.5 hr. Water was added to the reaction mixture, and the mixture was extracted with chloroform. The organic layer was washed with saturated aqueous sodium chloride solution, dried and the solvent was evaporated under reduced pressure. The obtained residue was purified by silica gel column chromatography to give crystals. The obtained crystals were recrystallized from ethyl acetate to give 0.80 g of 4-amino-5-chloro-2-methoxy-N-((1-(5-oxo-5-phenylpentyl)piperidin-4-yl)methyl)benzamide.

m.p. 100-102°C

Example 57

4-Amino-5-chloro-2-methoxy-N-(piperidin-4-ylmethyl)benzamide dihydrochloride (2.0 g) as starting compound, potassium carbonate (1.9 g) and 6-bromo-1-phenyl-1-hexanone (1.7 g) were reacted and treated in the same manner as in Example 56 to give 0.56 g of 4-amino-5-chloro-2-methoxy-N-((1-(6-oxo-6-phenylhexyl)piperidin-4-yl)methyl)benzamide.

m.p. 138-141°C

Example 58

4-Amino-5-chloro-2-methoxy-N-(piperidin-4-ylmethyl)benzamide dihydrochloride (1.3 g) as starting compound, potassium carbonate (2.1 g) and 7-bromo-1-phenyl-1-heptanone (0.92 g) were reacted and treated in the same manner as in Example 56 to give 0.80 g of 4-amino-5-chloro-2-methoxy-N-((1-(7-oxo-7-phenylheptyl)piperidin-4-yl)methyl)benzamide.

m.p. 128-130°C

Example 59

4-Amino-5-chloro-2-methoxy-N-(piperidin-4-ylmethyl)benzamide dihydrochloride (1.5 g) as starting compound, potassium carbonate (2.5 g) and 8-bromo-1-phenyl-1-octanone (1.3 g) were reacted and treated in the same manner as in Example 56 to give 0.70 g of 4-amino-5-chloro-2-methoxy-N-((1-(8-oxo-8-phenyloctyl)piperidin-4-yl)methyl)benzamide.

m.p. 138-140°C

Example 60

4-Amino-5-chloro-2-methoxy-N-(piperidin-4-ylmethyl)benzamide dihydrochloride (1.5 g) as starting compound, potassium carbonate (2.5 g) and 6-bromo-1-(4-methylphenyl)-1-hexanone (1.3 g) were reacted and treated in the same manner as in Example 56 to give 0.87 g of 4-amino-5-chloro-2-methoxy-N-((1-(6-(4-methylphenyl)-6-oxohexyl)piperidin-4-yl)methyl)benzamide.

m.p. 130-131°C

Example 61

4-Amino-5-chloro-2-methoxy-N-(piperidin-4-ylmethyl)benzamide dihydrochloride (1.1 g) as starting compound, potassium carbonate (2.0 g) and 6-bromo-1-(2,4-dimethylphenyl)-1-hexanone (0.94 g) were reacted and treated in the same manner as in Example 56 to give 0.63 g of 4-amino-5-chloro-N-((1-(6-(2,4-dimethylphenyl)-6-oxohexyl)piperidin-4-yl)methyl)-2-methoxybenzamide.

m.p. 106-110°C

Example 62

4-Amino-5-chloro-2-methoxy-N-(piperidin-4-ylmethyl)benzamide dihydrochloride (2.0 g) as starting compound, potassium carbonate (3.0 g) and 6-bromo-1-(3,4-dimethylphenyl)-1-hexanone (1.1 g) were reacted and treated in the same manner as in Example 56 to give 0.82 g of 4-amino-5-chloro-N-((1-(6-(3,4-dimethylphenyl)-6-oxohexyl)piperidin-4-yl)methyl)-2-methoxybenzamide.

m.p. 115-117°C

Example 63

4-Amino-5-chloro-2-methoxy-N-(piperidin-4-ylmethyl)benzamide dihydrochloride (1.1 g) as starting compound, potassium carbonate (1.0 g) and 6-bromo-1-(4-ethylphenyl)-1-hexanone (0.94 g) were reacted and treated in the same manner as in Example 56 to give 0.34 g of 4-amino-5-chloro-N-((1-(6-(4-ethylphenyl)-6-oxohexyl)piperidin-4-yl)methyl)-2-methoxybenzamide.

m.p. 140-145°C

Example 64

4-Amino-5-chloro-2-methoxy-N-(piperidin-4-ylmethyl)benzamide dihydrochloride (1.5 g) as starting compound, potassium carbonate (2.5 g) and 6-bromo-1-(4-methoxyphenyl)-1-hexanone (1.4 g) were reacted and treated in the same manner as in Example 56 to give 1.4 g of 4-amino-5-chloro-N-((1-(6-(4-methoxyphenyl)-6-oxohexyl)piperidin-4-yl)methyl)-2-methoxybenzamide.

m.p. 133-135°C

Example 65

4-Amino-5-chloro-2-methoxy-N-(piperidin-4-ylmethyl)benzamide dihydrochloride (1.1 g) as starting compound, potassium carbonate (2.0 g) and 6-bromo-1-(3,4-dimethoxyphenyl)-1-hexanone (1.0 g) were reacted and treated in the same manner as in Example 56 to give 0.56 g of 4-amino-5-chloro-N-((1-(6-(3,4-dimethoxyphenyl)-6-oxohexyl)piperidin-4-yl)methyl)-2-methoxybenzamide.

m.p. 102-105°C

Example 66

4-Amino-5-chloro-2-methoxy-N-(piperidin-4-ylmethyl)benzamide dihydrochloride (1.5 g) as starting compound, potassium carbonate (2.5 g) and 6-bromo-1-(4-chlorophenyl)-1-hexanone (1.3 g) were reacted and treated in the same manner as in Example 56 to give 1.0 g of 4-amino-5-chloro-N-((1-(6-(4-chlorophenyl)-6-oxohexyl)piperidin-4-yl)methyl)-2-methoxybenzamide.

m.p. 156-158°C

Example 67

4-Amino-5-chloro-2-methoxy-N-(piperidin-4-ylmethyl)benzamide dihydrochloride (1.6 g) as starting compound, potassium carbonate (2.3 g) and 7-bromo-1-(4-chlorophenyl)-1-heptanone (2.2 g) were reacted and treated in the same manner as in Example 56 to give 0.60 g of 4-amino-5-chloro-N-((1-(7-(4-chlorophenyl)-7-oxoheptyl)piperidin-4-yl)methyl)-2-methoxybenzamide.

m.p. 137-138°C

Example 68

4-Amino-5-chloro-2-methoxy-N-(piperidin-4-ylmethyl)benzamide dihydrochloride (0.23 g) as starting compound, potassium carbonate (0.34 g) and 6-bromo-1-(2,4-dichlorophenyl)-1-hexanone (0.20 g) were reacted and treated in the same manner as in Example 56 to give 0.11 g of 4-amino-5-chloro-N-((1-(6-(2,4-dichlorophenyl)-6-oxohexyl)piperidin-4-yl)methyl)-2-methoxybenzamide.

m.p. 150-155°C

Example 69

4-Amino-5-chloro-2-methoxy-N-(piperidin-4-ylmethyl)benzamide dihydrochloride (0.51 g) as starting compound, potassium carbonate (0.77 g) and 6-bromo-1-(3,4-dichlorophenyl)-1-hexanone (0.45 g) were reacted and treated in the same manner as in Example 56 to give 50 mg of 4-amino-5-chloro-N-((1-(6-(3,4-dichlorophenyl)-6-oxohexyl)piperidin-4-yl)methyl)-2-methoxybenzamide.

m.p. 203-205°C

Example 70

4-Amino-5-chloro-2-methoxy-N-(piperidin-4-ylmethyl)benzamide dihydrochloride (1.5 g) as starting compound, potassium carbonate (2.5 g) and 6-bromo-1-(4-fluorophenyl)-1-hexanone (1.3 g) were reacted and treated in the same manner as in Example 56 to give 1.0 g of 4-amino-5-chloro-N-((1-(6-(4-fluorophenyl)-6-oxohexyl)piperidin-4-yl)methyl)-2-methoxybenzamide.

m.p. 137-139°C

Example 71

4-Amino-5-chloro-2-methoxy-N-(piperidin-4-ylmethyl)benzamide dihydrochloride (1.0 g) as starting compound, potassium carbonate (1.5 g) and 6-bromo-1-(2,4-difluorophenyl)-1-hexanone (0.9 g) were reacted and treated in the

same manner as in Example 56 to give 0.55 g of 4-amino-5-chloro-N-((1-(6-(2,4-difluorophenyl)-6-oxohexyl)piperidin-4-yl)methyl)-2-methoxybenzamide.

m.p. 115-117°C

Example 72

4-Amino-5-chloro-2-methoxy-N-(piperidin-4-ylmethyl)benzamide dihydrochloride (0.57 g) as starting compound, potassium carbonate (0.82 g), and 6-bromo-1-(3,4-difluorophenyl)-1-hexanone (0.42 g) were reacted and treated in the same manner as in Example 56 to give 0.21 g of 4-amino-5-chloro-N-((1-(6-(3,4-difluorophenyl)-6-oxohexyl)piperidin-4-yl)methyl)-2-methoxybenzamide.

m.p. 177-179°C

Example 73

4-Amino-5-chloro-2-methoxy-N-(piperidin-4-ylmethyl)benzamide dihydrochloride (0.86 g) as starting compound, potassium carbonate (2.0 g) and 6-bromo-1-(3-chloro-4-methoxyphenyl)-1-hexanone (0.82 g) were reacted and treated in the same manner as in Example 56 to give 0.45 g of 4-amino-5-chloro-N-((1-(6-(3-chloro-4-methoxyphenyl)-6-oxohexyl)piperidin-4-yl)methyl)-2-methoxybenzamide.

m.p. 104-106°C

Example 74

4-Amino-5-chloro-2-methoxy-N-(piperidin-4-ylmethyl)benzamide dihydrochloride (1.0 g) as starting compound, potassium carbonate (1.5 g) and 6-bromo-1-(3-fluoro-4-methoxyphenyl)-1-hexanone (0.94 g) were reacted and treated in the same manner as in Example 56 to give 0.90 g of 4-amino-5-chloro-N-((1-(6-(3-fluoro-4-methoxyphenyl)-6-oxohexyl)piperidin-4-yl)methyl)-2-methoxybenzamide.

m.p. 112-113°C

Example 75

4-Amino-5-chloro-2-methoxy-N-(piperidin-4-ylmethyl)benzamide dihydrochloride (0.57 g) as starting compound, potassium carbonate (0.82 g) and 6-bromo-1-(4-hydroxyphenyl)-1-hexanone (0.42 g) were reacted and treated in the same manner as in Example 56 to give 0.21 g of 4-amino-5-chloro-N-((1-(6-(4-hydroxyphenyl)-6-oxohexyl)piperidin-4-yl)methyl)-2-methoxybenzamide.

m.p. 177-179°C

Example 76

4-Amino-5-chloro-2-methoxy-N-(piperidin-4-ylmethyl)benzamide dihydrochloride (2.0 g) as starting compound, potassium carbonate (3.0 g) and 6-bromo-1-(2-thienyl)-1-hexanone (1.6 g) were reacted and treated in the same manner as in Example 56 to give 0.27 g of 4-amino-5-chloro-N-((1-(6-oxo-6-(2-thienyl)hexyl)piperidin-4-yl)methyl)-2-methoxybenzamide.

m.p. 115-118°C

Example 77

4-Amino-5-chloro-2-methoxy-N-(piperidin-4-ylmethyl)benzamide dihydrochloride (1.2 g) as starting compound, potassium carbonate (1.8 g) and 6-bromo-1-(1-methyl-1H-indol-3-yl)-1-hexanone (1.0 g) were reacted and treated in the same manner as in Example 56 to give 0.71 g of 4-amino-5-chloro-2-methoxy-N-((1-(6-(1-methyl-1H-indol-3-yl)-6-oxohexyl)piperidin-4-yl)methyl)benzamide.

m.p. 125-127°C

Example 78

4-Amino-5-chloro-2-methoxy-N-(piperidin-4-ylmethyl)benzamide dihydrochloride (0.54 g) as starting compound, potassium carbonate (0.8 g) and 6-bromo-1-(2-benzo[b]thienyl)-1-hexanone (0.45 g) were reacted and treated in the same manner as in Example 56 to give 0.38 g of 4-amino-5-chloro-N-((1-(6-(2-benzo[b]thienyl)-6-oxohexyl)piperidin-4-yl)methyl)-2-methoxybenzamide.

m.p. 157-159°C

Example 79

4-Amino-5-chloro-2-methoxy-N-(piperidin-4-ylmethyl)benzamide dihydrochloride (0.50 g) as starting compound, potassium carbonate (0.75 g) and 6-bromo-1-(3-benzo[b]thienyl)-1-hexanone (0.42 g) were reacted and treated in the same manner as in Example 56 to give 0.32 g of 4-amino-5-chloro-N-((1-(6-(3-benzo[b]thienyl)-6-oxohexyl)piperidin-4-yl)methyl)-2-methoxybenzamide.

m.p. 130-132°C

Example 80

4-Amino-5-chloro-2-methoxy-N-(piperidin-4-ylmethyl)benzamide dihydrochloride (0.88 g) as starting compound, potassium carbonate (2.0 g) and 6-bromo-1-(3,4-methylenedioxyphenyl)-1-hexanone (0.80 g) were reacted and treated in the same manner as in Example 56 to give 0.62 g of 4-amino-5-chloro-2-methoxy-N-((1-(6-(3,4-methylenedioxyphenyl)-6-oxohexyl)piperidin-4-yl)methyl)benzamide.

m.p. 163°C (decomposition)

Example 81

4-Amino-5-chloro-2-methoxy-N-(piperidin-4-ylmethyl)benzamide dihydrochloride as starting compound and 6-bromo-1-(1-naphthyl)-1-hexanone were reacted and treated in the same manner as in Example 56 to give 4-amino-5-chloro-N-((1-(6-(1-naphthyl)-6-oxohexyl)piperidin-4-yl)methyl)-2-methoxybenzamide hydrochloride.

m.p. 144-146°C

Example 82

4-Amino-5-chloro-2-methoxy-N-(piperidin-4-ylmethyl)benzamide dihydrochloride (0.75 g) as starting compound, potassium carbonate (2.0 g) and 6-bromo-1-(2-naphthyl)-1-hexanone (0.80 g) were reacted and treated in the same manner as in Example 56 to give 0.40 g of 4-amino-5-chloro-N-((1-(6-(2-naphthyl)-6-oxohexyl)piperidin-4-yl)methyl)-2-methoxybenzamide.

m.p. 113-116°C

Example 83

Potassium carbonate (0.87 g) and 4-phenylsulfonylbutyl chloride (0.40 g) were added to a solution (15 ml) of 4-amino-5-chloro-2-methoxy-N-(piperidin-4-ylmethyl)benzamide dihydrochloride (0.52 g) in dimethylformamide, and the mixture was stirred at 70-80°C for 7.5 hr. Water was added to the reaction mixture, and the mixture was extracted with chloroform. The organic layer was washed with saturated brine and dried. The solvent was evaporated under reduced pressure, and the obtained residue was purified by silica gel column chromatography. The obtained oil was treated with hydrochloric acid-ethanol and the obtained crystals were recrystallized from ethyl acetate to give 0.10 g of 4-amino-5-chloro-2-methoxy-N-((1-(4-phenylsulfonylbutyl)piperidin-4-yl)methyl)benzamide hydrochloride.

m.p. 201-203°C

Example 84

4-Amino-5-chloro-2-methoxy-N-(piperidin-4-ylmethyl)benzamide dihydrochloride (0.48 g) as starting compound, potassium carbonate (0.80 g) and 5-phenylsulfonylpentyl chloride (0.40 g) were reacted and treated in the same manner as in Example 83 to give 0.17 g of 4-amino-5-chloro-2-methoxy-N-((1-(5-phenylsulfonylpentyl)piperidin-4-yl)methyl)benzamide hydrochloride.

m.p. 90-92°C

Example 85

30% Aqueous hydrogen peroxide solution (0.22 ml) was added to a solution (10 ml) of 4-amino-5-chloro-2-methoxy-N-((1-(6-phenylthiohexyl)piperidin-4-yl)methyl)benzamide (1.3 g) in formic acid, and the mixture was stirred at room temperature for 2 hr. Water was added to the reaction mixture, and the mixture was extracted with chloroform. The organic layer was washed with saturated aqueous sodium chloride solution and dried. The solvent was evaporated under reduced pressure, and the obtained residue was purified by silica gel column chromatography. The obtained oil was treated with hydrochloric acid-ethanol and the obtained crystals were recrystallized from ethyl acetate to give 0.17 g of 4-amino-5-chloro-2-methoxy-N-((1-(6-phenylsulfonylhexyl)piperidin-4-yl)methyl)benzamide hydrochloride.

m.p. 90-92°C

Example 86

4-Amino-5-chloro-2-methoxy-N-(piperidin-4-ylmethyl)benzamide dihydrochloride as starting compound and 5-phenylsulfinylpentyl chloride were reacted and treated in the same manner as in Example 83 to give 4-amino-5-chloro-2-methoxy-N-((1-(5-phenylsulfinylpentyl)piperidin-4-yl)methyl)benzamide.

$^1$H-NMR($COCl_3$,ppm) δ:1.23-1.97(11H,m), 2.26-2.31(2H,m), 2.78(2H,t,J=9Hz), 2.88-2.93(2H,m), 3.32(2H,t,J=6.1Hz), 3.90(3H,s), 4.38(2H,s), 6.29(1H,s), 7.48-7.63(5H,m), 7.69-7.80(1H,m), 8.10(1H,s)

Example 87

4-Amino-5-chloro-2-methoxy-N-(piperidin-4-ylmethyl)benzamide dihydrochloride (0.48 g) as starting compound, potassium carbonate (0.80 g) and 6-phenylsulfonylhexyl chloride (0.40 g) were reacted and treated in the same manner as in Example 83 to give 0.17 g of 4-amino-5-chloro-2-methoxy-N-((1-(6-phenylsulfonylhexyl)piperidin-4-yl)methyl)benzamide hydrochloride.

m.p. 90-92°C

Example 88

4-Amino-5-chloro-2-methoxy-N-(piperidin-4-ylmethyl)benzamide dihydrochloride as starting compound and 5-bromo-1-(1-methyl-1H-indol-3-yl)-1-pentanone were reacted and treated in the same manner as in Example 56 to give 4-amino-5-chloro-2-methoxy-N-((1-(5-(1-methyl-1H-indol-3-yl)-5-oxopentyl)piperidin-4-yl)methyl)benzamide.

m.p. 87-89°C

Example 89

4-Amino-5-chloro-2-methoxy-N-(piperidin-4-ylmethyl)benzamide dihydrochloride as starting compound and 6-bromo-1-(2-methyl-3-benzo[b]furyl)-1-hexanone were reacted and treated in the same manner as in Example 56 to give 4-amino-5-chloro-2-methoxy-N-((1-(6-(2-methyl-3-benzo[b]furyl)-6-oxohexyl)piperidin-4-yl)methyl)benzamide.

m.p. 133-135°C

Example 90

4-Amino-5-chloro-2-methoxy-N-(piperidin-4-ylmethyl)benzamide dihydrochloride as starting compound and 6-bromo-1-(3-chlorophenyl)-1-hexanone were reacted and treated in the same manner as in Example 56 to give 4-amino-5-chloro-2-methoxy-N-((1-(6-(3-chlorophenyl)-6-oxohexyl)piperidin-4-yl)methyl)benzamide hydrochloride.

m.p. 128-132°C

Example 91

4-Amino-5-chloro-2-methoxy-N-(piperidin-4-ylmethyl)benzamide dihydrochloride as starting compound and 7-bromo-1-(1-methyl-1H-indol-3-yl)-1-heptanone were reacted and treated in the same manner as in Example 56 to give 4-amino-5-chloro-2-methoxy-N-((1-(7-(1-methyl-1H-indol-3-yl)-7-oxoheptyl)piperidin-4-yl)methyl)benzamide hydrochloride.

m.p. 222-224°C

Example 92

4-Amino-5-chloro-2-methoxy-N-(piperidin-4-ylmethyl)benzamide dihydrochloride as starting compound and 6-bromo-1-(1H-indol-3-yl)-1-hexanone are reacted and treated in the same manner as in Example 56 to give 4-amino-5-chloro-2-methoxy-N-((1-(6-(1H-indol-3-yl)-6-oxohexyl)piperidin-4-yl)methyl)benzamide.

Example 93

4-Amino-5-chloro-2-methoxy-N-(piperidin-4-ylmethyl)benzamide dihydrochloride as starting compound and 6-bromo-1-(1-ethyl-1H-indol-3-yl)-1-hexanone are reacted and treated in the same manner as in Example 56 to give 4-amino-5-chloro-2-methoxy-N-((1-(6-(1-ethyl-1H-indol-3-yl)-6-oxohexyl)piperidin-4-yl)methyl)benzamide.

Example 94

4-Amino-5-chloro-2-methoxy-N-(piperidin-4-ylmethyl)benzamide dihydrochloride as starting compound and 6-bromo-1-(1-propyl-1H-indol-3-yl)-1-hexanone are reacted and treated in the same manner as in Example 56 to give 4-amino-5-chloro-2-methoxy-N-((1-(6-(1-propyl-1H-indol-3-yl)-6-oxohexyl)piperidin-4-yl)methyl)benzamide.

Example 95

4-Amino-5-chloro-2-methoxy-N-(piperidin-4-ylmethyl)benzamide dihydrochloride as starting compound and 6-bromo-1-(1-isopropyl-1H-indol-3-yl)-1-hexanone are reacted and treated in the same manner as in Example 56 to give 4-amino-5-chloro-2-methoxy-N-((1-(6-(1-isopropyl-1H-indol-3-yl)-6-oxohexyl)piperidin-4-yl)methyl)benzamide.

Example 96

4-Amino-5-chloro-2-methoxy-N-(piperidin-4-ylmethyl)benzamide dihydrochloride as starting compound and 6-bromo-1-(1-cyclohexylmethyl-1H-indol-3-yl)-1-hexanone are reacted and treated in the same manner as in Example 56 to give 4-amino-5-chloro-2-methoxy-N-((1-(6-(1-cyclohexylmethyl-1H-indol-3-yl)-6-oxohexyl)piperidin-4-yl)methyl)benzamide.

Example 97

4-Amino-5-chloro-2-methoxy-N-(piperidin-4-ylmethyl)benzamide dihydrochloride as starting compound and 6-bromo-1-(1-benzyl-1H-indol-3-yl)-1-hexanone are reacted and treated in the same manner as in Example 56 to give 4-amino-5-chloro-2-methoxy-N-((1-(6-(1-benzyl-1H-indol-3-yl)-6-oxohexyl)piperidin-4-yl)methyl)benzamide.

Example 98

4-Amino-5-chloro-2-methoxy-N-(piperidin-4-ylmethyl)benzamide dihydrochloride as starting compound and 6-bromo-1-(1,5-dimethyl-1H-indol-3-yl)-1-hexanone are reacted and treated in the same manner as in Example 56 to give 4-amino-5-chloro-2-methoxy-N-((1-(6-(1,5-dimethyl-1H-indol-3-yl)-6-oxohexyl)piperidin-4-yl)methyl)benzamide.

Example 99

4-Amino-5-chloro-2-methoxy-N-(piperidin-4-ylmethyl)benzamide dihydrochloride as starting compound and 6-bromo-1-(5-chloro-1-methyl-1H-indol-3-yl)-1-hexanone are reacted and treated in the same manner as in Example 56 to give 4-amino-5-chloro-2-methoxy-N-((1-(6-(5-chloro-1-methyl-1H-indol-3-yl)-6-oxohexyl)piperidin-4-yl)methyl)benzamide.

Example 100

4-Amino-5-chloro-2-methoxy-N-(piperidin-4-ylmethyl)benzamide dihydrochloride as starting compound and 6-bromo-1-(5-methoxy-1-methyl-1H-indol-3-yl)-1-hexanone are reacted and treated in the same manner as in Example 56 to give 4-amino-5-chloro-2-methoxy-N-((1-(6-(5-methoxy-1-methyl-1H-indol-3-yl)-6-oxohexyl)piperidin-4-yl)methyl)benzamide.

Example 101

4-Amino-5-chloro-2-methoxy-N-(piperidin-4-ylmethyl)benzamide dihydrochloride as starting compound and 6-bromo-1-(3-benzisothiazolyl)-1-hexanone are reacted and treated in the same manner as in Example 56 to give 4-amino-5-chloro-2-methoxy-N-((1-(6-(3-benzisothiazolyl)-6-oxohexyl)piperidin-4-yl)methyl)benzamide.

Example 102

4-Amino-5-chloro-2-methoxy-N-(piperidin-4-ylmethyl)benzamide dihydrochloride as starting compound and 6-bromo-1-(3-benzisoxazolyl)-1-hexanone are reacted and treated in the same manner as in Example 56 to give 4-amino-5-chloro-2-methoxy-N-((1-(6-(3-benzisoxazolyl)-6-oxohexyl)piperidin-4-yl)methyl)benzamide.

Example 103

4-Amino-5-chloro-2-methoxy-N-(piperidin-4-ylmethyl)benzamide dihydrochloride as starting compound and 6-bromo-1-(4-amino-5-chloro-2-methoxyphenyl)-1-hexanone are reacted and treated in the same manner as in Example 56 to give 4-amino-5-chloro-2-methoxy-N-((1-(6-(4-amino-5-chloro-2-methoxyphenyl)-6-oxohexyl)piperidin-4-yl)methyl)benzamide.

Example 104

4-Amino-5-chloro-2-methoxy-N-(piperidin-4-ylmethyl)benzamide dihydrochloride as starting compound and 6-bromo-1-(2-chlorophenyl)-1-hexanone are reacted and treated in the same manner as in Example 56 to give 4-amino-5-chloro-2-methoxy-N-((1-(6-(2-chlorophenyl)-6-oxohexyl)piperidin-4-yl)methyl)benzamide.

Example 105

4-Amino-5-chloro-2-methoxy-N-(piperidin-4-ylmethyl)benzamide dihydrochloride as starting compound and 6-bromo-1-(2-chloro-4-methylphenyl)-1-hexanone are reacted and treated in the same manner as in Example 56 to give 4-amino-5-chloro-2-methoxy-N-((1-(6-(2-chloro-4-methylphenyl)-6-oxohexyl)piperidin-4-yl)methyl)benzamide.

Example 106

4-Amino-5-chloro-2-methoxy-N-(piperidin-4-ylmethyl)benzamide dihydrochloride as starting compound and 6-bromo-1-(3-chloro-4-methylphenyl)-1-hexanone are reacted and treated in the same manner as in Example 56 to give 4-amino-5-chloro-2-methoxy-N-((1-(6-(3-chloro-4-methylphenyl)-6-oxohexyl)piperidin-4-yl)methyl)benzamide.

Example 107

4-Amino-5-chloro-2-methoxy-N-(piperidin-4-ylmethyl)benzamide dihydrochloride as starting compound and 6-bromo-1-(2,3-dichlorophenyl)-1-hexanone are reacted and treated in the same manner as in Example 56 to give 4-amino-5-chloro-2-methoxy-N-((1-(6-(2,3-dichlorophenyl)-6-oxohexyl)piperidin-4-yl)methyl)benzamide.

Example 108

4-Amino-5-chloro-2-methoxy-N-(piperidin-4-ylmethyl)benzamide dihydrochloride as starting compound and 6-bromo-1-(2-methoxyphenyl)-1-hexanone are reacted and treated in the same manner as in Example 56 to give 4-amino-5-chloro-2-methoxy-N-((1-(6-(2-methoxyphenyl)-6-oxohexyl)piperidin-4-yl)methyl)benzamide.

Example 109

4-Amino-5-chloro-2-methoxy-N-(piperidin-4-ylmethyl)benzamide dihydrochloride as starting compound and 6-bromo-1-(3-methoxyphenyl)-1-hexanone are reacted and treated in the same manner as in Example 56 to give 4-amino-5-chloro-2-methoxy-N-((1-(6-(3-methoxyphenyl)-6-oxohexyl)piperidin-4-yl)methyl)benzamide.

Example 110

4-Amino-5-chloro-2-methoxy-N-(piperidin-4-ylmethyl)benzamide dihydrochloride as starting compound and 6-bromo-1-(2-fluorophenyl)-1-hexanone are reacted and treated in the same manner as in Example 56 to give 4-amino-5-chloro-2-methoxy-N-((1-(6-(2-fluorophenyl)-6-oxohexyl)piperidin-4-yl)methyl)benzamide.

Example 111

4-Amino-5-chloro-2-methoxy-N-(piperidin-4-ylmethyl)benzamide dihydrochloride as starting compound and 6-bromo-1-(3-fluorophenyl)-1-hexanone are reacted and treated in the same manner as in Example 56 to give 4-amino-5-chloro-2-methoxy-N-((1-(6-(3-fluorophenyl)-6-oxohexyl)piperidin-4-yl)methyl)benzamide.

Example 112

4-Amino-5-chloro-2-methoxy-N-(piperidin-4-ylmethyl)benzamide dihydrochloride as starting compound and 6-bromo-1-(4-nitrophenyl)-1-hexanone are reacted and treated in the same manner as in Example 56 to give 4-amino-5-chloro-2-methoxy-N-((1-(6-(4-nitrophenyl)-6-oxohexyl)piperidin-4-yl)methyl)benzamide.

Example 113

4-Amino-5-chloro-2-methoxy-N-(piperidin-4-ylmethyl)benzamide dihydrochloride (1.50 g) as starting compound and 4-phenoxybutyl bromide (1.37 g) were reacted and treated in the same manner as in Example 52 to give 0.91 g of 4-amino-5-chloro-2-methoxy-N-((1-(4-phenoxybutyl)piperidin-4-yl)methyl)benzamide.

m.p. 71-73°C

Example 114

4-Amino-5-chloro-2-methoxy-N-(piperidin-4-ylmethyl)benzamide dihydrochloride as starting compound and 6-phenoxyhexyl bromide (1.56 g) were reacted and treated in the same manner as in Example 52 to give 0.78 g of 4-amino-5-chloro-2-methoxy-N-((1-(6-phenoxyhexyl)piperidin-4-yl)methyl)benzamide.

m.p. 113-115°C

Example 115

4-Amino-5-chloro-2-methoxy-N-(piperidin-4-ylmethyl)benzamide dihydrochloride (1.1 g) as starting compound, potassium carbonate (1.5 g) and 4-benzyloxybutyl bromide (1.0 g) were reacted and treated in the same manner as in Example 52 to give 0.32 g of 4-amino-5-chloro-2-methoxy-N-((1-(4-benzyloxybutyl)piperidin-4-yl)methyl)benzamide.

$^1$H-NMR ($CDCl_3$,ppm)δ:1.40-1.81(9H,m), 2.82(2H,t), 2.98(2H,t), 3.20(2H,t), 3.44(4H,m), 3.82(3H,s), 4.45(3H,s), 5.93(2H,s), 6.48(1H,s), 7.33-7.45(5H,m), 7.66(1H,s), 8.00(1H,t)

Example 116

4-Amino-5-chloro-2-methoxy-N-(piperidin-4-ylmethyl)benzamide dihydrochloride (1.00 g) as starting compound

and 6-benzyloxyhexyl bromide (0.75 g) were reacted and treated in the same manner as in Example 52 to give 0.45 g of 4-amino-5-chloro-2-methoxy-N-((1-(6-benzyloxyhexyl)piperidin-4-yl)methyl)benzamide.

m.p. 91-95°C

Example 117

4-Amino-5-chloro-2-methoxy-N-(piperidin-4-ylmethyl)benzamide dihydrochloride (1.0 g) as starting compound, potassium carbonate (1.5 g) and 5-(4-chlorobenzyloxy)pentyl chloride (1.5 g) were reacted and treated in the same manner as in Example 52 to give 1.3 g of 4-amino-5-chloro-2-methoxy-N-((1-(5-(4-chlorobenzyloxy)pentyl)piperidin-4-yl)methyl)benzamide.

m.p. 131-132°C

Example 118

4-Amino-5-chloro-2-methoxy-N-(piperidin-4-ylmethyl)benzamide dihydrochloride (2.0 g) as starting compound and 5-(2-phenylethyloxy)pentyl chloride were reacted and treated in the same manner as in Example 52 to give 0.69 g of 4-amino-5-chloro-2-methoxy-N-((1-(5-(2-phenylethyloxy)pentyl)piperidin-4-yl)methyl)benzamide.

m.p. 103-104°C

Example 119

4-Amino-5-chloro-2-methoxy-N-(piperidin-4-ylmethyl)benzamide dihydrochloride (2.00 g) as starting compound and 5-(2-naphthylmethoxy)pentyl chloride (1.88 g) were reacted and treated in the same manner as in Example 52 to give 1.45 g of 4-amino-5-chloro-2-methoxy-N-((1-(5-(2-naphthylmethoxy)pentyl)piperidin-4-yl)methyl)benzamide hydrochloride.

m.p. 161-165°C

Example 120

4-Amino-5-chloro-2-methoxy-N-(piperidin-4-ylmethyl)benzamide dihydrochloride (1.50 g) as starting compound and 5-(cyclohexylmethoxy)pentyl bromide (1.30 g) were reacted and treated in the same manner as in Example 52 to give 0.51 g of 4-amino-5-chloro-2-methoxy-N-((1-(5-(cyclohexylmethoxy)pentyl)piperidin-4-yl)methyl)benzamide hydrochloride.

m.p. 103-105°C

Example 121

4-Amino-5-chloro-2-methoxy-N-(piperidin-4-ylmethyl)benzamide dihydrochloride (2.5 g) as starting compound and 4-phenylthiobutyl bromide (2.48 g) were reacted and treated in the same manner as in Example 52 to give 0.84 g of 4-amino-5-chloro-2-methoxy-N-((1-(4-phenylthiobutyl)piperidin-4-yl)methyl)benzamide.

m.p. 102-105°C

Example 122

4-Amino-5-chloro-2-methoxy-N-(piperidin-4-ylmethyl)benzamide dihydrochloride (2.00 g) as starting compound and 5-phenylthiopentyl bromide (1.54 g) were reacted and treated in the same manner as in Example 52 to give 0.70 g of 4-amino-5-chloro-2-methoxy-N-((1-(5-phenylthiopentyl)piperidin-4-yl)methyl)benzamide.

m.p. 143-144°C

Example 123

4-Amino-5-chloro-2-methoxy-N-(piperidin-4-ylmethyl)benzamide dihydrochloride (2.5 g) as starting compound and 6-phenylthiohexyl chloride (2.76 g) were reacted and treated in the same manner as in Example 52 to give 1.40 g of 4-amino-5-chloro-2-methoxy-N-((1-(6-phenylthiohexyl)piperidin-4-yl)methyl)benzamide.

m.p. 68-70°C

Example 124

4-Amino-5-chloro-2-methoxy-N-(piperidin-4-ylmethyl)benzamide dihydrochloride as starting compound and 4-(3-methoxyphenoxy)butyl bromide are reacted and treated in the same manner as in Example 52 to give 4-amino-5-chloro-2-methoxy-N-((1-(4-(3-methoxyphenoxy)butyl)piperidin-4-yl)methyl)benzamide.

Example 125

4-Amino-5-chloro-2-methoxy-N-(piperidin-4-ylmethyl)benzamide dihydrochloride as starting compound and 5-(3-methoxyphenoxy)pentyl bromide were reacted and treated in the same manner as in Example 52 to give 4-amino-5-chloro-2-methoxy-N-((1-(5-(3-methoxyphenoxy)pentyl)piperidin-4-yl)methyl)benzamide.

m.p. 185-187°C

Example 126

4-Amino-5-chloro-2-methoxy-N-(piperidin-4-ylmethyl)benzamide dihydrochloride as starting compound and 4-(3-methoxybenzyloxy)butyl bromide are reacted and treated in the same manner as in Example 52 to give 4-amino-5-chloro-2-methoxy-N-((1-(4-(3-methoxybenzyloxy)butyl)piperidin-4-yl)methyl)benzamide.

Example 127

4-Amino-5-chloro-2-methoxy-N-(piperidin-4-ylmethyl)benzamide dihydrochloride as starting compound and 5-(3-methoxybenzyloxy)pentyl bromide are reacted and treated in the same manner as in Example 52 to give 4-amino-5-chloro-2-methoxy-N-((1-(5-(3-methoxybenzyloxy)pentyl)piperidin-4-yl)methyl)benzamide.

Example 128

4-Amino-5-chloro-2-methoxy-N-(piperidin-4-ylmethyl)benzamide dihydrochloride as starting compound and 4-(3,4-dimethoxyphenoxy)butyl bromide are reacted and treated in the same manner as in Example 52 to give 4-amino-5-chloro-N-((1-(4-(3,4-dimethoxyphenoxy)butyl)piperidin-4-yl)methyl)-2-methoxybenzamide.

Example 129

4-Amino-5-chloro-2-methoxy-N-(piperidin-4-ylmethyl)benzamide dihydrochloride (1.5 g) as starting compound and 5-(3,4-dimethoxyphenoxy)pentyl chloride (1.3 g) were reacted and treated in the same manner as in Example 52 to give 1.46 g of 4-amino-5-chloro-N-((1-(5-(3,4-dimethoxyphenoxy)pentyl)piperidin-4-yl)methyl)-2-methoxybenzamide.

m.p. 85-87°C

Example 130

4-Amino-5-chloro-2-methoxy-N-(piperidin-4-ylmethyl)benzamide dihydrochloride as starting compound and 4-(3,4-dimethoxybenzyloxy)butyl bromide are reacted and treated in the same manner as in Example 52 to give 4-amino-5-chloro-N-((1-(4-(3,4-dimethoxybenzyloxy)butyl)piperidin-4-yl)methyl)-2-methoxybenzamide.

Example 131

4-Amino-5-chloro-2-methoxy-N-(piperidin-4-ylmethyl)benzamide dihydrochloride as starting compound and 5-(3,4-dimethoxybenzyloxy)pentyl bromide are reacted and treated in the same manner as in Example 52 to give 4-amino-5-chloro-N-((1-(5-(3,4-dimethoxybenzyloxy)pentyl)piperidin-4-yl)methyl)-2-methoxybenzamide.

Example 132

4-Amino-5-chloro-2-methoxy-N-(piperidin-4-ylmethyl)benzamide dihydrochloride as starting compound and 4-(3,5-dimethoxyphenoxy)butyl bromide are reacted and treated in the same manner as in Example 52 to give 4-amino-5-chloro-N-((1-(4-(3,5-dimethoxyphenoxy)butyl)piperidin-4-yl)methyl)-2-methoxybenzamide.

Example 133

4-Amino-5-chloro-2-methoxy-N-(piperidin-4-ylmethyl)benzamide dihydrochloride (2.0 g) as starting compound and 5-(3,5-dimethoxyphenoxy)pentyl chloride (1.4 g) were reacted and treated in the same manner as in Example 52 to give 4-amino-5-chloro-N-((1-(5-(3,5-dimethoxyphenoxy)pentyl)piperidin-4-yl)methyl)-2-methoxybenzamide.

m.p. 112-113°C

Example 134

4-Amino-5-chloro-2-methoxy-N-(piperidin-4-ylmethyl)benzamide dihydrochloride as starting compound and 4-(3,5-dimethoxybenzyloxy)butyl bromide are reacted and treated in the same manner as in Example 52 to give 4-amino-5-chloro-N-((1-(4-(3,5-dimethoxybenzyloxy)butyl)piperidin-4-yl)methyl)-2-methoxybenzamide.

Example 135

4-Amino-5-chloro-2-methoxy-N-(piperidin-4-ylmethyl)benzamide dihydrochloride as starting compound and 5-(3,5-dimethoxybenzyloxy)pentyl bromide are reacted and treated in the same manner as in Example 52 to give 4-amino-5-chloro-N-((1-(5-(3,5-dimethoxybenzyloxy)pentyl)piperidin-4-yl)methyl)-2-methoxybenzamide.

Example 136

4-Amino-5-chloro-2-methoxy-N-(piperidin-4-ylmethyl)benzamide dihydrochloride (1.3 g) as starting compound and 5-(4-chlorophenoxy)pentyl bromide (1.0 g) were reacted and treated in the same manner as in Example 52 to give 4-amino-5-chloro-N-((1-(5-(4-chlorophenoxy)pentyl)piperidin-4-yl)methyl)-2-methoxybenzamide.

m.p. 104-105°C

Example 137

4-Amino-5-chloro-2-methoxy-N-(piperidin-4-ylmethyl)benzamide dihydrochloride (1.5 g) as starting compound and 5-(4-fluorophenoxy)pentyl bromide (1.1 g) were reacted and treated in the same manner as in Example 52 to give 0.42 g of 4-amino-5-chloro-N-((1-(5-(4-fluorophenoxy)pentyl)piperidin-4-yl)methyl)-2-methoxybenzamide.

m.p. 105-107°C

Example 138

4-Amino-5-chloro-2-methoxy-N-(piperidin-4-ylmethyl)benzamide dihydrochloride as starting compound and 4-(1-naphthyloxy)butyl bromide are reacted and treated in the same manner as in Example 52 to give 4-amino-5-chloro-N-((1-(4-(1-naphthyloxy)butyl)piperidin-4-yl)methyl)-2-methoxybenzamide.

Example 139

4-Amino-5-chloro-2-methoxy-N-(piperidin-4-ylmethyl)benzamide dihydrochloride (1.5 g) as starting compound and 5-(1-naphthyloxy)pentyl chloride (1.2 g) were reacted and treated in the same manner as in Example 52 to give 1.88 g of 4-amino-5-chloro-N-((1-(5-(1-naphthyloxy)pentyl)piperidin-4-yl)methyl)-2-methoxybenzamide.

m.p. 91-93°C

Example 140

4-Amino-5-chloro-2-methoxy-N-(piperidin-4-ylmethyl)benzamide dihydrochloride as starting compound and 4-((3,4-methylenedioxyphenyl)methoxy)butyl bromide are reacted and treated in the same manner as in Example 52 to give 4-amino-5-chloro-N-((1-(4-((3,4-methylenedioxyphenyl)methoxy)butyl)piperidin-4-yl)methyl)-2-methoxybenzamide.

Example 141

4-Amino-5-chloro-2-methoxy-N-(piperidin-4-ylmethyl)benzamide dihydrochloride (0.72 g) as starting compound and 5-((3,4-methylenedioxyphenyl)methoxy)pentyl chloride (0.5 g) were reacted and treated in the same manner as in Example 52 to give 0.4 g of 4-amino-5-chloro-N-((1-(5-((3,4-methylenedioxyphenyl)methoxy)pentyl)piperidin-4-yl)methyl)-2-methoxybenzamide.

$^1$H-NMR ($CDCl_3$,ppm)δ:1.25-1.42(4H,m), 1.47-1.75(7H,m), 1.90-1.98(2H,m), 2.22-2.36(2H,m), 2.93-2.98(2H,m), 3.32(2H,t,J=6.0Hz), 3.43(2H,t,J=6.6Hz), 3.89(3H,s), 4.38(4H,s), 5.93(2H,s), 6.28(1H,s), 6.77(2H,s), 6.83(1H,s), 7.74(1H,brs), 8.10(1H,s)

Example 142

4-Amino-5-chloro-2-methoxy-N-(piperidin-4-ylmethyl)benzamide dihydrochloride as starting compound and 4-((1,4-bendioxan-6-yl)methoxy)butyl bromide are reacted and treated in the same manner as in Example 52 to give 4-amino-5-chloro-N-((1-(4-((1,4-benzodioxan-6-yl)methoxy)butyl)piperidin-4-yl)methyl)-2-methoxybenzamide.

Example 143

4-Amino-5-chloro-2-methoxy-N-(piperidin-4-ylmethyl)benzamide dihydrochloride as starting compound and 5-((1,4-benzodioxan-6-yl)methoxy)pentyl bromide were reacted and treated in the same manner as in Example 52 to give 4-amino-5-chloro-N-((1-(5-((1,4-benzodioxan-6-yl)methoxy)pentyl)piperidin-4-yl)methyl)-2-methoxybenzamide.

$^1$H-NMR ($CDCl_3$,ppm)δ:1.37-1.81(11H,m), 2.04-2.18(2H,m), 2.45-2.51(2H,m), 3.06-3.17(2H,m), 3.32(2H,t,J=6.0Hz), 3.43(2H,t,J=6.6Hz), 3.89(3H,s), 4.24(4H,s), 4.36(2H,s), 4.45(2H,s), 6.31(1H,s), 6.79-6.88(3H,m), 7.75-7.79(1H,m), 8.08(1H,s)

Example 144

4-Amino-5-chloro-2-methoxy-N-(piperidin-4-ylmethyl)benzamide dihydrochloride as starting compound and 5-(1-naphthylthio)pentyl bromide are reacted and treated in the same manner as in Example 52 to give 4-amino-5-chloro-N-((1-(5-(1-naphthylthio)pentyl)piperidin-4-yl)methyl)-2-methoxybenzamide.

Example 145

To a solution of 4-amino-N-(1-(5-aminopentyl)piperidin-4-yl)methyl)-5-chloro-2-methoxybenzamide (1.5 g) in dimethylformamide (30 ml) were added diisopropylethylamine (1.5 g) and 2-chloropyrimidine (0.89 g), and the mixture was stirred at 100°C for 4 hr. Water was added to the reaction mixture, and the mixture was extracted with chloroform. The organic layer was washed with saturated brine, dried and the solvent was evaporated under reduced pressure. The obtained residue was purified by silica gel column chromatography (chloroform:methanol=10:1). The obtained oily substance was treated with hydrochloric acid-ethanol, and the obtained crystals were recrystallized from ethanol to give 0.28 g of 4-amino-5-chloro-2-methoxy-N-((1-(5-(2-pyrimidinylamino)pentyl)piperidin-4-yl)methyl)benzamide dihydrochloride.

m.p. 147-149°C

Example 146

4-Amino-5-chloro-2-methoxy-N-(piperidin-4-ylmethyl)benzamide dihydrochloride (0.78 g) as starting compound and 6-bromo-1-(1,2-dimethyl-1H-indol-3-yl)-1-hexanone (0.68 g) were reacted and treated in the same manner as in Example 56 to give 0.38 g of 4-amino-5-chloro-2-methoxy-N-((1-(6-(1,2-dimethyl-1H-indol-3-yl)-6-oxohexyl)piperidin-4-yl)methyl)benzamide.

m.p. 91-92°C

Example 147

4-Amino-5-chloro-2-methoxy-N-(piperidin-4-ylmethyl)benzamide dihydrochloride (2.74 g) as starting compound and 6-bromo-1-(1-butyl-1H-indol-3-yl)-1-hexanone (2.59 g) were reacted and treated in the same manner as in Example 83 to give 1.1 g of 4-amino-5-chloro-2-methoxy-N-((1-(6-(1-butyl-1H-indol-3-yl)-6-oxohexyl)piperidin-4-yl)methyl)benzamide hydrochloride.

m.p. 146-149°C

Example 148

4-Amino-5-chloro-2-methoxy-N-(piperidin-4-ylmethyl)benzamide dihydrochloride (0.58 g) as starting compound and 7-bromo-1-(3-chlorophenyl)-1-heptanone (0.45 g) were reacted and treated in the same manner as in Example 56 to give 0.11 g of 4-amino-5-chloro-2-methoxy-N-((1-(7-(3-chlorophenyl)-7-oxoheptyl)piperidin-4-yl)methyl)benzamide.

m.p. 104-105°C

Example 149

4-Amino-5-chloro-2-methoxy-N-(4-methoxypiperidin-4-ylmethyl)benzamide dihydrochloride as starting compound and 1-(4-chlorobutylsulfonyl)naphthalene are reacted and treated in the same manner as in Example 56 to give 4-amino-5-chloro-2-methoxy-N-((4-methoxy-1-(4-(1-naphthylsulfonyl)butyl)piperidin-4-yl)methyl)benzamide.

Example 150

4-Amino-5-chloro-2-methoxy-N-(4-methoxypiperidin-4-ylmethyl)benzamide dihydrochloride as starting compound and 1-(5-chloropentylsulfonyl)naphthalene are reacted and treated in the same manner as in Example 56 to give 4-amino-5-chloro-2-methoxy-N-((4-methoxy-1-(5-(1-naphthylsulfonyl)pentyl)piperidin-4-yl)methyl)benzamide.

Example 151

4-Amino-5-chloro-2-methoxy-N-(4-methoxypiperidin-4-ylmethyl)benzamide dihydrochloride as starting compound and 1-(4-chlorobutylsulfonyl)methylnaphthalene are reacted and treated in the same manner as in Example 56 to give 4-amino-5-chloro-2-methoxy-N-((4-methoxy-1-(4-((1-naphthyl)methylsulfonyl)butyl)piperidin-4-yl)methyl)benzamide.

Example 152

4-Amino-5-chloro-2-methoxy-N-(4-methoxypiperidin-4-ylmethyl)benzamide dihydrochloride as starting compound and 1-(5-chloropentylsulfonyl)methylnaphthalene are reacted and treated in the same manner as in Example 56 to give 4-amino-5-chloro-2-methoxy-N-((4-methoxy-1-(5-((1-naphthyl)methylsulfonyl)pentyl)piperidin-4-yl)methyl)benzamide.

Example 153

4-Amino-5-chloro-2-methoxy-N-(piperidin-4-ylmethyl)benzamide dihydrochloride (0.98 g) as starting compound and 7-bromo-1-(1-methyl-1H-indol-3-yl)-1-heptanone were reacted and treated in the same manner as in Example 56 to give 0.83 g of 4-amino-5-chloro-2-methoxy-N-((1-(7-(1-methyl-1H-indol-3-yl)-7-oxoheptyl)piperidin-4-yl)methyl)benzamide. This compound was treated with hydrochloric acid-ethanol to obtain its hydrochloride as crystals.

m.p. 222-224°C

Example 154

To a solution of 4-aminomethyl-4-hydroxy-1-(6-oxo-6-phenylhexyl)piperidine dihydrochloride (2.9 g) in dimethylformamide (50 ml) were added triethylamine (3.2 ml), 4-amino-5-chloro-2-methoxybenzoic acid (1.55 g) and 1-hydroxybenzotriazole (1.09 g), which was followed by the addition of 1-ethyl-3-(3-dimethylaminopropyl)carbodiimide (1.55 g) under ice cooling, and the mixture was stirred at room temperature for 6 hr. The reaction mixture was concentrated under reduced pressure, and water was added to the obtained residue, and the mixture was extracted with chloroform. The organic layer was washed successively with 10% aqueous solution of potassium carbonate and saturated brine, and dried. The solvent was evaporated under reduced pressure, and the obtained residue was purified by a silica gel column chromatography (chloroform:methanol=10:1) to give 2.98 g of 4-amino-5-chloro-N-((4-hydroxy-1-(6-oxo-6-phenylhexyl)piperidin-4-yl)methyl)-2-methoxybenzamide.

m.p. 102-103°C

Example 155

4-Aminomethyl-4-methoxy-1-(6-oxo-6-phenylhexyl)piperidine dihydrochloride (2.25 g) as starting compound, triethylamine (2.4 ml), 4-amino-5-chloro-2-methoxybenzoic acid (1.16 g), 1-hydroxybenzotriazole (0.815 g) and 1-ethyl-3-(3-dimethylaminopropyl)carbodiimide (1.16 g) were reacted and treated in the same manner as in Example 154 to give 1.43 g of 4-amino-5-chloro-2-methoxy-N-((4-methoxy-1-(6-oxo-6-phenylhexyl)piperidin-4-yl)methyl)benzamide.

m.p. 91-93°C

Example 156

4-Aminomethyl-4-methoxy-1-(4-phenylsulfonylbutyl)piperidine dihydrochloride (2.2 g) as starting compound, triethylamine (2.2 ml), 4-amino-5-chloro-2-methoxybenzoic acid (1.07 g), 1-hydroxybenzotriazole (0.75 g) and 1-ethyl-3-(3-dimethylaminopropyl)carbodiimide (1.07 g) were reacted and treated in the same manner as in Example 154 to give 1.44 g of 4-amino-5-chloro-2-methoxy-N-((4-methoxy-1-(4-phenylsulfonylbutyl)piperidin-4-yl)methyl)benzamide.

$^1$H-NMR ($CDCl_3$,ppm)δ:1.48-1.80(10H,m), 2.26-2.34(4H,m), 2.44-2.57(2H,m), 3.09-3.15(2H,m), 3.21(3H,s), 3.48(2H,d,J=5.3Hz), 3.87(3H,s), 4.46(2H,s), 6.30(1H,s), 7.53-7.70(3H,m), 7.88-7.92(3H,m), 8.08(1H,s)

Example 157

4-Amino-5-chloro-2-methoxy-N-(piperidin-4-ylmethyl)benzamide dihydrochloride (2 g) and 4-(benzylsulfonyl)butyl chloride (1.46 g) were reacted and treated in the same manner as in Example 83 to give 0.96 g of 4-amino-N-((1-(4-(benzylsulfonyl)butyl)piperidin-4-yl)methyl)-5-chloro-2-methoxybenzamide hydrochloride.

m.p. 114-116°C

Example 158

4-Amino-5-chloro-2-methoxy-N-(piperidin-4-ylmethyl)benzamide dihydrochloride and 5-bromo-1-(1-naphthyl)-1-pentanone were reacted and treated in the same manner as in Example 56 to give 4-amino-5-chloro-2-methoxy-N-((1-(4-(1-naphthoyl)butyl)piperidin-4-yl)methyl)benzamide.

$^1$H-NMR ($CDCl_3$,ppm)δ:1.35-1.88(9H,m), 1.90-2.05(2H,m), 2.38-2.43(2H,m), 2.94-3.05(2H,m), 3.07(2H,t,J=7.3Hz), 3.32(2H,t,J=6.0Hz), 3.88(3H,s), 4.41(2H,brs), 6.29(1H,s), 7.46-7.62(3H,m), 7.74(1H,br), 7.83-7.98(3H,m), 8.10(1H,s), 8.53(1H,d,J=8.6Hz)

Example 159

4-Amino-5-chloro-2-methoxy-N-(piperidin-4-ylmethyl)benzamide dihydrochloride and 5-benzylsulfonylpentyl chloride were reacted and treated in the same manner as in Example 83 to give 4-amino-5-chloro-2-methoxy-N-((1-(5-benzylsulfonylpentyl)piperidin-4-yl)methyl)benzamide.

$^1$H-NMR ($CDCl_3$,ppm)δ:1.42-1.89(11H,m), 2.26-2.35(2H,m), 2.55-2.61(2H,m), 2.86(2H,d,J=7.9Hz), 3.17-3.22(2H,m), 3.34(2H,t,J=5.9Hz), 3.91(3H,s), 4.24(2H,s), 4.41(2H,s), 6.31(1H,s), 7.40(5H,s), 7.77-7.82(1H,m), 8.08(1H,s)

Example 160

4-Amino-5-chloro-2-methoxy-N-(piperidin-4-ylmethyl)benzamide dihydrochloride and 3-phenoxypropyl chloride were reacted and treated in the same manner as in Example 52 to give 4-amino-5-chloro-2-methoxy-N-((1-(3-(phenoxy)propyl)piperidin-4-yl)methyl)benzamide.

m.p. 132-134°C

Example 161

4-Amino-5-chloro-2-methoxy-N-(piperidin-4-ylmethyl)benzamide dihydrochloride and 3-phenylthiopropyl chloride were reacted and treated in the same manner as in Example 52 to give 4-amino-5-chloro-2-methoxy-N-((1-(3-(phenylthio)propyl)piperidin-4-yl)methyl)benzamide.

m.p. 146-148°C

Example 162

4-Amino-5-chloro-2-methoxy-N-(piperidin-4-ylmethyl)benzamide dihydrochloride and 3-phenylsulfonylpropyl chloride were reacted and treated in the same manner as in Example 52 to give 4-amino-5-chloro-2-methoxy-N-((1-(3-(phenylsulfonyl)propyl)piperidin-4-yl)methyl) benzamide.

$^1$H-NMR ($CDCl_3$,ppm)δ:1.14-1.24(2H,m), 1.42-1.91(8H,m), 2.27(2H,t,J=7.0Hz), 2.67-2.80(2H,m), 3.04-3.14(2H,m), 3.22(2H,t,J=6.5Hz), 3.81(3H,s), 4.38(2H,brs), 6.23(1H,s), 7.45-7.88(5H,m), 8.01(1H,s)

Example 163

4-Amino-5-chloro-2-methoxy-N-((1-(6-aminohexyl)piperidin-4-yl)methyl)benzamide (2.0 g), 2-thiophenecarboxaldehyde (0.62 g), sodium borohydride (0.42 g) and ethanol (40 ml) were reacted and treated in the same manner as in Example 5 to give 1.0 g of 4-amino-5-chloro-2-methoxy-N-((1-(6-(2-thienylmethylamino)hexyl)piperidin-4-yl)methyl)benzamide.

$^1$H-NMR ($CDCl_3$,ppm)δ:1.20-1.95(16H,m), 2.22-2.31(2H,m), 2.38-2.49(2H,m), 2.88-2.99(2H,m), 3.34(2H,t), 3.82(2H,s), 3.91(3H,s), 4.39(2H,brs), 6.29(1H,s), 6.88(1H,d,J=2Hz), 6.93(1H,dd,J=3.3,5.5Hz), 7.20(1H,dd,J=1.3,5.2Hz), 7.67-7.82(1H,m), 8.11(1H,s)

Example 164

(1) 4-Amino-5-chloro-2-methoxy-N-(piperidin-4-ylmethyl)benzamide dihydrochloride (7.2 g) and 3,3-ethylenedioxy-3-phenylpropyl bromide (6.0 g) were reacted and treated in the same manner as in Example 56 to give 9.2 g of 4-amino-5-chloro-N-((1-(3,3-ethylenedioxy-3-phenylpropyl)piperidin-4-yl)methyl)-2-methoxybenzamide.

$^1$H-NMR ($CDCl_3$,ppm)δ:1.24-1.38(2H,m), 1.53-1.71(3H,m), 1.88-1.96(2H,m), 2.10-2.19(2H,m), 2.40-2.46(2H,m), 2.86-2.90(2H,m), 3.29(2H,t,J=5.9Hz), 3.73-3.77(2H,m), 3.86(3H,s), 3.97-4.02(2H,m), 4.47(2H,s), 6.29(1H,s), 7.28-7.45(5H,m), 7.70-7.74(1H,m), 8.07(1H,s) (2) Methanol (40 ml) and 1N hydrochloric acid (100 ml) were added to 4-amino-5-chloro-N-((1-(3,3-ethylenedioxy-3-phenylpropyl)piperidin-4-yl)methyl)-2-methoxybenzamide (8.8 g), and the mixture was stirred at refluxing temperature for 1.5 hr. The reaction mixture was further stirred under ice-cooling for 1 hr, and the precipitated crystals were collected by filtration to give 6.7 g of 4-amino-5-chloro-2-methoxy-N-((1-(3-oxo-3-phenylpropyl)piperidin-4-yl)methyl)benzamide hydrochloride. m.p. 141-143°C

Example 165

4-Amino-5-chloro-2-methoxy-N-(piperidin-4-ylmethyl)benzamide dihydrochloride (7 g) and 2-(phenylsulfonyl)ethyl bromide (5.6 g) were reacted and treated in the same manner as in Example 83 to give 2.74 g of 4-amino-5-chloro-2-methoxy-N-((1-(2-(phenylsulfonyl)ethyl)piperidin-4-yl)methyl)benzamide hydrochloride. m.p. 116-117°C

Example 166

4-Amino-5-chloro-2-methoxy-N-(piperidin-4-ylmethyl)benzamide dihydrochloride (7 g) and 4-bromo-1-phenyl-1-butanone (4.93 g) were reacted and treated in the same manner as in Example 56 to give 3.1 g of 4-amino-5-chloro-2-methoxy-N-((1-(4-oxo-4-phenylbutyl)piperidin-4-yl)methyl)benzamide.

m.p. 148-150°C

Example 167

Triethylamine (0.59 g) and 2-methylthio-2-imidazoline hydroiodide (1.4 g) were added to a solution of 4-amino-N-((1-(5-aminopentyl)piperidin-4-yl)methyl)-5-chloro-2-methoxybenzamide (1.5 g) in methanol (20 ml), and the mixture was stirred at refluxing temperature for 4 hr. A 10% aqueous sodium hydroxide solution was added to the reaction mixture, and the mixture was extracted with chloroform. The extract was washed with saturated brine and dried. The solvent was evaporated under reduced pressure, and the obtained residue was purified by silica gel column chromatography to give 0.84 g of 4-amino-5-chloro-N-((1-(5-(imidazolin-2-ylamino)pentyl)piperidin-4-yl)methyl)-2-methoxybenzamide.

$^1$H-NMR (DMSO-$d_6$,ppm)δ:1.12-1.61(11H,m), 1.75-1.82(2H,m), 2.18-2.23(2H,m), 2.79-2.83(2H,m), 3.06-3.17(4H,m), 3.52(4H,s), 3.82(3H,s), 5.92(2H,s), 6.49(1H,s), 7.66(1H,s), 7.85-7.89(1H,m)

The formulation example of the compound of the present invention as a medicament is given in the following.

| Formulation Example | (mg) |
|---|---|
| Compound of the present invention | 10.0 |
| Lactose | 109.6 |
| Microcrystalline cellulose | 27.4 |
| Light anhydrous silicic acid | 1.5 |
| Magnesium stearate | 1.5 |
| | 150 (per tablet) |

The compound of the present invention (30 g), lactose (328.8 g) and microcrystalline cellulose (82.8 g) are mixed. The mixture is compressed using a roller compactor to give flake-like compression product. Using a hammer mill, the flake-like compression product is pulverized. The pulverized product is passed through a 20-mesh sieve. Light anhydrous silicic acid (4.5 g) and magnesium stearate (4.5 g) are added and mixed. The mixture is punched with a 7.5 mm diameter pounder to give 3,000 tablets weighing 150 mg per tablet.

The bioactivity of the compounds of the present invention, isomers thereof and pharmaceutically acceptable salts thereof is explained by way of Experimental Examples.

Experimental Example 1 : 5-$HT_4$ receptor binding test

According to the method of C.J. Grossman et al. [British Journal of Pharmacology, vol. 109, pp. 618-624 (1993)], a specific 5-$HT_4$ receptor binding test was run using, as a tracer ligand, 1-methyl-1H-indole-3-carboxylic acid • 1-(2-methylsulfonylaminoethyl)-piperidin-4-ylmethyl ester substituted by tritium (hereinafter to be referred to as [3H]GR113808). Specifically, crude synapse membrane sample was prepared from guinea pig striatum, suspended in 50 mM HEPES buffer (pH 7.4) and used for testing. The test compound having several different concentrations and [3H]GR113808 (final concentration 0.1 nM) were added to this suspension and allowed to react at 37°C. Thirty minutes later, the reaction mixture was filtered by suction with cell harvester. The filter was washed with 50 mM HEPES buffer, and the radioactivity on the filter was counted by liquid scintillation counter. The non-specific binding was determined in the presence of 1 μM GR113808. The concentration of the test compound necessary for 50% inhibition ($IC_{50}$) was determined from a graph and converted to a Ki value. The results are shown in Table 1.

Experimental Example 2 : 5-$HT_3$ receptor binding test

According to the method of Peroutka et al. [European Journal of Pharmacology, vol. 148, pp. 297-299 (1988)], a specific 5-$HT_3$ receptor binding test was run using, as a tracer ligand, endo-1-methyl-N-(9-methyl-9-azabicyclo[3.3.1]non-3-yl)-1H-indazole-3-carboxamide substituted by tritium (hereinafter to be referred to as [3H]Granisetron). Specifically, crude synapse membrane sample was prepared from rat cerebral cortex, suspended in 50 mM HEPES buffer (pH 7.4) and used for testing. The test compound having several different concentrations and [3H]Granisetron (final concentration 1.0 nM) were added to this suspension and allowed to react at 25°C. Thirty minutes later, the reaction mixture was filtered by suction with cell harvester. The filter was washed with 50 mM Tris buffer (pH 7.4), and the radioactivity on the filter was counted using liquid scintillation counter or β-plate. The non-specific binding was determined in the presence of 1 μM Tropisetron. The concentration of the test compound necessary for 50% inhibition ($IC_{50}$) was determined from a graph and converted to a Ki value. The results are shown in Table 1, wherein * shows $IC_{50}$.

Table 1

| Example | Affinity for receptor (Ki value, nM) | |
|---|---|---|
| | 5-$HT_4$ | 5-$HT_3$ |
| 11 | 0.51 | >1000* |
| 14 | 0.97 | >1000* |
| 25 | 0.94 | >1000* |
| 33 | 0.84 | >1000* |
| 42 | 0.62 | >1000* |
| 44 | 0.67 | >1000* |
| 47 | 2.7 | >1000* |
| 53 | 3.5 | 370 |
| 54 | 1.6 | 340 |
| 57 | 2.4 | >1000* |
| 58 | 1.5 | >1000* |
| 73 | 1.8 | >1000* |
| 74 | 5.1 | >1000* |
| 75 | 1.5 | >1000* |
| 77 | 0.34 | >1000* |
| 81 | 0.50 | >1000* |
| 84 | 1.4 | >1000* |
| 93 | 0.47 | >1000* |

Experimental Example 3 : contraction of the isolated guinea pig ascending colon

About 3 cm long ascending colon segment is taken from male Hartley guinea pig and suspended in 10 ml of a Tyrode solution ventilated with a mixed gas of oxygen (95%) and $CO_2$ (5%) at 37°C with the load of 2 g. The contraction is isotonically measured via isotonic transducer. After an equibilium period of about 30 min, the test compound is administered non-cumulatively from lower concentrations, and concentration-dependent contraction is measured. The test compound is administered at 15 minutes intervals. Then, contraction by the test compound in the presence of a 5-$HT_4$ receptor antagonist, 1-methyl-1H-indole-3-carboxylic acid • 1-(2-methylsulfonylaminoethyl)-piperidin-4-ylmethyl ester (0.01 μM, hereinafter to be referred to as GR113808) is determined, and antagonistic effect on the reaction by GR113808 is confirmed.

The contraction by the test compound is evaluated based on the ratio of reaction relative to mean contraction induced by methacholine (30 μM) administered before and after each determination of concentration-dependent contraction. The contraction is probit-converted using the maximum value of concentration-response curve as 100, and expressed by $EC_{50}$ and maximum reaction rate as determined from linear regression.

Experimental Example 4 : promotion of postcibal enterokinesis of dog without anesthesia

According to the method of Ito et al. [Journal of Smooth Muscle Research, vol. 13, pp. 33-43 (1976)], male and female adult mongrel dogs are subjected to laparotomy under pentobarbital anesthesia, and force transducer is sutured to the serous membrane of various sites of gastrointestinal tract in the direction of circular muscle. After about two weeks' recovery period, postcibal gastrointestinal motility is measured in vigilance.

Experimental Example 5 : Bioavailability in rats

Female SD rats (3 per group) are administered with 2.5 mg/kg (i.v.) and 10 mg/kg (p.o.) of the drug. The blood is taken with the passage of time, and concentration of the drug in plasma is measured with high performance liquid chromatography. The bioavailability is calculated from the following formula wherein AUC means area under plasma concentration time curve.

$$\frac{\text{AUC by oral administration}}{\text{AUC by intravenous administration}} \times \frac{\text{Dose of intravenous administration}}{\text{Dose of oral administration}} \times 100(\%)$$

Experimental Example 6 : Absorption by small intestine in rat

Male SD rats (3 per group) fasted overnight are subjected to laparotomy under ether anesthesia, and the upper part of small intestine is ligated to form a loop (15-20 cm in length). The drug is dissolved in 5% glucose to the drug concentration of 5 mg/10 ml, and 10 ml/kg thereof is injected into the loop. At 2 hours from the drug injection, the loop is removed, washed with 100 ml of 0.1 N hydrochloric acid-methanol (3:7), and the drug concentration is determined by high performance liquid chromatography. The absorption ratio is calculated according to the following formula, and the value is expressed by mean±standard deviation.

$$\frac{\text{Injected amount (mg)-drug concentration in loop (mg/ml)}\times 100\text{ (ml)}}{\text{Injected amount (mg)}} \times 100(\%)$$

Experimental Example 7 : Stimulation of defecation in mouse

Male ddY mice were placed in partition box, and the number and wet weight of feces excreted for 2 hours from immediately after oral administration of the compound of Example 57 were measured.

| Compound | mg/kg p.o. | n | number | promotion (%) | wet weight (mg) | promotion (%) |
|---|---|---|---|---|---|---|
| Vehicle Example 57 | - | 20 | 2.0±0.3 | | 35.1± 5.4 | |
| | 0.1 | 20 | 3.4±0.3 | 70 | 59.2± 6.5 | 69 |
| | 0.3 | 20 | 4.7±0.5 ** | 135 | 82.7±11.5 ** | 136 |
| | 1 | 20 | 4.6±0.6 ** | 130 | 77.2±11.9 ** | 120 |
| | 3 | 20 | 4.7±0.5 ** | 135 | 81.6±10.4 ** | 132 |

**;P<0.01

Experimental Example 8 : Promotion of colonic transit in guinea pig

Male Hartley guinea pigs were subjected to laparotomy under ether anesthesia, and a polyethylene tube was inserted into the beginning of colon. The other end was passed under the skin to be exposed from the neck and fixed there. At day 5 postoperation, Evans blue solution was injected into the colon through the polyethylene tube. One hour

later, the colon was removed, and the range of the Evans blue solution was measured, which was calculated relative to the entire length of the colon and expressed as transit ratio. The compound of Example 57 was orally administered one hour before injection of the Evans blue solution.

| Compound | mg/kg p.o. | n | ratio of colonic transit (%) | promotion (%) |
|---|---|---|---|---|
| Vehicle Example 57 | - | 13 | 44.2±7.7 | |
| | 0.1 | 16 | 54.5±8.0 | 23 |
| | 0.3 | 16 | 54.7±5.9 | 24 |
| | 1 | 15 | 71.8±6.9 * | 62 |
| | 3 | 14 | 73.2±6.3 * | 66 |

*;P<0.05

Experimental Example 9 : Promotion of gastric emptying in rats

Barium pellets (ca. 1 mm diameter) coated with polystyrene was intragastrically administered to male Wistar rats. One hour later, stomach was removed, and the remaining pellets were counted. The compound of Example 57 was orally administered one hour before administration of the pellets. The promotion ratio was calculated from the comparison with the number of remaining pellets in the group administered with vehicle.

| Compound | mg/kg p.o. | number of pellets | promotion (%) |
|---|---|---|---|
| Vehicle Example 57 | - | 28.1 ±1.8 | |
| | 0.3 | 28.2 ±1.3 | 0 |
| | 1 | 24.3 ±2.5 | 14 |
| | 3 | 19.0 ±2.6 * | 32 |
| | 10 | 13.3 ±3.0 ** | 53 |

*;P<0.05,
**;P<0.01

From various pharmacological testings inclusive of the above-mentioned Experimental Examples and toxicity testing, it is evident that the present invention, optical isomers thereof and pharmaceutically acceptable salts thereof have selective and high affinity for 5-$HT_4$ receptors, activate them, are useful as a pharmaceutical preparation for the prophylaxis and treatment of various gastrointestinal diseases, central nervous disorders, cardiac function disorders, urinary diseases and the like, and show superior absorption.

**Claims**

**1.** A benzoic acid compound of the formula

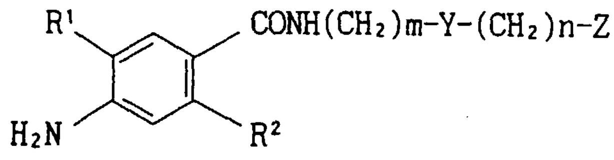

(I)

wherein

R[1] is a halogen;
R[2] is a lower alkoxy, a substituted lower alkoxy, a cycloalkyloxy or a cycloalkylalkoxy;
m is 1 or 2;
Y is

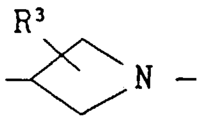
(II-a)

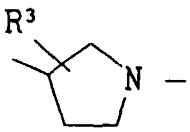
(II-b)

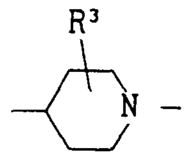
(II-c)

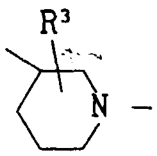
(II-d)

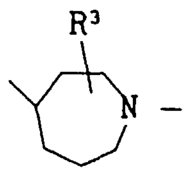
(II-e)

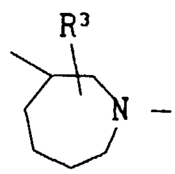
(II-f)

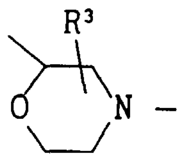
(II-g)

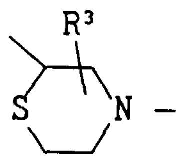
(II-h)

or

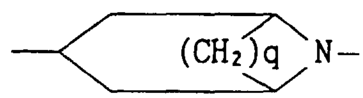
(II-i)

wherein

$R^3$ is hydrogen, hydroxy, lower alkyl or lower alkoxy, and q is 2 or 3;

n is an integer of 1-10; and
Z is a group of the formula

$-N(R^4)(R^5)$,
$-X^1-R^6$ or
$-X^2-R^7$

wherein
$R^4$ and $R^5$ are the same or different and each is hydrogen, lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl, $X^1$ is oxygen atom or sulfur atom, $R^6$ is lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl, $X^2$ is CO, CS, SO or $SO_2$, and $R^7$ is hydrogen, lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl,

an optical isomer thereof or a pharmaceutically acceptable salt thereof.

2. A benzoic acid compound of the formula

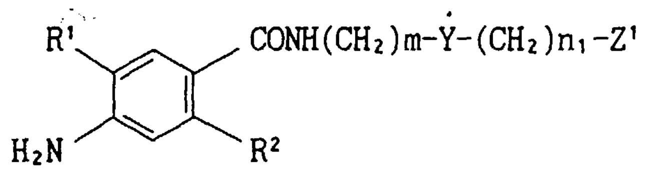

(I-A)

wherein

$R^1$ is a halogen;
$R^2$ is a lower alkoxy, a substituted lower alkoxy, a cycloalkyloxy or a cycloalkylalkoxy;
m is 1 or 2;
Y is

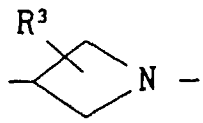

(II-a)

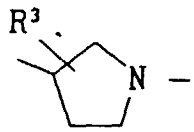

(II-b)

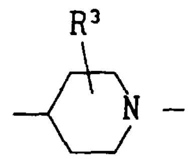

(II-c)

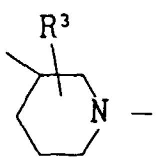

(II-d)

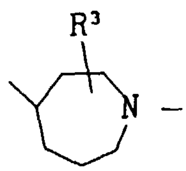

(II-e)

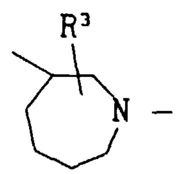

(II-f)

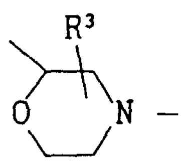

(II-g)

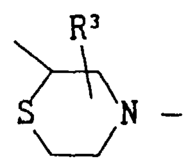

(II-h)

or

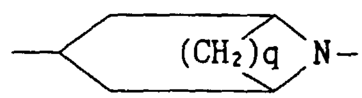

(II-i)

wherein

$R^3$ is hydrogen, hydroxy, lower alkyl or lower alkoxy, and q is 2 or 3;

$n_1$ is an integer of 1-8; and
$Z^1$ is a group of the formula

$-N(R^4)(R^5)$ or
$-X^1-R^6$

wherein

$R^4$ and $R^5$ are the same or different and each is hydrogen, lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl, $X^1$ is oxygen atom or sulfur atom, and $R^6$ is lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl,

an optical isomer thereof or a pharmaceutically acceptable salt thereof.

3. A benzoic acid compound of the formula

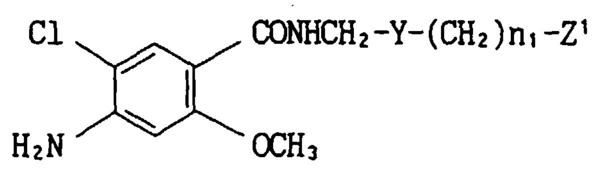

(I-1-A)

wherein Y, $n_1$ and $Z^1$ are as defined in claim 2,
an optical isomer thereof or a pharmaceutically acceptable salt thereof.

4. A benzoic acid compound of the formula

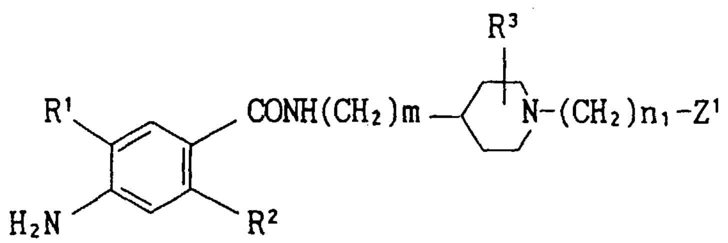

(I-2-A)

wherein $R^1$, $R^2$, $R^3$, m, $n_1$ and $Z^1$ are as defined in claim 2,
or a pharmaceutically acceptable salt thereof.

5. A benzoic acid compound of the formula

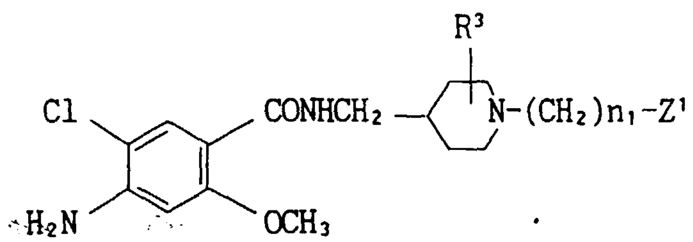

(I-3-A)

wherein $R^3$, $n_1$ and $Z^1$ are as defined in claim 2,
or a pharmaceutically acceptable salt thereof.

6. A benzoic acid compound of any one of claims 2 to 5, wherein $R^4$ and $R^5$ are the same or different and each is hydrogen, lower alkyl, cycloalkylalkyl, aralkyl, substituted aralkyl, heteroarylalkyl or substituted heteroarylalkyl,
an optical isomer thereof or a pharmaceutically acceptable salt thereof.

7. A benzoic acid compound of any one of claims 2 to 5, wherein $R^6$ is aryl, substituted aryl, aralkyl or substituted aralkyl,
an optical isomer thereof or a pharmaceutically acceptable salt thereof.

8. A benzoic acid compound of any one of claims 2 to 7, wherein $n_1$ is an integer of 3 to 8,
an optical isomer thereof or a pharmaceutically acceptable salt thereof.

9. A benzoic acid compound of any one of claims 2 to 7, wherein $n_1$ is an integer of 4 to 6,
an optical isomer thereof or a pharmaceutically acceptable salt thereof.

**10.** A benzoic acid compound of any one of claims 2 to 6, 8 and 9, which is a member selected from the group consisting of

4-amino-5-chloro-N-((1-(6-(N-ethyl-N-benzylamino)hexyl)piperidin-4-yl)methyl)-2-methoxybenzamide,
4-amino-5-chloro-N-((1-(5-(benzylamino)pentyl)piperidin-4-yl)methyl)-2-methoxybenzamide,
4-amino-5-chloro-2-methoxy-N-((1-(5-(1-naphthylmethylamino)pentyl)piperidin-4-yl)methyl)benzamide,
4-amino-5-chloro-N-((1-(5-(N-ethyl-N-(4-fluorobenzyl)amino)pentyl)piperidin-4-yl)methyl)-2-methoxybenzamide,
4-amino-5-chloro-2-methoxy-N-((1-(5-(N-n-propyl-N-benzylamino)pentyl)piperidin-4-yl)methyl)benzamide,
4-amino-5-chloro-N-((1-(5-(cyclohexylmethylamino)pentyl)piperidin-4-yl)methyl)-2-methoxybenzamide,
4-amino-5-chloro-N-((1-(4-(N-(3,4-dichlorobenzyl)-N-ethylamino)butyl)piperidin-4-yl)methyl)-2-methoxybenzamide,
4-amino-5-chloro-N-((1-(6-(3,4-dichlorobenzylamino)hexyl)piperidin-4-yl)methyl)-2-methoxybenzamide,
4-amino-5-chloro-N-((1-(6-(N-ethyl-N-(4-methylbenzyl)amino)hexyl)piperidin-4-yl)methyl)-2-methoxybenzamide,
4-amino-5-chloro-N-((1-(6-(N-ethyl-N-(4-nitrobenzyl)amino)hexyl)piperidin-4-yl)methyl)-2-methoxybenzamide,
4-amino-5-chloro-2-methoxy-N-((1-(5-(4-methylbenzylamino)pentyl)piperidin-4-yl)methyl)benzamide,
4-amino-5-chloro-N-((1-(6-(N-ethyl-N-(2-thienylmethyl)amino)hexyl)piperidin-4-yl)methyl)-2-methoxybenzamide,
4-amino-5-chloro-2-methoxy-N-((1-(5-(2-naphthylmethylamino)pentyl)piperidin-4-yl)methyl)benzamide,
4-amino-5-chloro-2-methoxy-N-((1-(6-(4-methoxybenzylamino)hexyl)piperidin-4-yl)methyl)benzamide and
4-amino-5-chloro-2-methoxy-N-((1-(6-(2-thienylmethylamino)hexyl)piperidin-4-yl)methyl)benzamide,
or a pharmaceutically acceptable salt thereof.

**11.** A benzoic acid compound of any one of claims 2 to 5 and 7 to 9, which is a member selected from the group consisting of

4-amino-N-((1-(5-benzylthiopentyl)piperidin-4-yl)methyl)-5-chloro-2-methoxybenzamide,
4-amino-N-((1-(5-benxyloxypentyl)piperidin-4-yl)methyl)-5-chloro-2-methoxybenzamide,
4-amino-5-chloro-2-methoxy-N-((1-(5-phenoxypentyl)piperidin-4-yl)methyl)benzamide,
4-amino-5-chloro-2-methoxy-N-((1-(5-phenylthiopentyl)piperidin-4-yl)methyl)benzamide,
4-amino-5-chloro-N-((1-(5-(4-chlorobenzyloxy)pentyl)piperidin-4-yl)methyl)-2-methoxybenzamide,
4-amino-5-chloro-2-methoxy-N-((1-(4-phenoxybutyl)piperidin-4-yl)methyl)benzamide and
4-amino-N-((1-(6-benzyloxyhexyl)piperidin-4-yl)methyl)-5-chloro-2-methoxybenzamide,
or a pharmaceutically acceptable salt thereof.

**12.** A benzoic acid compound of the formula

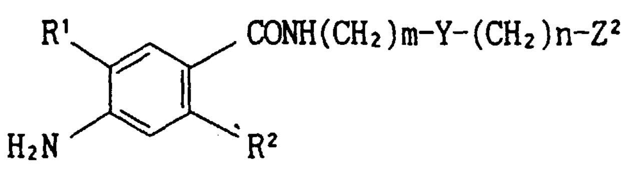

(I-B)

wherein

$R^1$ is a halogen;
$R^2$ is a lower alkoxy, a substituted lower alkoxy, a cycloalkyloxy or a cycloalkylalkoxy;
m is 1 or 2;
Y is

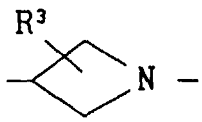 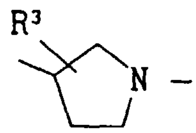 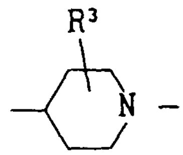

(II-a) (II-b) (II-c)

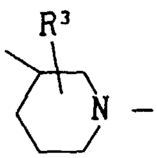 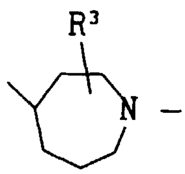 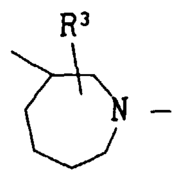

(II-d) (II-e) (II-f)

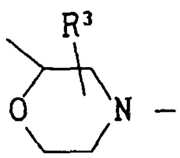 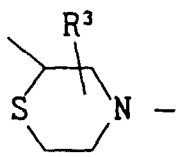 or 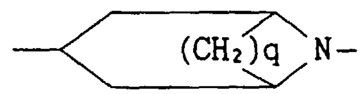

(II-g) (II-h) (II-i)

wherein

$R^3$ is hydrogen, hydroxy, lower alkyl or lower alkoxy, and q is 2 or 3;

n is an integer of 1-10; and

$Z^2$ is a group of the formula

$-X^2-R^7$

wherein

$X^2$ is CO, CS, SO or $SO_2$, and $R^7$ is hydrogen, lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl,

an optical isomer thereof or a pharmaceutically acceptable salt thereof.

**13.** A benzoic acid compound of the formula

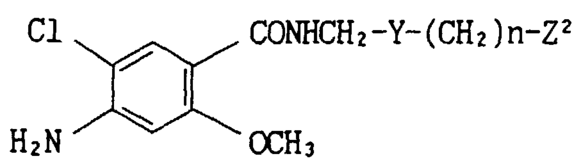

(I-1-B)

wherein Y, n and $Z^2$ are as defined in claim 12, an optical isomer thereof or a pharmaceutically acceptable salt thereof.

**14.** A benzoic acid compound of the formula

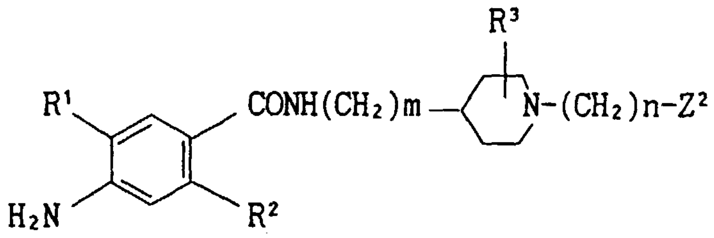

(I-2-B)

wherein $R^1$, $R^2$, $R^3$, m, n and $Z^2$ are as defined in claim 12, or a pharmaceutically acceptable salt thereof.

**15.** A benzoic acid compound of the formula

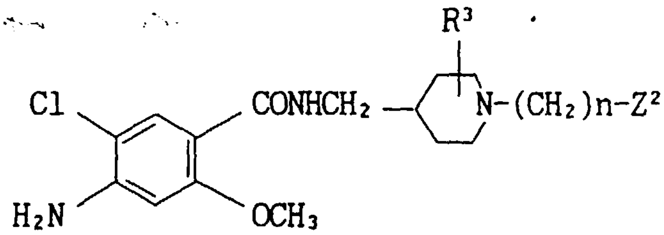

(I-3-B)

wherein $R^3$, n and $Z^2$ are as defined in claim 12, or a pharmaceutically acceptable salt thereof.

**16.** A benzoic acid compound of any one of claims 12 to 15, wherein $X^2$ is CO, and $R^7$ is aryl, substituted aryl, heteroaryl or substituted heteroaryl, an optical isomer thereof or a pharmaceutically acceptable salt thereof.

**17.** A benzoic acid compound of any one of claims 12 to 15, wherein $X^2$ is $SO_2$, and $R^7$ is aryl, substituted aryl, aralkyl or substituted aralkyl, an optical isomer thereof or a pharmaceutically acceptable salt thereof.

**18.** A benzoic acid compound of any one of claims 12 to 17, wherein n is an integer of 3 to 8, an optical isomer thereof or a pharmaceutically acceptable salt thereof.

**19.** A benzoic acid compound of any one of claims 12 to 17, wherein n is an integer of 4 to 6, an optical isomer thereof or a pharmaceutically acceptable salt thereof.

**20.** A benzoic acid compound of any one of claims 12 to 16, 18 and 19, which is a member selected from the group consisting of

4-amino-5-chloro-2-methoxy-N-((1-(6-oxo-6-phenylhexyl)piperidin-4-yl)methyl)benzamide,
4-amino-5-chloro-2-methoxy-N-((1-(7-oxo-7-phenylheptyl)piperidin-4-yl)methyl)benzamide,
4-amino-5-chloro-2-methoxy-N-((1-(6-(1-isopropyl-1H-indol-3-yl)-6-oxohexyl)piperidin-4-yl)methyl)benzamide,
4-amino-5-chloro-2-methoxy-N-((1-(6-(1-ethyl-1H-indol-3-yl)-6-oxohexyl)piperidin-4-yl)methyl)benzamide,
4-amino-5-chloro-2-methoxy-N-((1-(6-(1-naphthyl)-6-oxohexyl)piperidin-4-yl)methyl)benzamide,
4-amino-5-chloro-N-((1-(6-(3-fluoro-4-methoxyphenyl)-6-oxohexyl)piperidin-4-yl)methyl)-2-methoxybenzamide,
4-amino-5-chloro-N-((1-(6-(3-chloro-4-methoxyphenyl)-6-oxohexyl)piperidin-4-yl)methyl)-2-methoxybenzamide,
4-amino-5-chloro-N-((1-(6-(3,4-dimethoxyphenyl)-6-oxohexyl)piperidin-4-yl)methyl)-2-methoxybenzamide,
4-amino-5-chloro-N-((1-(6-(4-hydroxyphenyl)-6-oxohexyl)piperidin-4-yl)methyl)-2-methoxybenzamide,
4-amino-5-chloro-2-methoxy-N-((1-(6-(1-methyl-1H-indol-3-yl)-6-oxohexyl)piperidin-4-yl)methyl)benzamide,
4-amino-5-chloro-2-methoxy-N-((1-(5-(1-methyl-1H-indol-3-yl)-5-oxopentyl)piperidin-4-yl)methyl)benzamide,
4-amino-5-chloro-2-methoxy-N-((1-(7-(1-methyl-1H-indol-3-yl)-7-oxoheptyl)piperidin-4-yl)methyl)benzamide,

4-amino-5-chloro-2-methoxy-N-((1-(5-oxo-5-phenylpentyl)piperidin-4-yl)methyl)benzamide and
4-amino-5-chloro-2-methoxy-N-((1-(6-(1H-indol-3-yl)-6-oxohexyl)piperidin-4-yl)methyl)benzamide,
or a pharmaceutically acceptable salt thereof.

**21.** A benzoic acid compound of any one of claims 12 to 15, and 17 to 19, which is a member selected from the group consisting of

4-amino-5-chloro-2-methoxy-N-((1-(4-phenylsulfonylbutyl)piperidin-4-yl)methyl)benzamide and
4-amino-5-chloro-2-methoxy-N-((1-(5-phenylsulfonylpentyl)piperidin-4-yl)methyl)benzamide,
or a pharmaceutically acceptable salt thereof.

**22.** A pharmaceutical composition comprising a benzoic acid compound of any one of claims 1 to 21, an optical isomer thereof or a pharmaceutically acceptable salt thereof, and a pharmaceutically acceptable additive.

**23.** A serotonin 4 receptor agonist comprising a benzoic acid compound of any one of claims 1 to 21, an optical isomer thereof or a pharmaceutically acceptable salt thereof as an active ingredient.

**24.** A gastrointestinal prokinetic agent comprising a benzoic acid compound of any one of claims 1 to 21, an optical isomer thereof or a pharmaceutically acceptable salt thereof as an active ingredient.

**25.** A therapeutic agent for gastrointestinal diseases selected from the group consisting of reflux esophagitis; gastroesophageal reflux; Barrett syndrome; intestinal pseudoileus; acute or chronic gastritis; gastric or duodenal ulcer; Crohn's disease; non-ulcer dyspepsia; ulcerative colitis; postgastrectomy syndrome; postoperative digestive function failure; delayed gastric emptying caused by gastric neurosis, gastroptosis and diabetes; gastrointestinal disorders such as indigestion, meteorism and abdominal indefinite complaint; constipation such as atonic constipation, chronic constipation, and that caused by spinal cord injury and pelvic diaphragm failure; and irritable bowel syndrome, which comprises a benzoic acid compound of any one of claims 1 to 21, an optical isomer thereof or a pharmaceutically acceptable salt thereof as an active ingredient.

**Amended claims under Art. 19.1 PCT**

1. (amended) A benzoic acid compound of the formula

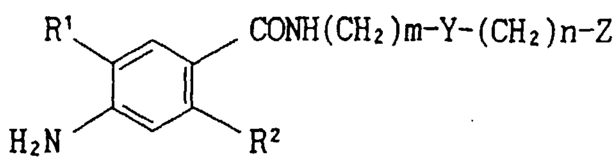

(I)

wherein

$R^1$ is a halogen;
$R^2$ is a lower alkoxy, a substituted lower alkoxy, a cycloalkyloxy or a cycloalkylalkoxy;
m is 1 or 2;
Y is

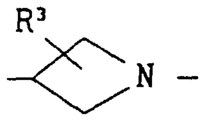

(II-a)

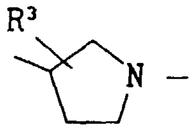

(II-b)

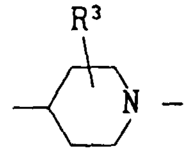

(II-c)

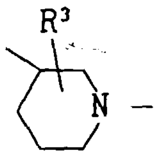

(II-d)

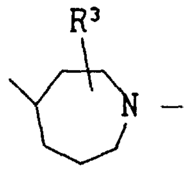

(II-e)

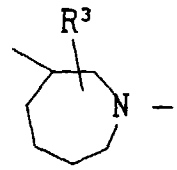

(II-f)

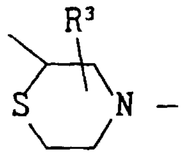

(II-h)

or

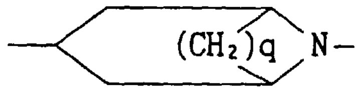

(II-i)

wherein

$R^3$ is hydrogen, hydroxy, lower alkyl or lower alkoxy, and q is 2 or 3;

n is an integer of 1-10; and

Z is a group of the formula

$-N(R^4)(R^5)$,
$-X^1-R^6$ or
$-X^2-R^7$

wherein

$R^4$ and $R^5$ are the same or different and each is hydrogen, lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl, $X^1$ is oxygen atom or sulfur atom, $R^6$ is lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl, $X^2$ is CO, CS, SO or $SO_2$, and $R^7$ is hydrogen, lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl,

an optical isomer thereof or a pharmaceutically acceptable salt thereof.

2. (amended) A benzoic acid compound of the formula

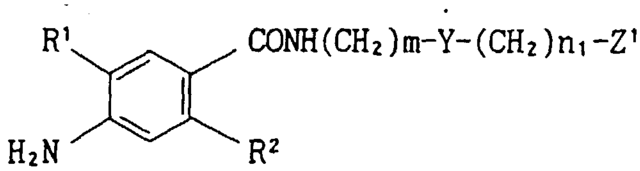

(I-A)

wherein

$R^1$ is a halogen;
$R^2$ is a lower alkoxy, a substituted lower alkoxy, a cycloalkyloxy or a cycloalkylalkoxy;
m is 1 or 2;
Y is

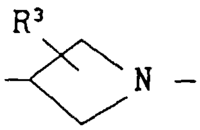
(II-a)

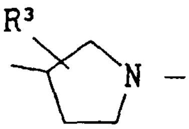
(II-b)

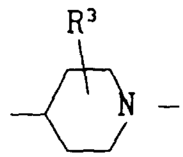
(II-c)

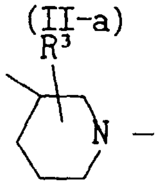
(II-d)

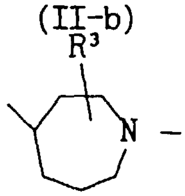
(II-e)

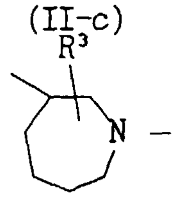
(II-f)

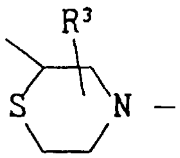
(II-h)

or

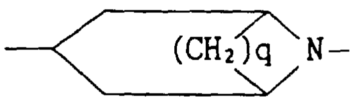
(II-i)

wherein

$R^3$ is hydrogen, hydroxy, lower alkyl or lower alkoxy, and q is 2 or 3;

$n_1$ is an integer of 1-8; and
$Z^1$ is a group of the formula

$-N(R^4)(R^5)$ or
$-X^1-R^6$
wherein
$R^4$ and $R^5$ are the same or different and each is hydrogen, lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl, $X^1$ is oxygen atom or sulfur atom, and $R^6$ is lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl,

an optical isomer thereof or a pharmaceutically acceptable salt thereof.

3. A benzoic acid compound of the formula

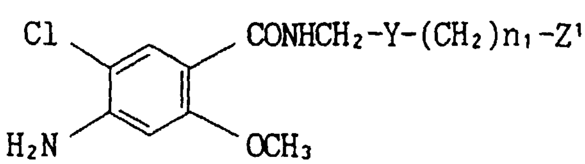
(I-1-A)

wherein Y, $n_1$ and $Z^1$ are as defined in claim 2,
an optical isomer thereof or a pharmaceutically acceptable salt thereof.

4. A benzoic acid compound of the formula

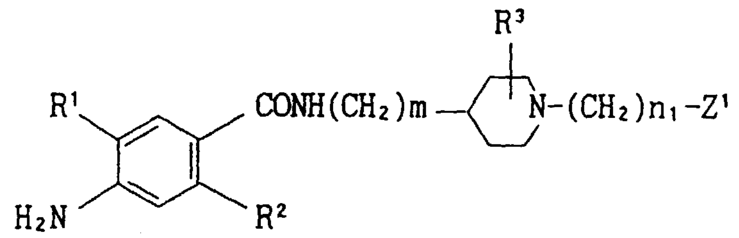

(I-2-A)

wherein $R^1$, $R^2$, $R^3$, m, $n_1$ and $Z^1$ are as defined in claim 2,
or a pharmaceutically acceptable salt thereof.

5. A benzoic acid compound of the formula

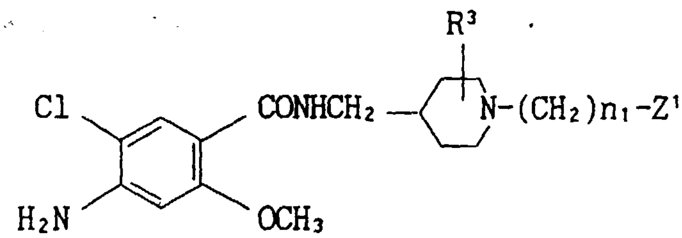

(I-3-A)

wherein $R^3$, $n_1$ and $Z^1$ are as defined in claim 2,
or a pharmaceutically acceptable salt thereof.

6. A benzoic acid compound of any one of claims 2 to 5, wherein $R^4$ and $R^5$ are the same or different and each is hydrogen, lower alkyl, cycloalkylalkyl, aralkyl, substituted aralkyl, heteroarylalkyl or substituted heteroarylalkyl,
an optical isomer thereof or a pharmaceutically acceptable salt thereof.

7. A benzoic acid compound of any one of claims 2 to 5, wherein $R^6$ is aryl, substituted aryl, aralkyl or substituted aralkyl,
an optical isomer thereof or a pharmaceutically acceptable salt thereof.

8. A benzoic acid compound of any one of claims 2 to 7, wherein $n_1$ is an integer of 3 to 8,
an optical isomer thereof or a pharmaceutically acceptable salt thereof.

9. A benzoic acid compound of any one of claims 2 to 7, wherein $n_1$ is an integer of 4 to 6,
an optical isomer thereof or a pharmaceutically acceptable salt thereof.

10. A benzoic acid compound of any one of claims 2 to 6, 8 and 9, which is a member selected from the group consisting of

4-amino-5-chloro-N-((1-(6-(N-ethyl-N-benzylamino)hexyl)piperidin-4-yl)methyl)-2-methoxybenzamide,
4-amino-5-chloro-N-((1-(5-(benzylamino)pentyl)piperidin-4-yl)methyl)-2-methoxybenzamide,
4-amino-5-chloro-2-methoxy-N-((1-(5-(1-naphthylmethylamino)pentyl)piperidin-4-yl)methyl)benzamide,
4-amino-5-chloro-N-((1-(5-(N-ethyl-N-(4-fluorobenzyl)amino)pentyl)piperidin-4-yl)methyl)-2-methoxybenzamide,
4-amino-5-chloro-2-methoxy-N-((1-(5-(N-n-propyl-N-benzylamino)pentyl)piperidin-4-yl)methyl)benzamide,
4-amino-5-chloro-N-((1-(5-(cyclohexylmethylamino)pentyl)piperidin-4-yl)methyl)-2-methoxybenzamide,
4-amino-5-chloro-N-((1-(4-(N-(3,4-dichlorobenzyl)-N-ethylamino)butyl)piperidin-4-yl)methyl)-2-methoxybenzamide,
4-amino-5-chloro-N-((1-(6-(3,4-dichlorobenzylamino)hexyl)piperidin-4-yl)methyl)-2-methoxybenzamide,
4-amino-5-chloro-N-((1-(6-(N-ethyl-N-(4-methylbenzyl)amino)hexyl)piperidin-4-yl)methyl)-2-methoxybenzamide,

4-amino-5-chloro-N-((1-(6-(N-ethyl-N-(4-nitrobenzyl)amino)hexyl)piperidin-4-yl)methyl)-2-methoxybenzamide,
4-amino-5-chloro-2-methoxy-N-((1-(5-(4-methylbenzylamino)pentyl)piperidin-4-yl)methyl)benzamide,
4-amino-5-chloro-N-((1-(6-(N-ethyl-N-(2-thienylmethyl)amino)hexyl)piperidin-4-yl)methyl)-2-methoxybenzamide,
4-amino-5-chloro-2-methoxy-N-((1-(5-(2-naphthylmethylamino)pentyl)piperidin-4-yl)methyl)benzamide,
4-amino-5-chloro-2-methoxy-N-((1-(6-(4-methoxybenzylamino)hexyl)piperidin-4-yl)methyl)benzamide and
4-amino-5-chloro-2-methoxy-N-((1-(6-(2-thienylmethylamino)hexyl)piperidin-4-yl)methyl)benzamide,
or a pharmaceutically acceptable salt thereof.

11. A benzoic acid compound of any one of claims 2 to 5 and 7 to 9, which is a member selected from the group consisting of

4-amino-N-((1-(5-benzylthiopentyl)piperidin-4-yl)methyl)-5-chloro-2-methoxybenzamide,
4-amino-N-((1-(5-benzyloxypentyl)piperidin-4-yl)methyl)-5-chloro-2-methoxybenzamide,
4-amino-5-chloro-2-methoxy-N-((1-(5-phenoxypentyl)piperidin-4-yl)methyl)benzamide,
4-amino-5-chloro-2-methoxy-N-((1-(5-phenylthiopentyl)piperidin-4-yl)methyl)benzamide,
4-amino-5-chloro-N-((1-(5-(4-chlorobenzyloxy)pentyl)piperidin-4-yl)methyl)-2-methoxybenzamide,
4-amino-5-chloro-2-methoxy-N-((1-(4-phenoxybutyl)piperidin-4-yl)methyl)benzamide and
4-amino-N-((1-(6-benzyloxyhexyl)piperidin-4-yl)methyl)-5-chloro-2-methoxybenzamide,
or a pharmaceutically acceptable salt thereof.

12. (amended) A benzoic acid compound of the formula

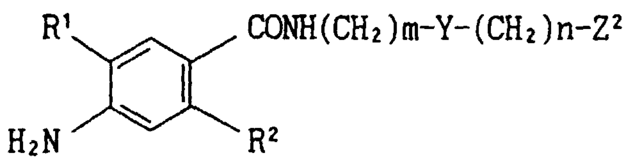

(I-B)

wherein

$R^1$ is a halogen;
$R^2$ is a lower alkoxy, a substituted lower alkoxy, a cycloalkyloxy or a cycloalkylalkoxy;
m is 1 or 2;
Y is

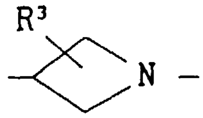

(II-a)

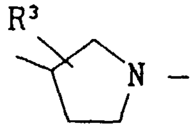

(II-b)

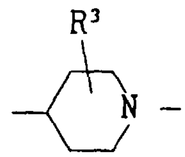

(II-c)

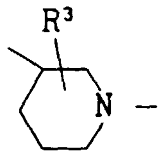

(II-d)

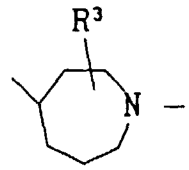

(II-e)

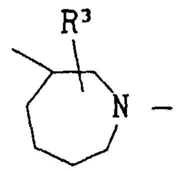

(II-f)

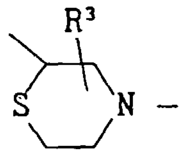

(II-h)

or

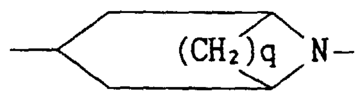

(II-i)

wherein

$R^3$ is hydrogen, hydroxy, lower alkyl or lower alkoxy, and q is 2 or 3;

n is an integer of 1-10; and

$Z^2$ is a group of the formula

$-X^2-R^7$

wherein

$X^2$ is CO, CS, SO or $SO_2$, and $R^7$ is hydrogen, lower alkyl, cycloalkyl, cycloalkylalkyl, aryl, substituted aryl, aralkyl, substituted aralkyl, heteroaryl, substituted heteroaryl, heteroarylalkyl or substituted heteroarylalkyl,

an optical isomer thereof or a pharmaceutically acceptable salt thereof.

13. A benzoic acid compound of the formula

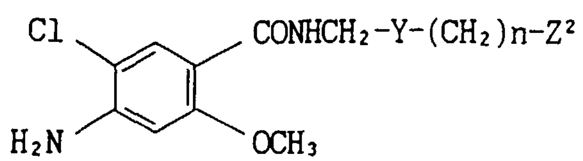

(I-1-B)

wherein Y, n and $Z^2$ are as defined in claim 12,
an optical isomer thereof or a pharmaceutically acceptable salt thereof.

14. A benzoic acid compound of the formula

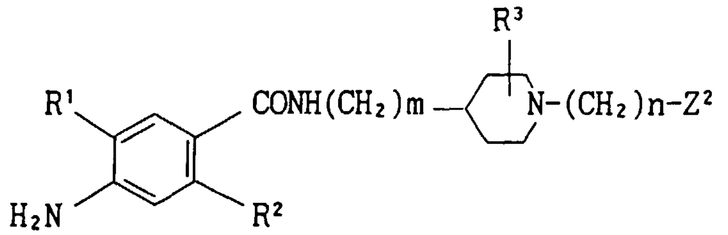

(I-2-B)

wherein $R^1$, $R^2$, $R^3$, m, n and $Z^2$ are as defined in claim 12, or a pharmaceutically acceptable salt thereof.

15. A benzoic acid compound of the formula

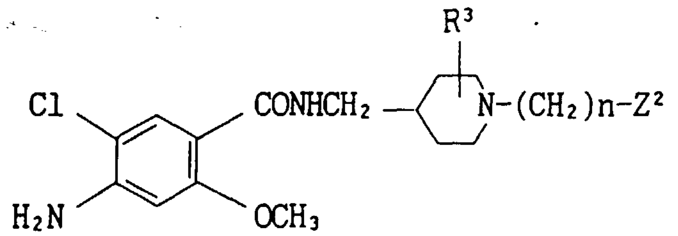

(I-3-B)

wherein $R^3$, n and $Z^2$ are as defined in claim 12, or a pharmaceutically acceptable salt thereof.

16. A benzoic acid compound of any one of claims 12 to 15, wherein $X^2$ is CO, and $R^7$ is aryl, substituted aryl, heteroaryl or substituted heteroaryl,
an optical isomer thereof or a pharmaceutically acceptable salt thereof.

17. A benzoic acid compound of any one of claims 12 to 15, wherein $X^2$ is $SO_2$, and $R^7$ is aryl, substituted aryl, aralkyl or substituted aralkyl,
an optical isomer thereof or a pharmaceutically acceptable salt thereof.

**INTERNATIONAL SEARCH REPORT**

International application No.
PCT/JP96/02711

**A. CLASSIFICATION OF SUBJECT MATTER** Int. Cl[6] C07D207/14, 209/14, 211/34, 211/40, 233/48, 239/42, 261/20, 307/79, 401/06, 401/12, 405/12, 409/06, 409/12, 413/12, 417/12, A61K31/40, 415, 44, 445, 505

According to International Patent Classification (IPC) or to both national classification and IPC

**B. FIELDS SEARCHED**

Minimum documentation searched (classification system followed by classification symbols)
Int. Cl[6] C07D207/14, 209/14, 211/34, 211/40, 233/48, 239/42, 261/20, 307/79, 401/06, 401/12, 405/12, 409/06, 409/12, 413/12, 417/12, A61K31/40, 415, 44, 445, 505

Documentation searched other than minimum documentation to the extent that such documents are included in the fields searched

Electronic data base consulted during the international search (name of data base and, where practicable, search terms used)
CAS ONLINE

**C. DOCUMENTS CONSIDERED TO BE RELEVANT**

| Category* | Citation of document, with indication, where appropriate, of the relevant passages | Relevant to claim No. |
|---|---|---|
| P,A | WO, 96/28424, A1 (Boehringer Ingelheim Italia S.P.A.), September 19, 1996 (19. 09. 96), Claim (Family: none) | 1 - 25 |
| P,A | WO, 95/26953, A1 (Yoshitomi Pharmaceutical Industries Ltd.), October 12, 1995 (12. 10. 95), Claim (Family: none) | 1 - 25 |
| Y | WO, 92/14705, A1 (Hokuriku Seiyaku Co., Ltd.), September 3, 1992 (03. 09. 92), Full descriptions & TW, 213460, A & EP, 640601, A1 & US, 5395832, A & TW, 243449, A & US, 5500422, A | 1-3, 6-9, 12, 13, 16, 18, 19, 22-25 |
| X | JP, 2-42069, A (Dainippon Pharmaceutical Co., Ltd.), February 13, 1990 (13. 02. 90), Full descriptions & EP, 243959, A & AU, 8772275, A & ZA, 8703022, A | 1-3, 6-9, 12, 13, 16, 18, 19, 22-25 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| January 7, 1997 (07. 01. 97) | January 14, 1997 (14. 01. 97) |
| Name and mailing address of the ISA/ Japanese Patent Office | Authorized officer |
| Facsimile No. | Telephone No. |

Form PCT/ISA/210 (second sheet) (July 1992)

INTERNATIONAL SEARCH REPORT

International application No.
PCT/JP96/02711

C (Continuation). DOCUMENTS CONSIDERED TO BE RELEVANT

| Category* | Citation of document, with indication, where appropriate, of the relevant passages | Relevant to claim No. |
|---|---|---|
| | & FI, 8701828, A & DK, 8702187, A & DD, 256325, A & JP, 63-264467, A & US, 4870074, A & SU, 1597101, A | |
| X | Chem. Pharm. Bull., 40(3), (1992), Shiro Kato et al., "Novel Benzamides as Selective and Potent Gastrokinetic Agents. III. Synthesis and Structure-Activity Relationships of 4-Amino-5-chloro-2-methoxy- and 2-Ethoxy-N-((4-substituted 2-morpholinyl)mehyl)-benzamides", p. 652-660 | 1-3, 6-9, 12, 13, 16, 18, 19, 22-25 |

Form PCT/ISA/210 (continuation of second sheet) (July 1992)